# NEW EUROPEAN PATENT SPECIFICATION

(11) **EP 2 780 022 B2**
(45) Date of publication and mention of the opposition decision: **07.09.2022**
(45) Mention of the grant of the patent: 05.06.2019
(21) Application number: 12848968.9
(22) Date of filing: 14.11.2012
(51) Int. Cl.: A61K 35/44, A61K 35/50, A61K 35/12, A61P 27/02, C12N 5/095

(54) **PHARMACEUTICAL PREPARATIONS OF HUMAN RPE CELLS AND USES THEREOF**
PHARMAZEUTISCHE ZUBEREITUNGEN VON MENSCHLICHEN RPE-ZELLEN UND IHRE VERWENDUNG
PRÉPARATIONS PHARMACEUTIQUES DE CELLULES DE RPE HUMAINES ET LEURS UTILISATIONS

(30) Priority: 14.11.2011 US 201161559521 P; 23.01.2012 US 201261589741 P; 08.11.2012 US 201261724047 P
(43) Date of publication of application: 24.09.2014
(60) Divisional application: 19164689.2
(73) Proprietor: Astellas Institute for Regenerative Medicine, Marlborough, MA 01752 (US)
(72) Inventor: GAY, Roger, Belmont, Massachusetts 02478 (US); KLIMANSKAYA, Irina, Upton, Massachusetts 01568 (US); LANZA, Robert, Clinton, Massachusetts 01510 (US)
(74) Representative: Boult Wade Tennant LLP
(86) International application number: PCT/US2012/065091
(87) International publication number: WO 2013/074681

(56) References cited:
- WO-A1-2009/051671
- WO-A2-2011/063005
- US-A1- 2007 031 386
- US-B2- 7 794 704
- LU FAN ET AL: "Expression of melanin-related genes in cultured adult humanretinal pigment epithelium and uveal melanoma cells", MOLECULAR VISION, vol. 13, 2007, pages 2066-2072, XP002741203,
- VUGLER A ET AL: "Elucidating the phenomenon of HESC-derived RPE: Anatomy of cell genesis, expansion and retinal transplantation", EXPERIMENTAL NEUROLOGY, ACADEMIC PRESS, NEW YORK, NY, US, vol. 214, no. 2, 1 December 2008 (2008-12-01), pages 347-361, XP025695837, ISSN: 0014-4886, DOI: 10.1016/J.EXPNEUROL.2008.09.007 [retrieved on 2008-09-27]
- MICHAEL E. BOULTON: "Studying melanin and lipofuscin in RPE cell culture models", EXPERIMENTAL EYE RESEARCH, vol. 126, 1 September 2014 (2014-09-01), pages 61-67, XP055197430, ISSN: 0014-4835, DOI: 10.1016/j.exer.2014.01.016
- HARUTA ET AL.: 'In Vitro and In Vivo Characterization of Pigment Epithelial Cells Differentiated from Primate Embryonic Stem Cells' INVESTIGATIVE OPHTHALMOLOGY & VISUAL SCIENCE vol. 45, no. 3, March 2004, pages 1020 - 1025, XP002487262

## Description

### BACKGROUND

Human embryonic stem cells (hESCs) are considered a promising source of replacement cells for regenerative medicine (1). Despite great scientific progress, hESCs are among the most complex biological therapeutic entities proposed for clinical use to date (2). In addition to the dynamic complexity of their biology, numerous regulatory concerns have hindered clinical translation, including the risk of teratoma formation and the challenges associated with histoincompatibility. Until cellular reprogramming technologies, such as somatic cell nuclear transfer (3) or induced pluripotent stem cells (4, 5) are further developed, diseases affecting the eye and other immunoprivileged sites are likely to be the first pluripotent stem cell-based therapies in patients. It is well established that the subretinal space is protected by a blood-ocular barrier, and is characterized by antigen-specific inhibition of both the cellular and humoral immune responses (6).

In the retina, degeneration of the retinal pigment epithelium (RPE) leads to photoreceptor loss in a variety of sight-threatening diseases, including dry age-related macular degeneration (AMD) and Stargardt's macular dystrophy (SMD), two of the leading causes of adult and juvenile blindness in the world, respectively. Although both are currently untreatable, there is evidence in preclinical models of macular degeneration that transplantation of hESC-derived RPE can rescue photoreceptors and prevent visual loss (7, 8). Among its functions, the RPE maintains the health of photoreceptors by recycling photopigments, delivering, metabolizing and storing vitamin A, phagocytosing photoreceptor outer segments, transporting iron and small molecules between the retina and choroid, and absorbing stray light to allow better image resolution (9, 10). In the Royal College of Surgeons (RCS) rat, an animal model in which vision deteriorates due to RPE dysfunction, subretinal transplantation of hESC-derived RPE resulted in extensive photoreceptor rescue and improvement in visual performance (100% over untreated controls) without evidence of untoward pathology (7).

The retinal pigment epithelium (RPE) is the pigmented cell layer outside the neurosensory retina between the underlying choroid (the layer of blood vessels behind the retina) and overlying retinal visual cells (*e.g*., photoreceptors-rods and cones). The RPE is critical to the function and health of photoreceptors and the retina. The RPE maintains photoreceptor function by recycling photopigments, delivering, metabolizing, and storing vitamin A, phagocytosing rod photoreceptor outer segments, transporting iron and small molecules between the retina and choroid, maintaining Bruch's membrane and absorbing stray light to allow better image resolution. Engelmann and Valtink (2004) "RPE Cell Cultivation." Graefe's Archive for Clinical and Experimental Ophthalmology 242(1): 65-67; *See also* Irina Klimanskaya, Retinal Pigment Epithelium Derived From Embryonic Stem Cells, in STEM CELL ANTHOLOGY 335-346 (Bruce Carlson ed., 2009). Degeneration of the RPE can cause retinal detachment, retinal dysplasia, or retinal atrophy that is associated with a number of vision-altering ailments that result in photoreceptor damage and blindness, such as, choroideremia, diabetic retinopathy, macular degeneration (including age-related macular degeneration), retinitis pigmentosa, and Stargardt's Disease (fundus flavimaculatus). *See, e.g.*, WO 2009/051671.

Certain subject matter including methods of making RPE cells, compositions of RPE cells, and uses thereof are disclosed in co-owned U.S. applications and patents including U.S. Ser. No. 11/186720, filed 20-Jul-2005, now U.S. Patent No. 7736896; U.S. Ser. No. 11/490953, filed 21-Jul-2006, now U.S. Pat. No. 7795025; U.S. Ser. No. 11/041382, filed 24-Jan-2005, now U.S. Pat. No. 7794704; U.S. Provisional Application No. 60/538964, filed 23-Jan-2004; U.S. Ser. No. 12/682,712, filed 10-Oct-2010; U.S. Provisional Application No. 60/998668, filed 12-Oct-2007; U.S. Provisional Application No. 60/998766, filed 12-Oct-2007; U.S. Provisional Application No. 61/009908, filed 02-Jan-2008; U.S. Provisional Application No. 61/009911, filed 02-Jan-2008; U.S. Provisional Application No. 61/367038, filed 23-Jul-2010; U.S. Provisional Application No. 61/414770, filed 17-Nov-2010; International Patent Application No. PCT/US11/45232, filed 25-Jul-2011; U.S. Ser. No. 12/682712, filed 14-Dec-2010; International Patent Application No. PCT/US05/02273, filed 24-Jan-2005; International Patent Application No. PCT/US2010/57056 filed November 17, 2010 (published as WO 2011/063005); and U.S. Provisional Patent Application No. 61/262,002, filed November 17, 2009.

Though transplantation of intact sheets and suspensions of primary RPE cells has been previously attempted in human subjects, results have been mixed, both in terms of graft survival and visual improvement (11-19). To date, consistently effective human therapeutics using primary RPE cells has not been reported.

### SUMMARY

The present invention is defined by the claims. This disclosure reports Phase 1/2 clinical data that help demonstrate the safety of human embryonic stem cell (hESC)-derived retinal pigment epithelium (RPE) cells for the treatment of Stargardt's macular dystrophy (SMD) and dry age-related macular degeneration (dry AMD). Results are reported for two patients, the first in each of the Phase 1/2 clinical trials. In addition to showing no adverse safety issues, structural evidence confirmed that the hESC-derived cells survived and continued to persist during the study period reported. Both patients had measurable improvements in their vision that persisted for at least one year.

At one year following treatment, no hyperproliferation, tumorigenicity, ectopic tissue formation, or apparent rejection were observed in either patient at any time. Detailed clinical and diagnostic laboratory assessments were performed at multiple post-transplantation evaluations. Abnormal growth (or tumor formation) would be considered a significant safety concern for stem-cell based therapies, in particular those derived from hESCs due to their pluripotency; it is therefore critical to control the differentiation of hESCs. Results reported indicate that stem cell differentiation was well controlled in these patients. No adverse safety signals were detected.

Anatomic evidence of successful stem cell derived RPE transplantation was observed clinically and with high resolution imaging technology in the patient with SMD. This evidence included increasing pigmentation at the level of RPE, within the area of the transplant, beginning one week after transplantation and throughout the follow-up period. Transplanted stem cell derived RPE appeared to engraft in the proper location and assume normal RPE morphology. Engraftment and increasing pigmentation were not detected in the dry AMD patient. However, both patients showed some visual improvement at the four month follow-up period, which persisted at least out to the one year follow-up period.

As further described below, the visual acuity of the Stargardt's patient improved from hand motions only to 20/800 vision. Before treatment, the patient was unable to read any letter on the ETDRS visual acuity chart. However, by two weeks post-transplantation, she was able to start reading letters, which improved to five letters at one to three months and 15 letters at one year in the treated eye (20/500 vision).

Although several new drugs are available for the treatment of the wet type of AMD, no proven treatments currently exist for either dry AMD or Stargardt's disease. Despite the progressive nature of these conditions, the vision of both patients appears to have improved after transplantation of the cells, even at the lowest dosage. Applicants expect even more significant improvement when treating patients earlier in the course of the disease, where more significant results might potentially be expected. Increased cell dosage may also lead to more significant improvement.

Human embryonic stem cells can provide a superior source of replacement tissue by producing an unlimited number of healthy "young" cells with potentially reduced immunogenicity. The eye is an immune privileged site due to the protection of the subretinal space by a blood-ocular barrier, and as a result only low and transient doses of immunosuppression were used. No signs of rejection or inflammation were observed in either patient, and doctors are continuing to monitor both patients.

The results presented herein underscore the potential of stem cell therapies and regenerative medicine to realize the possibility repairing or replacing tissues damaged from disease.

The hESC-derived RPE cells underwent extensive safety studies prior to transplantation. The cells were confirmed to be free of animal and human pathogens, and a high sensitivity assay was performed to rule out the presence of any undifferentiated hESCs in the final product, a risk factor for tumor formation. Controlled hESC differentiation resulted in near-100 percent pure RPE. A central feature of hESCs is that the stage of *in vitro* differentiation can be controlled to maximize survival and functionality. The data here show that the extent of RPE maturity and pigmentation may dramatically impact subsequent attachment and growth of the cells after transplantation.

Both trials are prospective, open-label studies designed to determine the safety and tolerability of hESC-derived RPE cells following sub-retinal transplantation into patients with SMD and dry AMD at 12 months, the studies' primary endpoint. Each trial will enroll 12 patients each, with cohorts of three patients each in an ascending dosage format. Both the SMD and dry AMD patient had subretinal transplantation of the lowest dose (50,000 cells) of fully-differentiated RPE cells derived from hESCs.

In an aspect, the present disclosure provides a pharmaceutical composition comprising: a plurality of retinal pigment epithelial (RPE) cells; and a pharmaceutically acceptable carrier; wherein the average melanin content of said plurality of RPE cells is less than 8 pg/cell. Said RPE cells may be contained in a suspension, gel, colloid, matrix, substrate, scaffold, or graft.

Said pharmaceutically acceptable carrier may comprise a sterile solution having an osmolality of between about 290 mOsm/kg and about 320 mOsm/kg, or between about 300 mOsm/kg and 310 mOsm/kg or about 305 mOsm/kg. Said pharmaceutically acceptable carrier may comprise a balanced salt solution. Said balanced salt solution may comprise, consists of, or consists essentially of, in each mL, sodium chloride 7.14 mg, potassium chloride 0.38 mg, calcium chloride dihydrate 0.154 mg, magnesium chloride hexahydrate 0.2 mg, dibasic sodium phosphate 0.42 mg, sodium bicarbonate 2.1 mg, dextrose 0.92 mg, glutathione disulfide (oxidized glutathione) 0.184 mg, and hydrochloric acid and/or sodium hydroxide (to adjust pH to approximately 7.4) in water.

The volume of said pharmaceutical composition may be between about 100 µL and 1000 µL or may be at least about 150 µL. Said pharmaceutical composition may comprise between about 1,000 and about 1x10⁹ viable RPE cells. Said pharmaceutical composition may comprise between about 333 viable RPE cells/µL and about 2,000 viable RPE cells/µL, between about 444 viable RPE cells/µL and about 1766 viable RPE cells/µL, about 333 viable RPE cells/µL, about 444 viable RPE cells/µL, about 666 viable RPE cells/µL, about 888 viable RPE cells/µL, about 999 viable RPE cells/µL, or about 1,333 viable RPE cells/µL.

The concentration of RPE cells in said pharmaceutical composition may be sufficiently high that no more than about 30% of said RPE cells lose viability in 60 minutes, and optionally no more than about 10% of said RPE cells lose viability in 4 hours. Said concentration of RPE cells may be at least about 1,000 cells/µL, at least about 2,000 cells/µL, between about 1,000-10,000 cells/µL, or between about 2,000-5,000 cells/µL.

The pharmaceutical preparation may comprise less than about 25%, 20%, 15%, 10%, 5%, 1%, 0.5%, 0.1%, 0.01%, 0.001%, or 0.0001% cells that may be not RPE cells.

The average melanin content of said RPE cells may be less than 8 pg/cell, less than 7 pg/cell, less than 6 pg/cell, less than 5 pg/cell, less than 4 pg/cell, less than 3 pg/cell, less than 2 pg/cell and at least 0.1 pg/cell and optionally at least 0.5 pg/cell or 1 pg/cell; between 0.1-8 pg/cell, between 0.1-7 pg/cell, between 0.1-6 pg/cell, between 0.1-5 pg/cell, between 0.1-4 pg/cell, between 0.1-3 pg/cell, between 0.1-2 pg/cell, between 0.1-1 pg/cell, between 1-7 pg/cell, between 0.5-6 pg-cell, or between 1-5 pg/cell.

At least 50%, at least 60%, at least 70%, or at least 80% of the cells in said pharmaceutical composition may be bestrophin+. At least 80%, at least 85%, at least 90%, at least 95%, or at least 99% of the cells in said pharmaceutical composition may be PAX6+ and/or MITF+. At least 80%, at least 85%, at least 90%, at least 95%, or at least 99% of the cells in said pharmaceutical composition may be PAX6+ and/or bestrophin+. At least 80%, at least 85%, at least 90%, at least 95%, or at least 99% of the cells in said pharmaceutical composition may be ZO-1 +. At least 50%, at least 60%, or at least 70% of the cells in the pharmaceutical composition may be PAX6+ and bestrophin+. At least 90%, at least 95%, or at least 99% of the cells in said pharmaceutical composition may be PAX6+.

In an exemplary embodiment, no more than about one cell per million cells and optionally no more than two cells per nine million cells in said pharmaceutical composition may be positive for both OCT-4 and alkaline phosphatase (AP) expression.

A needle or an injection cannula may contain at least a portion of said RPE cells.The concentration of said RPE cells upon loading into said needle or injection cannula may be between about 444 viable cells/µL and about 1,766 viable cells/µL. The concentration of viable RPE cells to be delivered from said needle or injection cannula may be between about 333 viable cells/µL and about 1,333 viable cells/µL. The diameter of said needle or injection cannula may be between about 0.3 mm and about 0.9. The diameter of said needle or injection cannula may be between about 0.5 and about 0.6 mm. Said needle or injection cannula may comprise a tip having a diameter between about 0.09 mm and about 0.15 mm. Said cannula may be a MEDONE POLYTIP^{®} Cannula 25/38g (a 0.50mm (25g) x 28mm cannula with 0.12mm (38g) x 5mm tip) or a Synergetics Angled 39g Injection Cannula.

Said RPE cells may comprise RPE cells which have been cryopreserved and thawed.

Said RPE cells may be human.

The pharmaceutical composition may further comprise at least one angiogenesis inhibitor which may be administered to a subject in need thereof prior to, concurrently with, subsequent to, and/or with said RPE cells. Exemplary angiogenesis inhibitors may be selected from the group consisting of: pegaptanib sodium; aflibercept; bevasiranib; rapamycin; AGN-745; vitalanib; pazopanib; NT-502; NT-503; PLG101; CPD791; anti-VEGF antibodies or functional fragments thereof; bevacizumab; ranibizumab; anti-VEGFR1 antibodies; anti-VEGFR2 antibodies; anti-VEGFR3 antibodies; IMC-1121(B); IMC-18F1; fragments or domains of VEGF; fragments or domains of a VEGFR receptor; VEGF-Trap (Aflibercept); AZD-2171 (Cediranib); tyrosine kinase inhibitors (TKIs); TKIs that inhibit VEGFR-1 and/or VEGFR-2; sorafenib (Nexavar); SU5416 (Semaxinib); SU11248/Sunitinib (Sutent); Vandetanib (ZD 6474); Ly317615 (Enzastaurin); anti-alpha5betal integrin antibodies or functional fragments thereof; volociximab; 3-(2-{1-alkyl-5-[(pyridine-2-ylamino)-methyl]-pyrrolidin-3-yloxy}-acetylamino)-2-(alkyl-amino)-propionic acid; (S)-2-[(2,4,6-trimethylphenyl)sulfonyl]amino-3-[7-benzyloxycarbonyl-8-(2-pyridinylaminomethyl)-1-oxa-2,7-diazaspiro-(4,4)-non-2-en-3-yl]carbonylamino propionic acid; EMD478761; or RC*D(ThioP)C* (Arg-Cys-Asp-Thioproline-Cys (asterisks denote cyclizing by a disulfide bond through the cysteine residues); 2-methoxyestradiol; alphaVbeta3 inhibitors; angiopoietin 2; angiostatic steroids and heparin; angiostatin; angiostatin-related molecules; anti-cathepsin S antibodies; antithrombin III fragment; calreticulin; canstatin; carboxyamidotriazole; Cartilage-Derived Angiogenesis Inhibitory Factor; CDAI; CM101; CXCL10; endostatin; IFN-α; IFN-β; IFN-γ; IL-12; IL-18; IL-4; linomide; maspin; matrix metalloproteinase inhibitors; Meth-1; Meth-2; osteopontin; pegaptanib; platelet factor-4; prolactin; proliferin-related protein; prothrombin (kringle domain-2); restin; soluble NRP-1; soluble VEGFR-1; SPARC; SU5416; suramin; tecogalan; tetrathiomolybdate; thalidomide; lenalidomide; thrombospondin; TIMP; TNP-470; TSP-1; TSP-2; vasostatin; VEGFR antagonists; VEGI; Volociximab (M200); a fibronectin fragment or domain; anastellin; Lenvatinib (E7080); Motesanib (AMG 706); Pazopanib (Votrient); inhibitors of VEGF; inhibitors of VEGFR1; inhibitors of VEGFR2; inhibitors of VEGFR2; inhibitors of alpha5betal integrin; peptide, peptidomimetic, small molecule, chemical, and/or nucleic acid inhibitors of VEGF, VEGFR1, VEGFR2, VEGFR3, and/or alpha5betal integrin; an IL-6 antagonist; an anti-IL-6 antibody; and any combination thereof; optionally in an amount sufficient to prevent or treat proliferative (neovascular) eye disease.

TSaid RPE cells may be genetically engineered. For example, said RPE cells may be produced from a pluripotent cell that is genetically engineered. Said genetic engineering may result in production by said RPE cells of one or more factors that inhibit angiogenesis. Exemplary factors that inhibit angiogenesis include at least one factor selected from the group consisting of: a fibronectin fragment or domain; anastellin; a specific anti-VEGF antibody or a functional fragment or domain thereof; a specific anti-VEGF receptor antibody or a functional fragment or domain thereof; a specific anti-alpha5beta1 integrin antibody or a functional fragment or domain thereof; fragments or domains of VEGF; fragments or domains of a VEGFR receptor; VEGF-Trap; and any combination thereof.

Production of said factor that inhibits angiogenesis may be regulated by an RPE-specific promoter. Said RPE-specific promoter may be selected from the group consisting of: the RPE65 promoter, Cathepsin D Proximal Promoter, and the VMD2 promoter.

Said RPE cells may be produced from a pluripotent cell. Said pluripotent stem cell may be positive for expression of one or more markers may comprise OCT-4, alkaline phosphatase, Sox2, TDGF-1, SSEA-3, SSEA-4, TRA-1-60, and/or TRA-1-80. Said pluripotent cells may be human pluripotent cells that may be cultured in a multilayer population or embryoid body for a time sufficient for pigmented epithelial cells to appear in said culture. Said time sufficient for pigmented epithelial cells to appear in said culture may comprise at least about 1 week, at least about 2 weeks, at least about 3 weeks, at least about 4 weeks, at least about 5 weeks, at least about 6 weeks, or at least about 7 weeks, at least about 8 weeks. Said multilayer population or embryoid body may be cultured in a medium may comprise DMEM. Said medium may comprise, consists essentially of, or consists of EB-DM. Said pigmented epithelial cells may be isolated and cultured, thereby producing a population of RPE cells. Said isolating may comprise dissociating cells or clumps of cells from the culture enzymatically, chemically, or physically and selecting pigmented epithelial cells or clumps of cells may comprise pigmented epithelial cells. Said embryoid body may be cultured in suspension and/or as an adherent culture (e.g., in suspension followed by adherent culture). Said embryoid body cultured as an adherent culture may produce one or more outgrowths comprising pigmented epithelial cells. Said pluripotent stem cells have reduced HLA antigen complexity. Prior to RPE formation said pluripotent cells may be cultured on a matrix which may be selected from the group consisting of laminin, fibronectin, vitronectin, proteoglycan, entactin, collagen, collagen I, collagen IV, collagen VIII, heparan sulfate, Matrigel(TM) (a soluble preparation from Engelbreth-Holm-Swarm (EHS) mouse sarcoma cells), CellStart, a human basement membrane extract, and any combination thereof. Said matrix may comprise Matrigel(TM) (a soluble preparation from Engelbreth-Holm-Swarm (EHS) mouse sarcoma cells).

The pharmaceutical composition which may comprise cells that lack substantial expression of one or more embryonic stem cell markers. Said one or more embryonic stem cell markers may comprise OCT-4, NANOG, Rex-1, alkaline phosphatase, Sox2, TDGF-1, SSEA-3, SSEA-4, TRA-1-60, and/or TRA-1-80.

Said RPE cells may be positive for expression of one or more RPE cell markers. Said one or more RPE cell markers may comprise RPE65, CRALBP, PEDF, Bestrophin, MITF, Otx2, PAX2, PAX6, ZO-1, and/or tyrosinase.

Said RPE cells may be produced by a method comprising maintaining RPE cells as quiescent cells for a time sufficient to attain said average melanin content. Said RPE cells may be produced by a method comprising maintaining RPE cells as quiescent cells for a time sufficient to establish bestrophin expression in at least 50% of said RPE cells.

Said pharmaceutical composition may be substantially free of mouse embryonic feeder cells (MEF) and human embryonic stem cells (hES).

Said RPE may be produced by a method comprising culturing said RPE cells under conditions that increase expression of one or more alpha integrin subunit, e.g., alpha integrin subunit 1, alpha integrin subunit 2, alpha integrin subunit 3, alpha integrin subunit 4, alpha integrin subunit 5, alpha integrin subunit 6, or alpha integrin subunit 9. Said conditions may comprise exposure to manganese, exposure to an anti-CD29 antibody, exposure to monoclonal antibody HUTS-21, exposure to monoclonal antibody mAb TS2/16, and/or passaging said RPE cells for at least about 4 passages.

The RPE cells meet at least one of the criteria recited in Table 5 and/or manufactured in accordance with Good Manufacturing Practices (GMP).

The pharmaceutical composition may further comprise at least one immunosuppressive or immune tolerizing agent which may be administered to a subject in need thereof prior to, concurrently with, subsequent to, and/or with said RPE cells. Said immunosuppressive or immune-tolerizing agent may comprise one or more of: mesenchymal stem cells, anti-lymphocyte globulin (ALG) polyclonal antibody, anti-thymocyte globulin (ATG) polyclonal antibody, azathioprine, BASILIXIMAB^{®} (anti-IL-2Rα receptor antibody), cyclosporin (cyclosporin A), DACLIZUMAB^{®} (anti-IL-2Rα receptor antibody), everolimus, mycophenolic acid, RITUXIMAB^{®} (anti-CD20 antibody), sirolimus, tacrolimus, and mycophemolate mofetil.

In an aspect, the present disclosure provides a kit comprising a pharmaceutical composition as described above and a separate container comprising a pharmaceutically acceptable diluent in a volume sufficient to dilute said plurality of RPE cells to a desired target concentration. The volume of said pharmaceutically acceptable diluent may be such that combining the entire volume of said pharmaceutically acceptable diluent with the entirety of said plurality of RPE cells results in said plurality of RPE cells having said desired target concentration. The temperature of said pharmaceutically acceptable diluent may be between about 0-10 degrees C, optionally between about 2-8 degrees C. The temperature of said plurality of RPE cells or the pharmaceutically acceptable carrier containing said plurality of RPE cells may be between about 0-10 degrees C, optionally between about 2-8 degrees C.

The kit may further comprise at least one immunosuppressive or immune tolerizing agent which may be administered to a subject in need thereof prior to, concurrently with, subsequent to, and/or with said RPE cells, which immunosuppressive or immune tolerizing agent may include one or more of those listed above.

The kit may further comprise one or more angiogenesis inhibitors which may be administered to a subject in need thereof prior to, concurrently with, subsequent to, and/or with said RPE cells, such as one or more of the angiogenesis inhibitors listed above.

In another aspect, the present disclosure provides a cryopreserved composition comprising: a plurality of cryopreserved retinal pigment epithelial (RPE) cells having an average maturity level at the time of freezing such that the RPE cells that may be recovered subsequent to thawing having a seeding efficiency of at least about 60%. Said seeding efficiency may be at least about 70%, at least about 80%, at least about 85%, at least about 90%, or at least about 95%. Said average maturity level may be determined by measuring the average melanin content of a cell population representative of said plurality of cryopreserved RPE cells. The average melanin content of said plurality of cryopreserved RPE cells may be less than 8 pg/cell.

In an aspect, the present disclosure provides a cryopreserved composition comprising: a plurality of cryopreserved retinal pigment epithelial (RPE) cells; wherein the average melanin content of said plurality of cryopreserved RPE cells may be less than 8 pg/cell.

Said cells may be contained in a cryopreservation medium.Said cryopreservation medium may comprise one or more of DMSO (dimethyl sulfoxide), ethylene glycol, glycerol, 2-methyl-2,4-pentanediol (MPD), propylene glycol, and sucrose, e.g., between about 5% and about 50% DMSO and between about 30 % and about 95% serum, wherein said serum may be optionally fetal bovine serum (FBS). Said cryopreservation medium may comprise about 90% FBS and about 10% DMSO.

The RPE cells that may be recovered subsequent to thawing have a seeding efficiency of at least about 60%, such as at least about 70%, at least about 80%, at least about 85%, at least about 90%, or at least about 95%.

Said cryopreserved composition may comprise between about 5,000 and about 1x10⁸ viable RPE cells at the time of freezing, such as between about 200,000 and about 10,000,000, between about 20,000 and about 50,000,000, between about 250,000 and about 5,000,000, between about 500,000 and about 4,000,000, or between about 1,000,000 and about 4,000,000 viable RPE cells at the time of freezing

The the RPE cells recovered subsequent to thawing may have a seeding efficiency of at least about 60%, at least about 70%, at least about 80%, at least about 85%, at least about 90%, or at least about 95%. at a time at least about 3, 6, 9, or 12 months after freezing.

At least 85% of the cells that are viable upon thawing may remain viable stored between 2-8 degrees C for up to 1 hour, up to 2 hours, up to 3 hours, up to 4 hours, up to 5 hours, or up to 6 hours after thawing.

The cryopreserved composition may comprise less than about 25%, 20%, 15%, 10%, 5%, 1%, 0.5%, 0.1%, 0.01%, 0.001%, or 0.0001% cells that are not RPE cells.

The average melanin content of said RPE cells may be less than 8 pg/cell, less than 7 pg/cell, less than 6 pg/cell, less than 5 pg/cell, less than 4 pg/cell, less than 3 pg/cell, less than 2 pg/cell and at least 0.1 pg/cell and optionally at least 0.5 pg/cell or 1 pg/cell; between 0.1-8 pg/cell, between 0.1-7 pg/cell, between 0.1-6 pg/cell, between 0.1-5 pg/cell, between 0.1-4 pg/cell, between 0.1-3 pg/cell, between 0.1-2 pg/cell, between 0.1-1 pg/cell, between 1-7 pg/cell, between 0.5-6 pg-cell, or between 1-5 pg/cell.

At least 50%, at least 60%, at least 70%, or at least 80% of the cells in said cryopreserved composition may be bestrophin+. At least 80%, at least 85%, at least 90%, at least 95%, or at least 99% of the cells in said cryopreserved composition may be PAX6+ and/or MITF+. At least 80%, at least 85%, at least 90%, at least 95%, or at least 99% of the cells in said cryopreserved composition may be PAX6+ and/or bestrophin+. At least 80%, at least 85%, at least 90%, at least 95%, or at least 99% of the cells in said cryopreserved composition may be ZO-1+. At least 50%, at least 60%, or at least 70% of the cells in the cryopreserved composition may be PAX6+ and bestrophin+. At least 95%, or at least 99% of the cells in said cryopreserved composition may be PAX6+.

In the cryopreserved composition, optionally no more than about one cell per million cells and optionally no more than two cells per nine million cells in said cryopreserved composition may be positive for both OCT-4 and alkaline phosphatase (AP) expression.

The cryopreserved composition may further comprise at least one angiogenesis inhibitor which may be administered to a subject in need thereof prior to, concurrently with, subsequent to, and/or with said RPE cells, such as one or more of the angiogenesis inhibitors listed above.

Said RPE cells may be genetically engineered. Said RPE cells may be produced from a pluripotent cell. Said RPE cells may be produced from a pluripotent cell that may be genetically engineered. Said genetic engineering results in production by said RPE cells of one or more factors that inhibit angiogenesis. Said one or more factors that inhibit angiogenesis include at least one factor selected from the group consisting of: a fibronectin fragment or domain; anastellin; a specific anti-VEGF antibody or a functional fragment or domain thereof; a specific anti-VEGF receptor antibody or a functional fragment or domain thereof; a specific anti-alpha5beta1 integrin antibody or a functional fragment or domain thereof; fragments or domains of VEGF; fragments or domains of a VEGFR receptor; VEGF-Trap; and any combination thereof, e.g., regulated by an RPE-specific promoter such as the RPE65 promoter, Cathepsin D Proximal Promoter, and the VMD2 promoter.

Said pluripotent stem cell may be positive for expression of one or more markers comprising OCT-4, alkaline phosphatase, Sox2, TDGF-1, SSEA-3, SSEA-4, TRA-1-60, and/or TRA-1-80.

Said pluripotent cells may be human pluripotent cells that may be cultured in a multilayer population or embryoid body for a time sufficient for pigmented epithelial cells to appear in said culture.

Said time sufficient for pigmented epithelial cells to appear in said culture may comprise at least about 1 week, at least about 2 weeks, at least about 3 weeks, at least about 4 weeks, at least about 5 weeks, at least about 6 weeks, or at least about 7 weeks, at least about 8 weeks.

In an aspect, the present disclosure provides a method of producing retinal pigment epithelial (RPE) cells for use in a pharmaceutical preparation, comprising: (a)
culturing RPE cells under adherent conditions to form a substantially monolayer culture of pigmented RPE cells having a cobblestone morphology; and (b)
harvesting RPE cells from the culture for cryopreservation or pharmaceutical formulation wherein at the time of harvesting the harvested population of pigmented RPE cells have an average melanin content less than 8 pg/cell.

At least 10⁶ RPE cells may be harvested for cryopreservation or pharmaceutical formulation. Said RPE cells may be produced from pluripotent stem cells, wherein said pluripotent stem cells may be optionally human embryonic stem cells or human iPS cells.

The average melanin content may be determined for the cell population excluding the five percent of the most pigmented and the five percent of the least pigmented harvested RPE cells. Said average melanin content is between 1-5 pg/cell.

In an aspect, the present disclosure provides a method of producing retinal pigment epithelial (RPE) cells for use in a pharmaceutical preparation, comprising: (a)
culturing RPE cells under adherent conditions to form a substantially monolayer culture of pigmented RPE cells having a cobblestone morphology; (b) passaging the RPE cells at least once at a time prior to the RPE cells reaching an average melanin content greater than 8 pg/cell; and (c)optionally, after the one or more passages, harvesting RPE cells for cryopreservation or pharmaceutical formulation, wherein, at the time of harvesting, said RPE cells have an average melanin content of less than 8 pg/cell.

In an aspect, the present disclosure provides a method of producing retinal pigment epithelial (RPE) cells, comprising: (a) culturing pluripotent stem cells to form embryoid bodies (EBs) or culturing pluripotent stem cells to form a multilayer population, wherein said pluripotent stem cells may be optionally human embryonic stem cells or human iPS cells; (b) culturing the multilayer population of cells or EBs for a sufficient time for the appearance of pigmented cells may comprise brown pigment dispersed in their cytoplasm; and (c) isolating and culturing the pigmented cells of (b) to produce a cultured population containing RPE cells having an average pigmentation level of. Step (b) may comprise culturing said embryoid bodies to form an adherent culture. Step (a) may comprise allowing a culture of pluripotent cells to overgrow, thereby forming a multilayer population. Step (a) may comprise culturing said pluripotent cells on a low-adherent substrate or culturing said pluripotent cells using a hanging drop method, thereby forming embryoid bodies from said pluripotent cells. Said pluripotent stem cells may be induced pluripotent stem (iPS) cells, embryonic stem (ES) cells, adult stem cells, hematopoietic stem cells, fetal stem cells, mesenchymal stem cells, postpartum stem cells, multipotent stem cells, or embryonic germ cells. The pluripotent stem cells may be human ES cells or human iPS cells. The pluripotent stem cells may be genetically engineered. Said genetic engineering results in production by said RPE cells of a factor that inhibits angiogenesis, such as those identified above. The culture medium in which the embryoid bodies may be formed in step (a) and/or the pigmented cells may be cultured in step (c) may comprise DMEM. The embryoid bodies may be formed step (a) and/or the pigmented cells may be cultured in step (c) may comprise, consists essentially of, or consists of EB-DM. The medium in which said pigmented cells may be cultured in step (c) may comprise EB-DM. Said pigmented epithelial cells may be cultured in step (c) may comprise, consists essentially of, or consists of RPE-GM/MM. The duration of culturing in step (b) may be at least about 1, 2, 3, 4, 5, 6, 7, or 8 weeks, or at least about 1, 2, 3, 4, 5, or 6 months. The culture medium used in step (a), (b), or (c), may be EB-DM, RPE-GM/MM, MDBK-GM, OptiPro SFM, VP-SFM, EGM-2, or MDBK-MM. Step (c) may comprise contacting the culture with an enzyme selected from the group consisting of trypsin, collagenase, dispase, papain, a mixture of collagenase and dispase, and a mixture of collagenase and trypsin, or may comprise mechanical disruption or isolation of the culture, or may comprise contacting the culture with EDTA or EGTA, thereby disrupting adhesion of said pigmented cells to the culture substrate. The pluripotent stem cells have reduced HLA antigen complexity. The RPE cells may lack substantial expression of one or more embryonic stem cell markers. Said one or more embryonic stem cell markers may be Oct-4, NANOG, Rex-1, alkaline phosphatase, Sox2, TDGF-1, DPPA-2, and/or DPPA-4.

The embryoid bodies may be cultured as adherent cultures subsequent to their formation, for example to permit outgrowths to grow. The RPE cells may be positive for at least one RPE cell marker. Said at least one RPE cell marker includes one or more of RPE65, CRALBP, PEDF, Bestrophin, MITF, Otx2, PAX2, PAX6, or tyrosinase or optionally PAX6 and bestrophin.

The method may further comprise culturing said RPE cells under conditions that increase alpha integrin subunit expression, e.g., as described above

The said EBs may be formed in the presence of a rho-associated protein kinase (ROCK) inhibitor, wuch as Y-27632. Prior to said RPE formation said pluripotent cells may be cultured on Matrigel(TM) (a soluble preparation from Engelbreth-Holm-Swarm (EHS) mouse sarcoma cells).

In an aspect, the present disclosure provides a pharmaceutical preparation comprising RPE cells suitable for treatment of retinal degradation, wherein said RPE cells contain an average melanin content of less than 8 pg/cell, and wherein said RPE cells may have at least one of the following properties: maintain their phenotype after transplantation for at least about one month, maintain their phenotype in culture for at least about one month, integrate into the host after transplantation, do not substantially proliferate after transplantation, may be phagocytositic, deliver, metabolize, or store vitamin A, transport iron between the retina and choroid after transplantation, attach to the Bruch's membrane after transplantation, absorb stray light after transplantation, have elevated expression of alpha integrin subunits, have greater average telomere length than RPE cells derived from donated human tissue, have greater replicative lifespan in culture than RPE cells derived from donated human tissue, have greater expression of one or more alpha integrin subunits than RPE cells derived from donated human tissue, have lower A2E content than RPE cells derived from donated human tissue, have lower lipofuscin content than RPE cells derived from donated human tissue, exhibit less accumulated ultraviolet damage than RPE cells derived from donated human tissue, or contain a greater number of phagosomes than RPE cells derived from donated human tissue. In an aspect, the present disclosure provides a pharmaceutical preparation may comprise RPE cells suitable for treatment of retinal degradation, wherein said RPE cells contain an average melanin content of less than 8 pg/cell, and wherein said RPE cells have at least one of the following properties: attach to the Bruch's membrane after transplantation, absorb stray light after transplantation, have greater average telomere length than RPE cells derived from donated human tissue, have greater replicative lifespan in culture than RPE cells derived from donated human tissue, have lower A2E content than RPE cells derived from donated human tissue, have lower lipofuscin content than RPE cells derived from donated human tissue, exhibit less accumulated ultraviolet damage than RPE cells derived from donated human tissue, or contain a greater number of phagosomes than RPE cells derived from donated human tissue.

In an aspect, the present disclosure provides a method of treatment of a retinal degenerative condition, comprising administering a pharmaceutical preparation comprising administering the RPE cells of a composition or kit, a pharmaceutical preparation or manufactured according to the method as described above, to the eye of a subject in need thereof in an amount effective to treat said retinal degenerative condition.

The retinal degenerative condition may comprise choroideremia, diabetic retinopathy, age-related macular degeneration (dry or wet), retinal detachment, retinitis pigmentosa, Stargardt's Disease, Angioid streaks, or Myopic Macular Degeneration. Said step of administering may comprise intraocular administration of said RPE cells into an eye in need thereof. Said intraocular administration may comprise injection of said RPE cells into the subretinal space. Said intraocular administration may comprise injection of an aqueous solution, optionally an isotonic solution and/or a saline solution, into the subretinal space, thereby forming a pre-bleb, and removal of said aqueous solution, prior to administration of said RPE cells into the same subretinal space as said aqueous solution. Said injection may be through a needle or injection cannula. The diameter of said needle or injection cannula may be between about 0.3 mm and 0.9 mm or between about 0.5 and about 0.6 mm. Said needle or injection cannula may comprise a tip having a diameter between about 0.09 mm and about 0.15 mm. Said cannula may be a MEDONE POLYTIP^{®} Cannula 25/38g (a 0.50mm (25g) x 28mm cannula with 0.12mm (38g) x 5mm tip). The effectiveness of treatment may be assessed by determining the visual outcome by one or more of: slit lamp biomicroscopic photography, fundus photography, IVFA, and SD-OCT, and best corrected visual acuity (BCVA). The method may produce an improvement in corrected visual acuity (BCVA) and/or an increase in letters readable on the Early Treatment Diabetic Retinopathy Study (ETDRS) visual acuity chart. The condition of retinal degeneration may be dry AMD or Stargardt's Disease

The amount effective to treat said retinal degenerative condition may be at between about 20,000-200,000 RPE cells, between about 20,000-500,000 RPE cells, between about 20,000-2,000,000 RPE cells, or at least about 20,000 RPE cells, or at least about 20,000, 50,000, 75,000, 100,000, 125,000, 150,000, 175,000, 180,000, 185,000, 190,000, 200,000, or 500,000 RPE cells.

Said subject may be not administered a corticosteroid prior to or concurrently with said administration of said RPE cells, such as prednisolone or methylprednisolone. Said subject may be not administered a corticosteroid within at least 3, 6, 12, 24, 48, 72, or 96 hours prior to said administration of said RPE cells or concurrently with said administration of said RPE cells. Said subject may be not administered a corticosteroid within at least 1 hour prior to said administration of said RPE cells or immediately prior to or concurrently with said administration of said RPE cells. Said subject may be not administered a corticosteroid within at least 12, 24, 48, 72, or 96 hours subsequent to said administration of said RPE cells. Said subject may be not administered a corticosteroid within at least 48 hours subsequent to said administration of said RPE cells.

Said RPE cells may be administered to a patient in combination with one or more agents selected from the group consisting of: angiogenesis inhibitors, antioxidants, antioxidant cofactors, other factors contributing to increased antioxidant activity, macular xanthophylls, long-chain omega-3 fatty acids, amyloid inhibitors, CNTF agonists, inhibitors of RPE65, factors that target A2E and/or lipofuscin accumulation, downregulators or inhibitors of photoreceptor function and/or metabolism, α2-adrenergic receptor agonists, selective serotonin 1A agonists, factors targeting C-5, membrane attack complex (C5b-9) and any other Drusen component, immunosuppressants, and agents that prevent or treat the accumulation of lipofuscin.

Said one or more agents may be administered to said patient concurrently with, prior to, and/or subsequent to said preparation of RPE cells.

Said composition, kit, or pharmaceutical preparation may be used in the manufacture of a medicament for the treatment of a retinal degenerative condition, such as Choroideremia, diabetic retinopathy, dry age-related macular degeneration, wet age-related macular degeneration, retinal detachment, retinitis pigmentosa, Stargardt's Disease, angioid streaks, or myopic macular degeneration.

Said pluripotent stem cells express one or more markers selected from the group consisting of: OCT-4, alkaline phosphatase, SSEA-3, SSEA-4, TRA-1-60, and TRA-1-80.

Said RPE cells exhibit one or more of the following characteristics: a replicative lifespan that may be greater than the replicative lifespan of RPE cells obtained from other sources; an average telomere length that may be at least 30 percent of the telomere length of a hESC and/or human iPS cell (or the average of a population of hESC and/or human iPS cells), or at least 40, 50, 60, 70 80 or 90 percent of the telomere length of an hESC and/or human iPS cell; a mean terminal restriction fragment length (TRF) that may be longer than 4 kb, or longer than 5, 6, 7, 8, 9, 10, 11, 12 or even 13kb, or 10kb or longer; an average lipofuscin content that may be less than 50 percent of the average lipofuscin content of the equivalent number of RPE cells isolated from adult eyes, or less than 40, 30, 20 or 10 percent of the average lipofuscin content of the equivalent number of RPE cells isolated from adult eyes; an average N-retinylidene-N-retinylethanolamine (A2E) content that may be less than 50 percent of the average A2E content of the equivalent number of RPE cells isolated adult eyes, or less than 40, 30, 20 or 10 percent of the average A2E content of the equivalent number of RPE cells isolated from adult eyes; an average N-retinylidene-N-retinylethanolamine (A2E) content that may be less than 50ng per 10⁵ (100,000) cells; a rate of phagocytosis of photoreceptor outer segments (POS) that may be at least 50 percent greater than the rate of phagocytosis of POS for an equivalent number of RPE cells isolated adult eyes, or at least than 75, 100, 150 or 200 percent greater than the rate of phagocytosis of POS for an equivalent number of RPE cells isolated adult eyes; rate of phagocytosis of photoreceptor outer segments (POS) that may be at least 20 percent of the total concentration of POS after 24 hours, or at least than 25, 30, 25, 40 or 50 percent of the total concentration of POS after 24 hours; a decreased level of accumulated oxidative stress and/or DNA damage compared to RPE cells isolated from an adult host; an average proteasome activity that may be at least 50 percent greater than the average proteosome activity of the equivalent number of RPE cells isolated adult eyes, or at least 60, 70, 80, 90 or 100 percent greater than the average proteosome activity of the equivalent number of RPE cells isolated from adult eyes; an average accumulation of ubiquitin conjugates that may be less than 50 percent of the average accumulation of ubiquitin conjugates for an equivalent number of RPE cells isolated adult eyes, or less than 40, 30, 20 or even 10 percent of the average accumulation of ubiquitin conjugates of the equivalent number of RPE cells isolated from adult eyes.

### BRIEF DESCRIPTION OF THE DRAWINGS

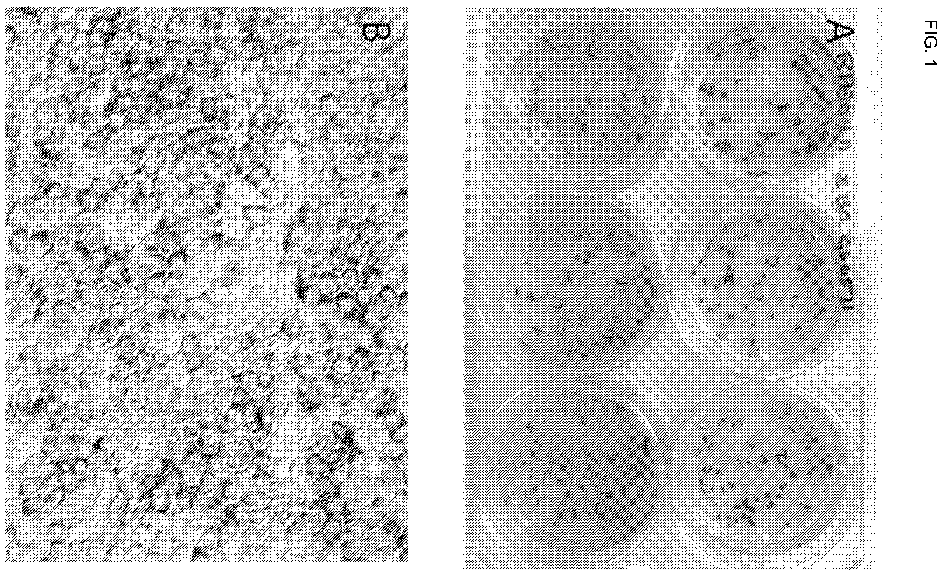

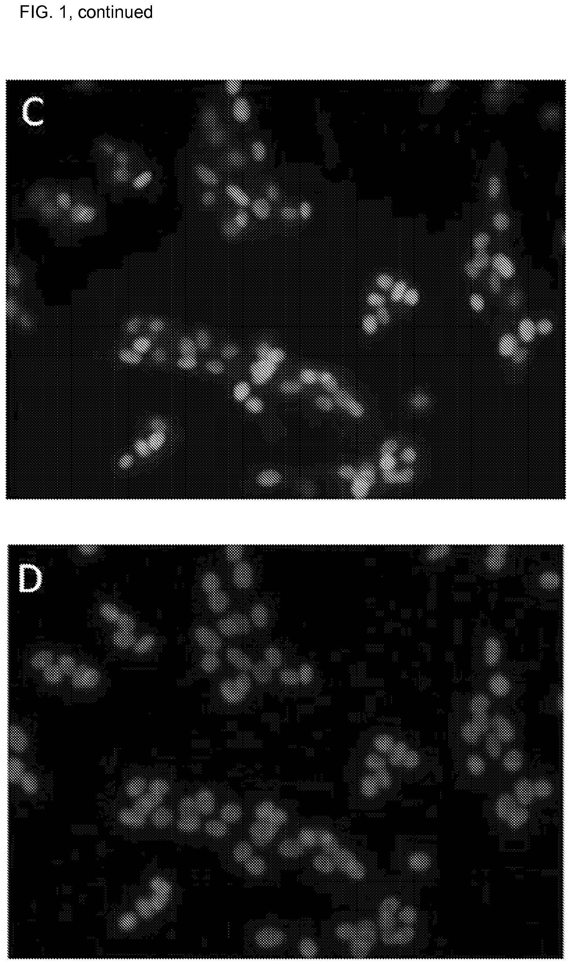

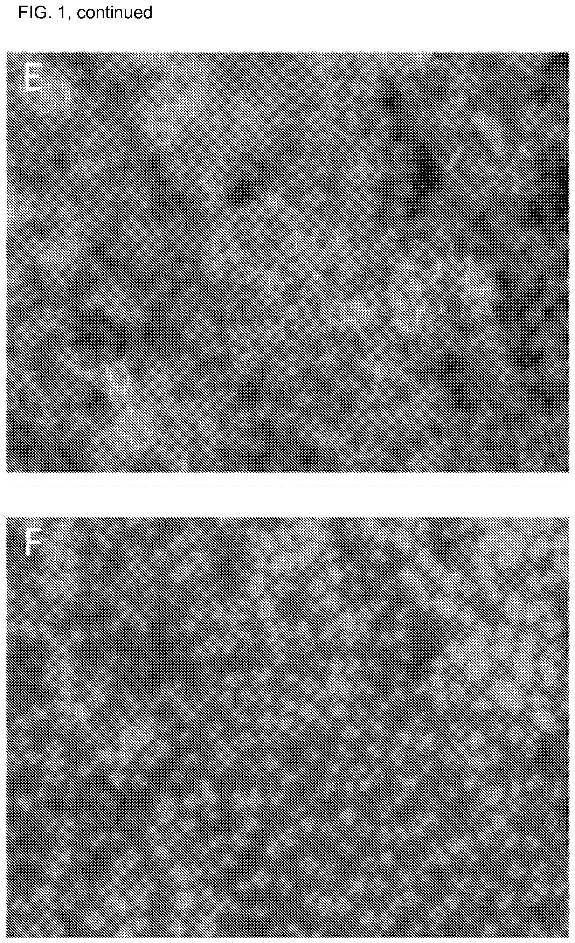

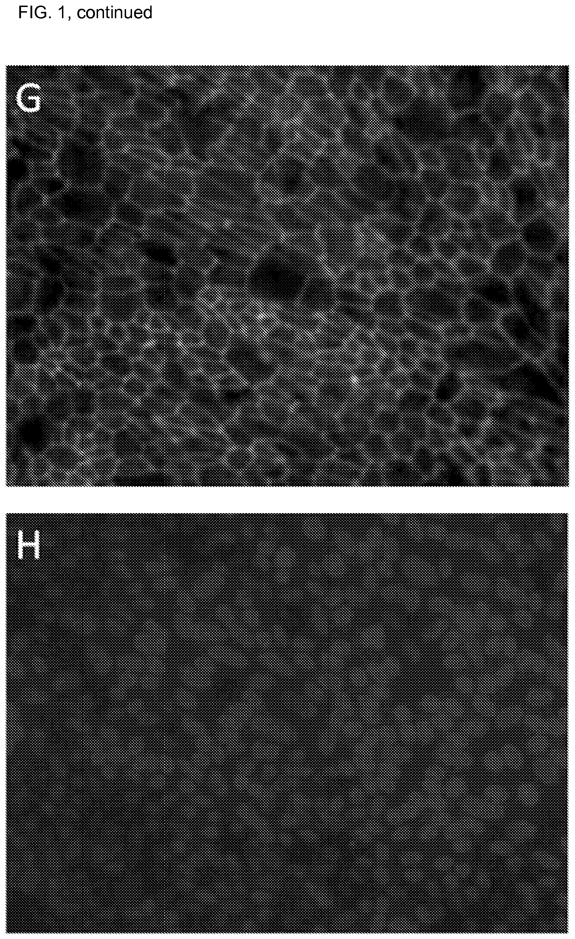

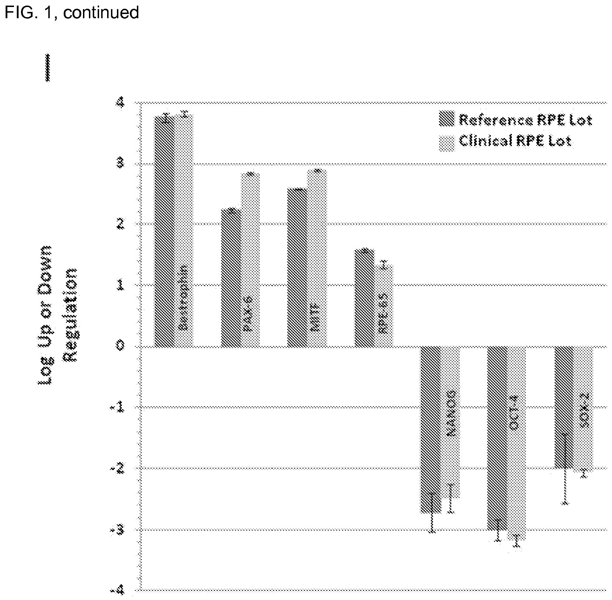

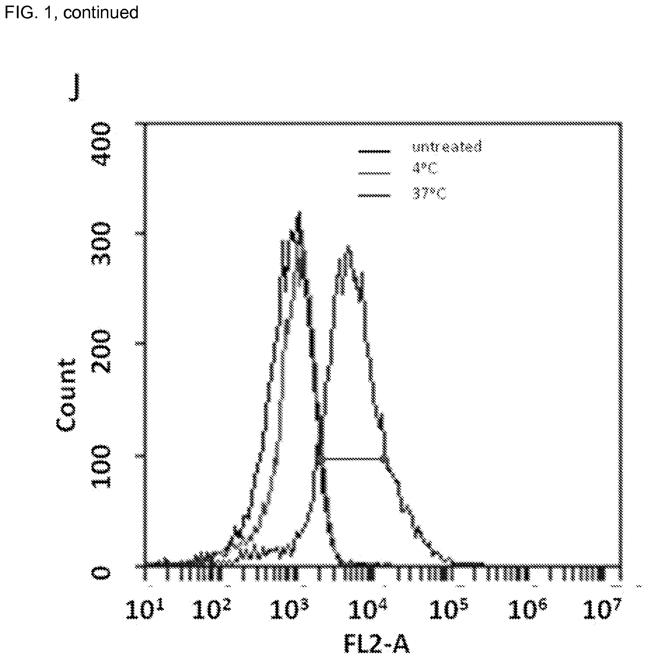

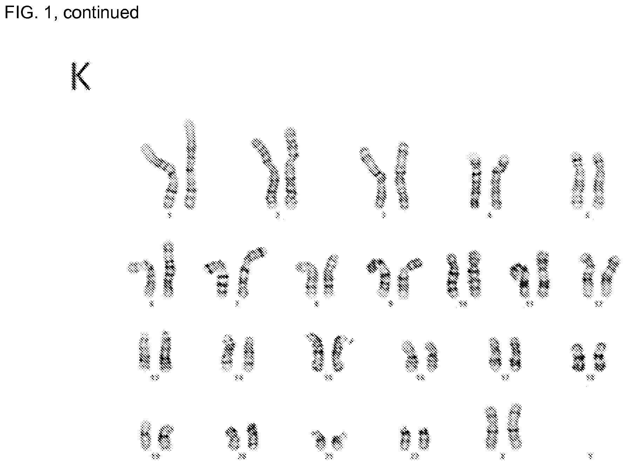

**FIG. 1**. Characterization of RPE generated from hESC MA09. A, a six-well plate showing pigmented patches of RPE formed in differentiating culture of embryoid bodies, B -H, assessment of molecular markers in thawed and formulated RPE. MITF and PAX6 (C-D) were assessed in overnight cultures of freshly formulated cells and bestrophin/PAX6 and ZO-1 immunostaining was performed on 3 week old cultures. B - HMC microphotography of 3-week old RPE post-formulation showing that the confluent cobblestone monolayer with medium pigmentation has been established. C - MITF/PAX6 merged (MITF-red in originals, PAX6 - green in originals), D - DAPI corresponding to MITF/PAX6; E - bestrophin/PAX6 merged, F - corresponding DAPI; G - ZO-1, H-corresponding DAPI. Note near 100% cells in C-H are positive for the marker(s) assessed. Magnification, x400 (B-H). I -q-PCR showing upregulation of RPE markers and down-regulation of hESC markers in the thawed clinical RPE (right panel in each group, green in original) compared to a reference RPE lot (left panel in each group, blue in original). Genes shown (in order from left to right) are: Bestrophin, Pax-6, MITF, RPE-65, NANOG, OCT-4, and SOX-2. J, FACS showing phagocytosis of PhRodo bioparticles by hES-RPE at 37°C and at 4°C (control). Shown are untreated cells (black line in original; leftmost curve) and 4°C control cells (red line in original; left part of the curve rises slightly to the right of the untreated cells curve and right part of the curve overlaps with the right part of the untreated cells curve), and 37°C treated cells (blue line in original; rightmost curve). K - normal female (46 XX) karyotype of the clinical RPE lot.

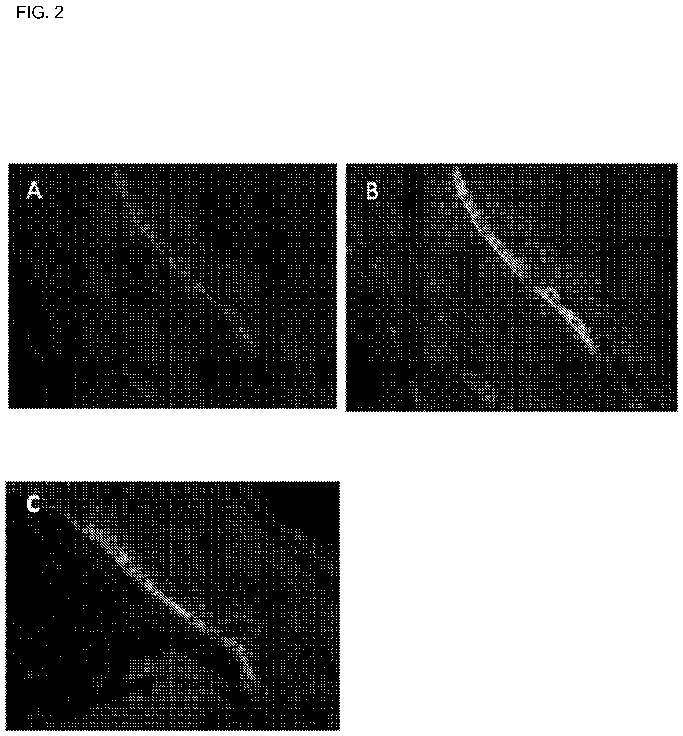

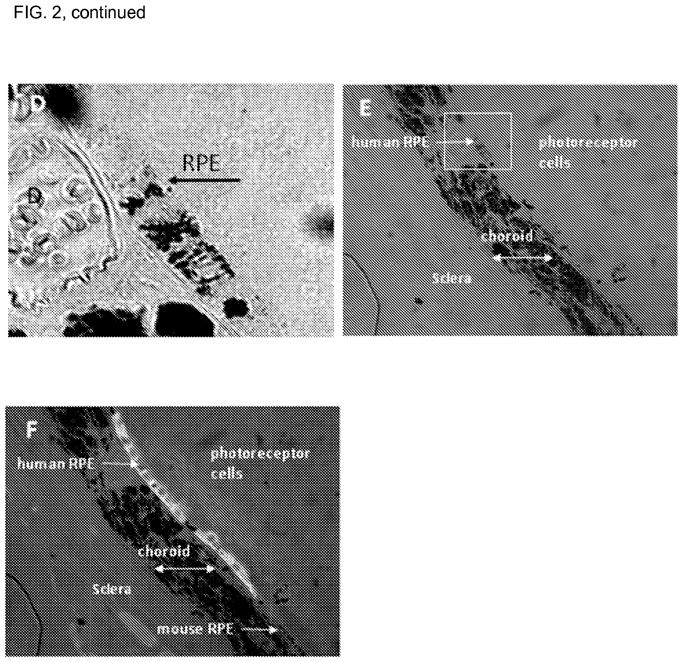

**FIG. 2**. Survival and integration of RPE generated from hESC-MA09 into NIH III mouse eye after nine months. Section stained with anti-human mitochondria (A, red in original) and anti-human bestrophin (B, green in original). Notice precise colocalization of human mitochondria and bestrophin staining in the same cells (C: A and B merged) and absence of staining in mouse RPE (F: A, B, C, E merged). Frame on the bright field image (E) is enlarged in "D" to show morphology of human RPE. Magnification 200x (A-C, E, F), D is additionally magnified x4.5.

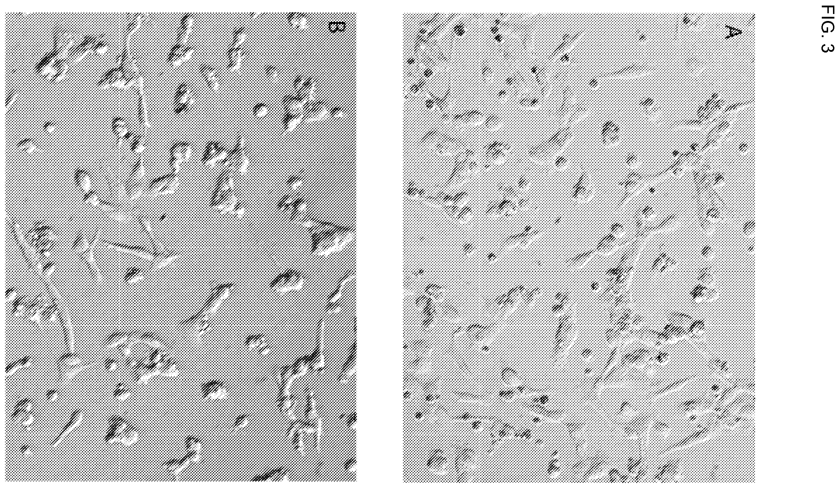

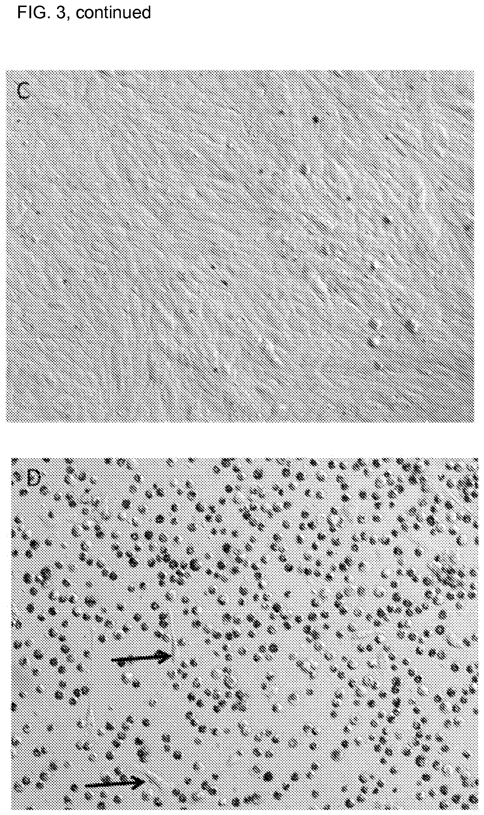

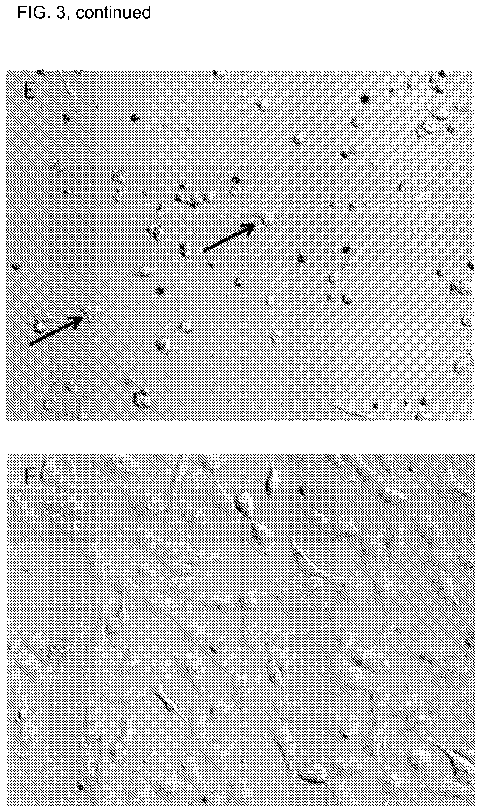

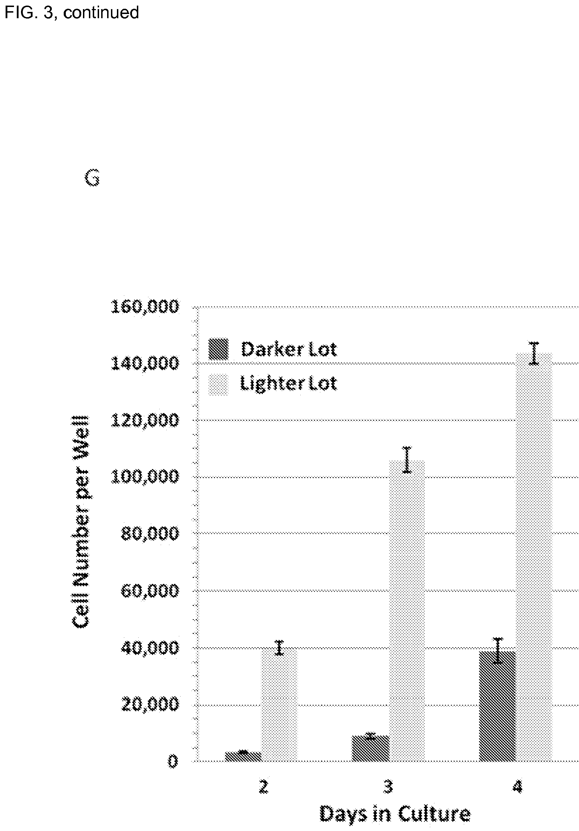

**FIG. 3**. Difference in attachment and growth of RPE with different degrees of pigmentation. Micrographs show attachment and behavior of lighter (A-C) and darker (D-F) pigmented lots. G illustrates the growth rate of RPE for the darker lots (left panel in each group) and lighter lots (right panel in each group) showing the total number of cells per well for three consecutive days after plating. A and D show the total cell population at 21 h after plating, B and E show the same cultures as in A and D after removal of the floating cells, C and F show same cultures three days after plating. Note that the majority of the cells had attached at 21 h post-thaw in the lighter pigmented lot (A, B, G) while only a few cells of the darker pigmented lot had attached (D, E, G arrows), and most cells were floating. After three days in culture the lighter pigmented lot (C) had a greater number of cells, and a confluent monolayer was established, while the darker pigmented lot was still under-confluent. Magnification, x200.

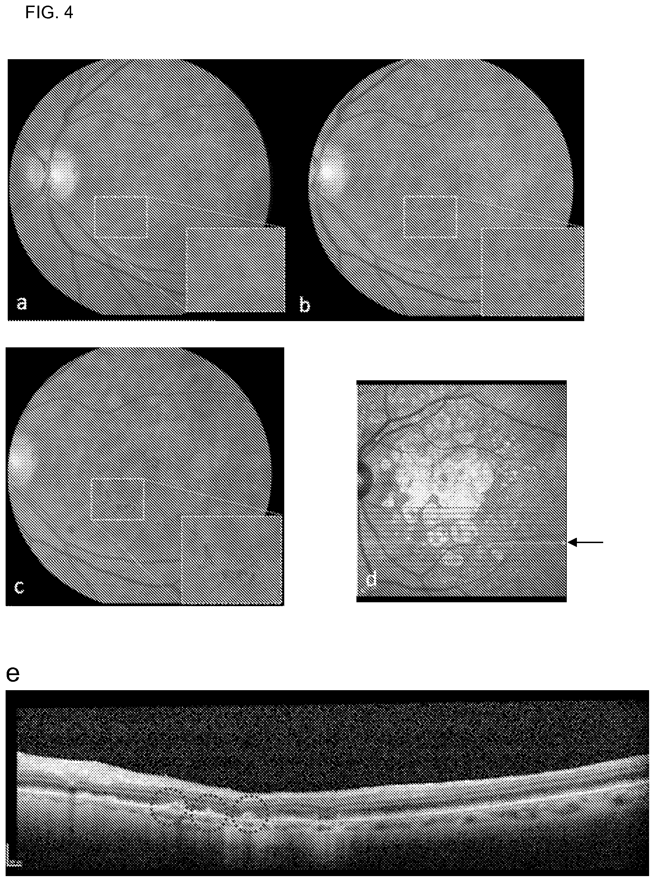

**FIG. 4****.** Images of the hESC-derived RPE transplantation sites. Color fundus photographs of an SMD patient's left macula pre- and post-operatively (A-C). The area inside the rectangle bisects the border of the surgical transplantation site and corresponds to macular atrophy not included in the surgical injection. A - Baseline macular color image with widespread RPE and neurosensory macular atrophy. B - Color macular image one week after hESC-RPE transplantation. Notice the mild pigmentation most evident in the area of baseline RPE atrophy. This pigmentation increased at week 6 (C). Panels D and E show a spectral domain ocular coherence tomograph (SD-OCT) and registration black and white photograph (Hiedelberg Engineering). The cross sectional view shown in panel E corresponds to the horizontal line (bright green in original) indicated by an arrow in panel D. The dashed circles in panel E (red in original) highlight what appear to be hESC-RPE cells settling on or attached to the compromised native RPE layer.

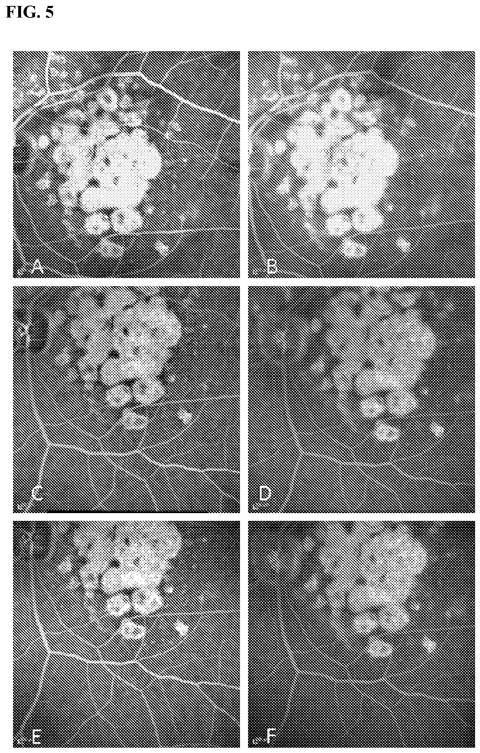

**FIG. 5****.** Fluorescein angiography images of AMD patient. No evidence of leakage is noted at the different time intervals. A: baseline early phase, B: baseline late phase, C: 4 weeks early phase, D: 4 weeks late phase, E: 8 weeks early phase, F: 8 weeks late phase. (images selected are decentered inferiorly to represent area of the transplant).

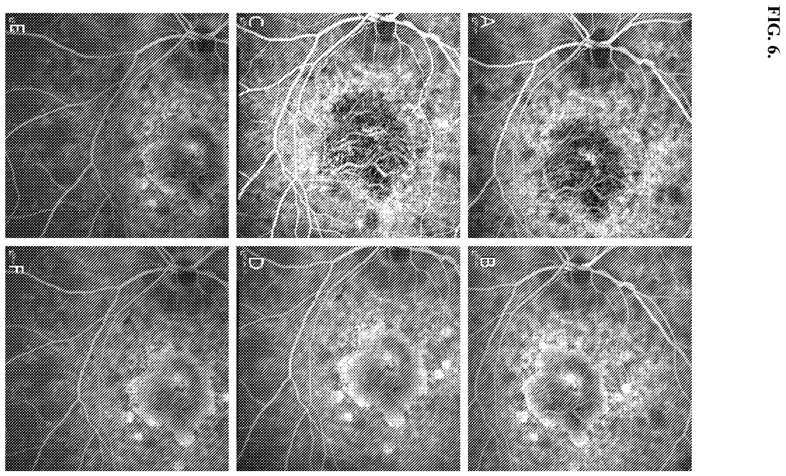

**FIG. 6****.** Fluorescein angiography images of a Stargardt's patient. No evidence of leakage is noted at the different time intervals. A: baseline early phase, B: baseline late phase, C: 4 weeks early phase, D: 4 weeks late phase, E: 8 weeks early phase, F: 8 weeks late phase. (images selected are decentred inferiorly to represent area of the transplant).

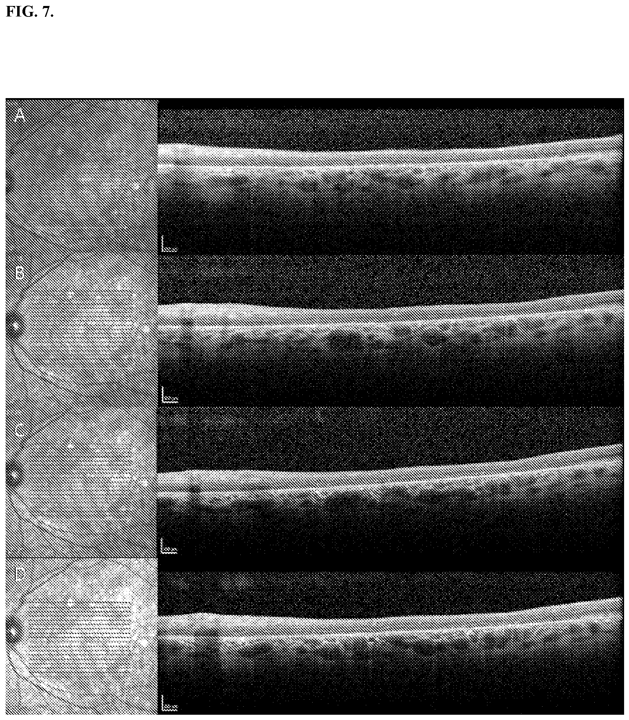

**FIG. 7****:** OCT images of a Stargardt's patient at different time intervals. No evidence of edema or subretinal fluid was noted in any of the images at the different time periods. (OCT selected represent area of the transplant). Panel A: baseline, B: 1 week, C: 4 weeks, D: 8 weeks.

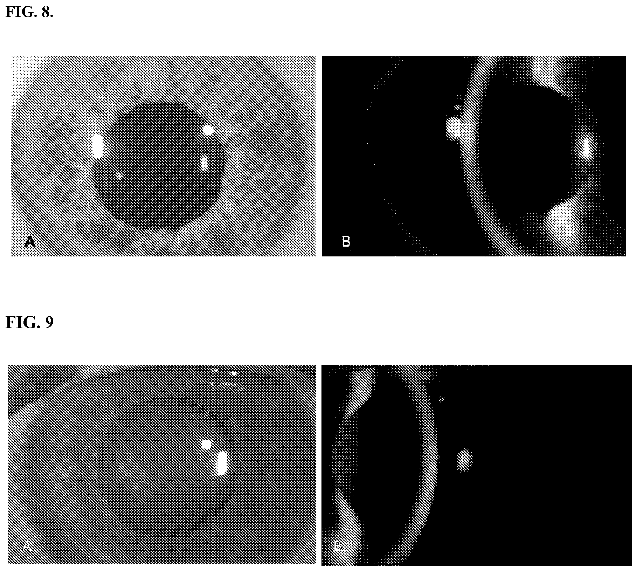

**FIG. 8****.** Slit lamp images of AMD patient. Panels A and B: 1 week post op. No evidence of anterior segment inflammation or corneal edema is noted.
**FIG. 9****.** Slit lamp images of a Stargardt's patient. Panels A and B: 1 week post op. No evidence of anterior segment inflammation or corneal edema is noted.

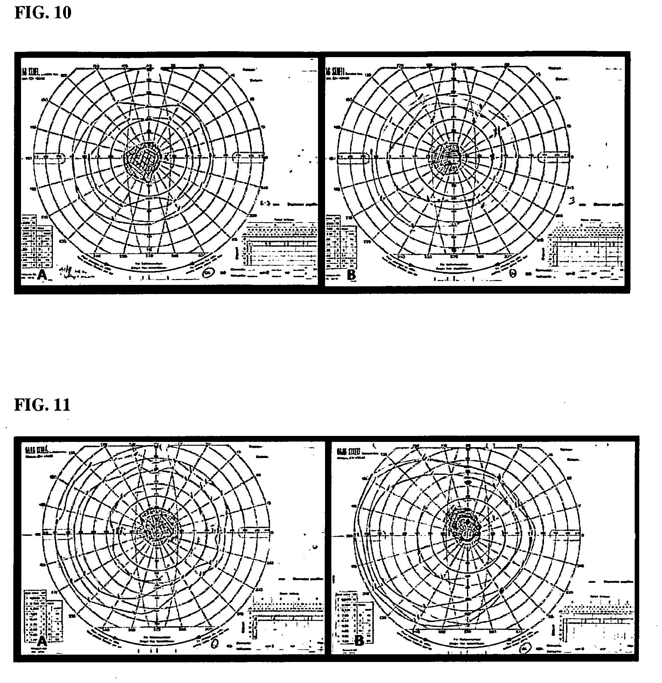

**FIG. 10****.** Goldmann Visual fields done on an AMD patient: panel A: baseline, panel B: 6 weeks. Slightly smaller central scotoma is observed.
**FIG. 11****.** Goldmann Visual fields done on a Stargardt's patient: panel A: baseline, panel B: 6 weeks. Minimal diminution of the scotoma is observed.

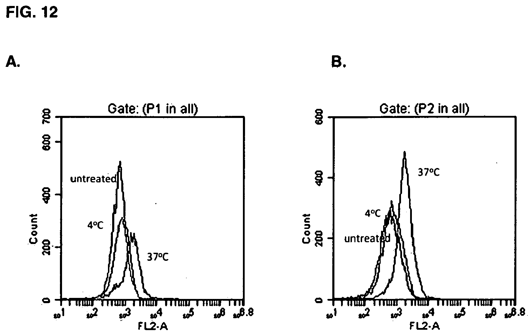

**FIG. 12****.** Phagocytosis assay results for two lots of RPE cells produced using different media. RPE were produced using either MDBK media (panel A) or EB-DM and RPE-GM/MM (panel B). Results are presented as histograms from FACS analyses for cells incubated without fluorescent bioparticles ("untreated"), negative control cells incubated with fluorescent bioparticles at 4 degrees C ("4°C"), and cells incubated with fluorescent bioparticles at 37 degrees C ("37°C").

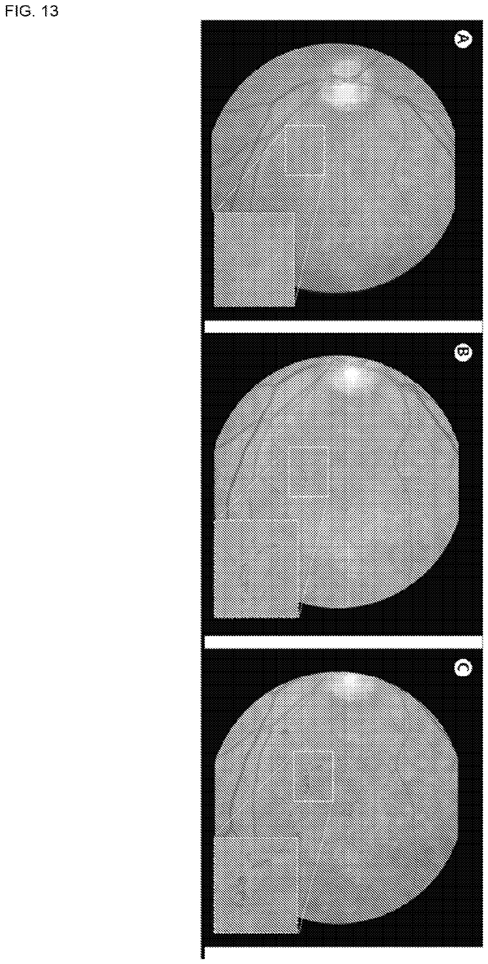

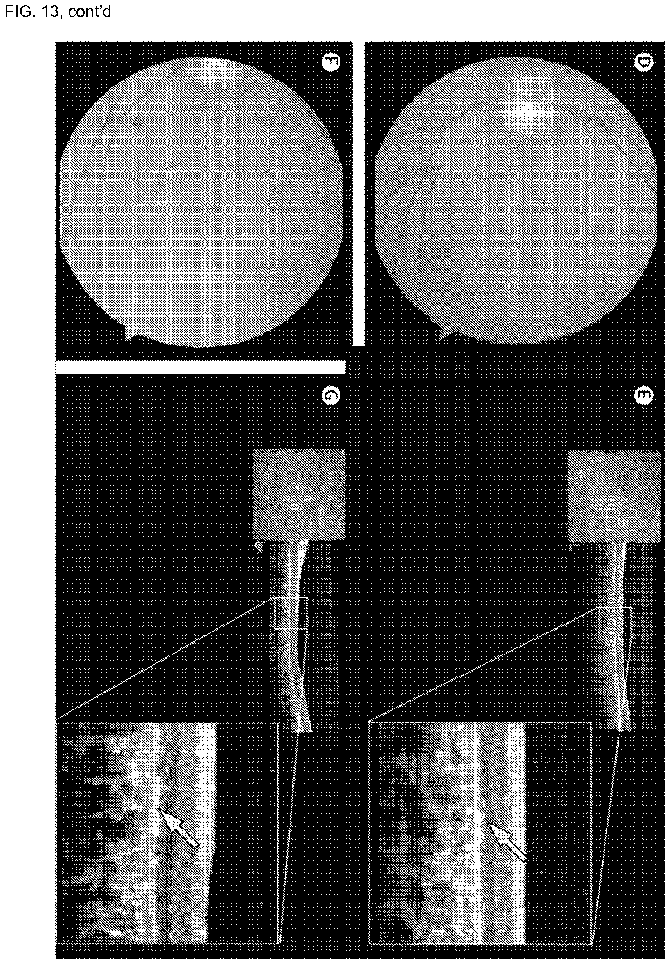

**FIG. 13****.** Images of the hESC-RPE transplantation site in a patient with Stargardt's macular dystrophy. Color fundus photographs of the patient's left macula preoperatively and postoperatively (A-C). The region inside the rectangle bisects the border of the surgical transplantation site and corresponds to macular atrophy not included in the surgical injection. (A) Baseline macular color image with widespread RPE and neurosensory macular atrophy. (B) Color macular image 1 week after hESC-RPE transplantation. Note the mild pigmentation most evident in the region of baseline RPE atrophy. This pigmentation increased at week 6 (C). (D-G) Color fundus photographs and SD-OCT images at baseline (D) and month 3 after transplant (F). Color images show increasing pigmentation at the level of the RPE from baseline to month 3. Registered SD-OCT images (E, G) show increasing pigmentation is at the level of the RPE, normal monolayer RPE engraftment, and survival at month 3 (arrow) adjacent to region of bare Bruch's membrane devoid of native RPE. hESC=human embryonic stem cells. RPE=retinal pigment epithelium. SD-OCT=spectral domain ocular coherence tomography.

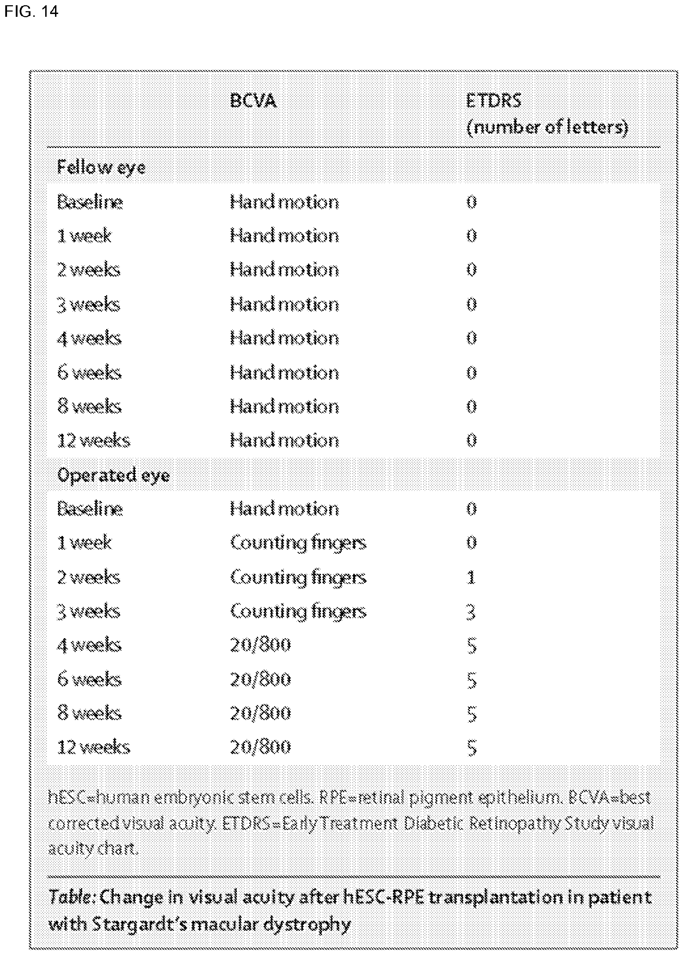

FIG. 14. Tabular summary of change in visual acuity after hESC-RPE transplantation in patient with Stargardt's macular dystrophy in the untreated eye ("Fellow eye") and the eye into which RPE cells were injected ("Operated eye") for an SMD patient. The operated eye showed improvement detectable by ETDRS and BCVA, whereas there was no detected change in visual acuity in the untreated eye. hESC=human embryonic stem cells. RPE=retinal pigment epithelium. BCVA=best corrected visual acuity. ETDRS=Early Treatment Diabetic Retinopathy Study visual acuity chart.

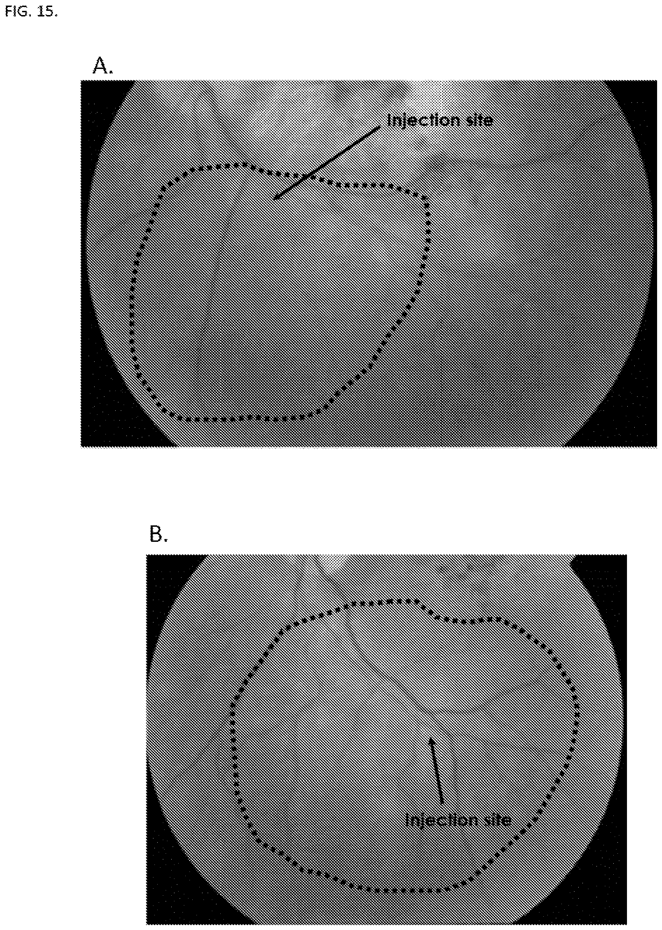

FIG. 15 shows shows two fundus photographs including the retina, optic disc, macula, and posterior pole for two additional Stargardt's patients, each treated with 50,000 RPE cells derived from an hESC source. Each photo indicates the site of injection and the area of the bleb created upon injection of the solution containing the RPE cells.

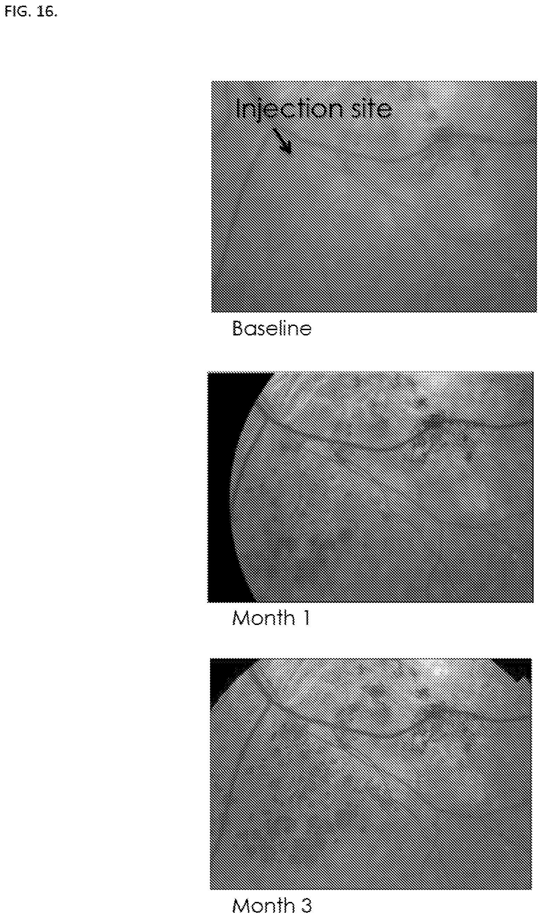

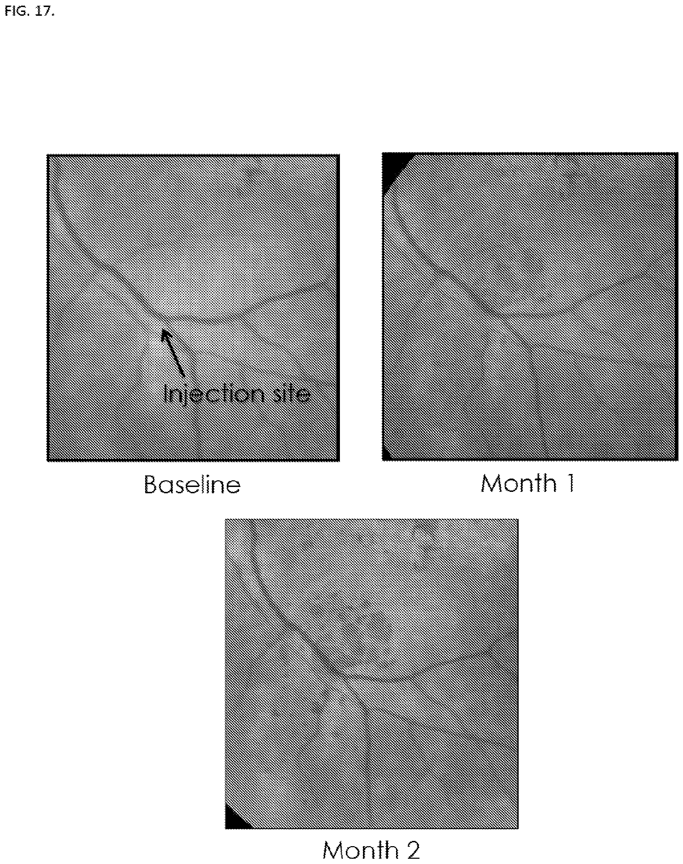

FIGs. 16 and 17 (two different additional SMD patients show three fundus photographs each, taken at the indicated time points for each patient (i.e., at baseline, 1 month, and two or three months), showing the establishment of areas within the injection bleb which have increasing patches of pigmented RPE cells, suggesting the engraftment and resurfacing of areas of the retina with a new RPE layer.

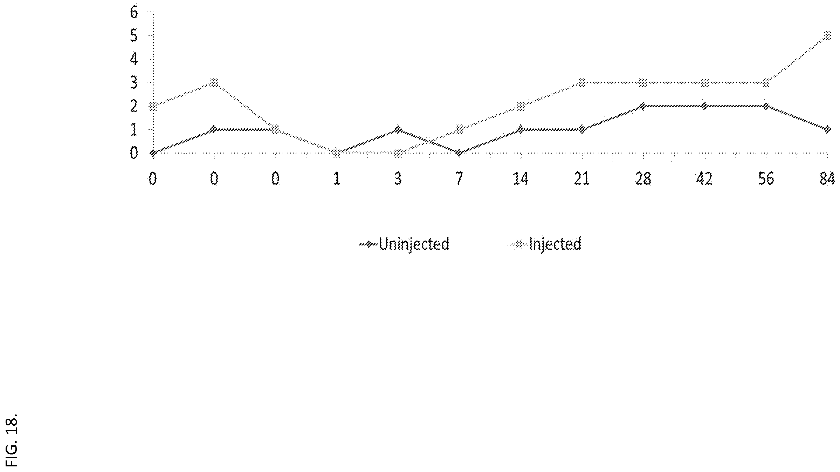

FIG. 18 shows the measured visual acuity in the treated ("injected") and untreated ("uninjected") eye of the patient shown in the top panel of FIG. 16 and in FIG. 17. The vertical axis indicates Early Treatment Diabetic Retinopathy Study (ETDRS score and the horizontal axis shows the number of days postsurgery.

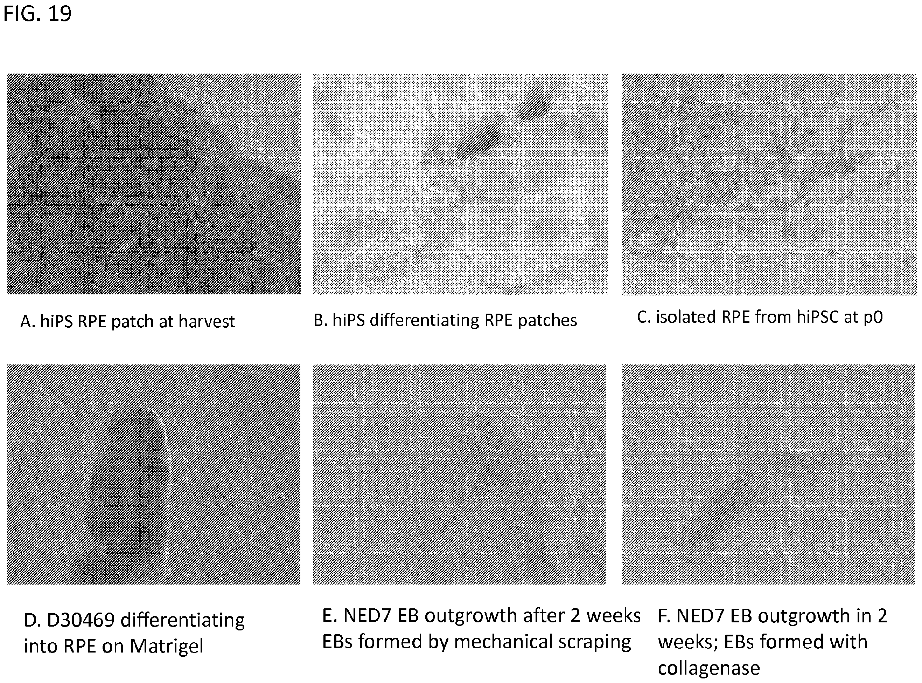

FIG. 19. Visible light migrographs illustrate expected pigmentation and morphology of RPE cultures produced from hESC which were generated without embryo destruction. The upper three panels (A, B, C) show RPE produced from three different human iPS (hiPS) cell lines. The lower three panels (D, E, F) show RPE produced from hES cells generated from biopsied blastomeres (cell lines designated D30469 and NED7), wherein the remaining embryo remained viable and was cryopreserved.

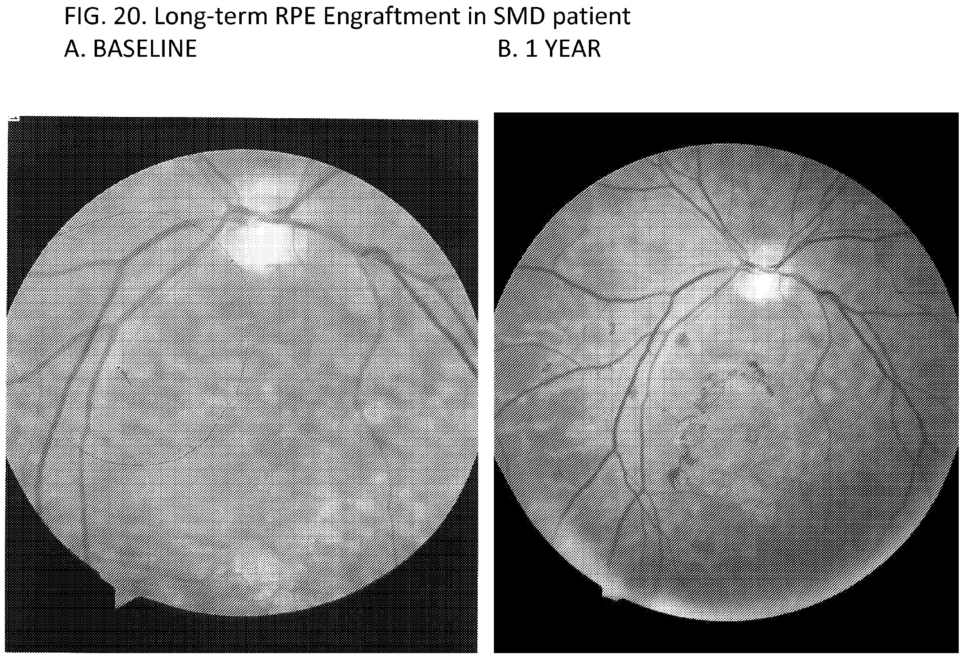

FIG. 20. Long-term RPE engraftment in the same SMD patient shown at earlier time points in FIG. 4. Fundus photographs taken (A) at baseline and (B) one year after RPE injection show the presence of pigmented cells, indicating long-term engraftment of RPE cells persisting for at least one year after injection.

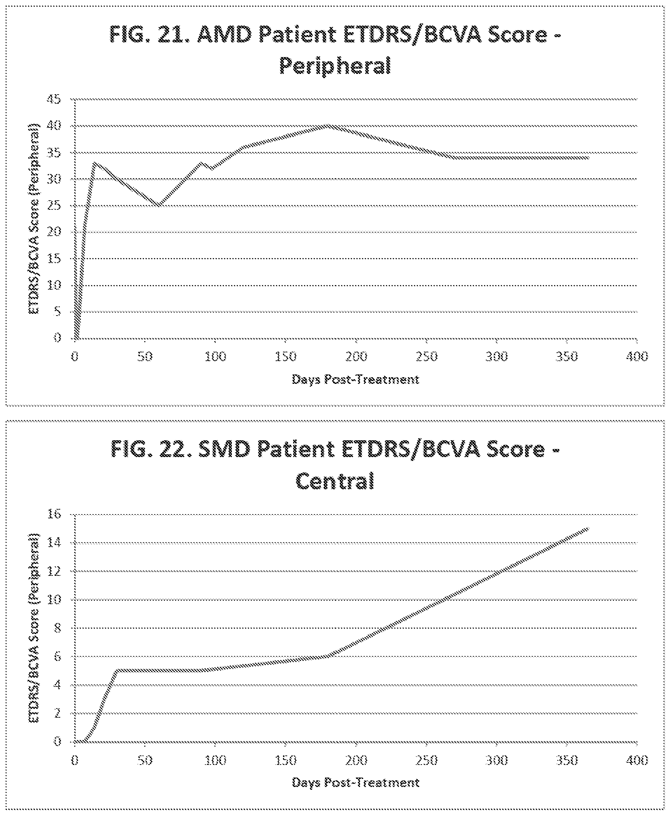

FIG. 21. AMD Patient ETDRS/BCVA Score - Peripheral for time points up to one year from treatment.
FIG. 22. SMD Patient ETDRS/BCVA Score - Central for time points up to one year from treatment.

### DETAILED DESCRIPTION

This disclosure describes initial results for two patients in two prospective clinical trials exploring the safety and tolerability of these hESC-derived RPE in patients with dry AMD and Stargardt's disease. The hESC-derived RPE cells have shown no signs of rejection or tumorigenicity at the time of this report. Visual measurements suggest improvement in both patients. These results indicate that hESCs could serve as a potentially safe and inexhaustible source of RPE for the efficacious treatment of a range of retinal degenerative diseases.

Also described are methods of producing hESC-derived RPE cell populations having advantageous characteristics. Controlling the differentiation pathway, including the degree of gene and pigment expression, was demonstrated to significantly enhance survival, attachment and growth of the cells after injection. Specifically, data presented here shows that the extent of RPE maturity and pigmentation dramatically impacts subsequent attachment and growth of the cells *in vitro.* These results illustrate advantages that may be obtained using cells differentiated from hESC for therapeutic use, as compared to use of primary cells. These results demonstrate that in addition to producing an unlimited number of healthy "young" cells with potentially reduced immunogenicity (20, 21), the stage of *in vitro* differentiation can be controlled at the cellular and molecular level to ensure safety, identity, purity, and potency before transplantation into patients.

We initiated two prospective clinical studies to determine the safety and tolerability of sub-retinal transplantation of hESC-derived retinal pigment epithelium (RPE) in patients with Stargardt's Macular Dystrophy (SMD) and Dry Age-Related Macular Degeneration (AMD), the leading cause of blindness in the developed world. Pre- and postoperative ophthalmic examinations, including visual acuity, fluorescein angiography, optical coherence tomography (OCT), and visual field testing, were carried out on the first patient in each trial.

Controlled hESC differentiation resulted in near-100% pure RPE populations. Immediately after surgery, hyperpigmentation was visible at the transplant site in both patients, with subsequent evidence the cells had attached and integrated into the native RPE layer. No signs of inflammation or hyperproliferation were observed. Visual measures showed signs of improvement during the first two months. At 2 weeks, best corrected visual acuity (BCVA) had improved from 20/500 pre-treatment to 20/200 in the study eye of the AMD patient, and continued to show improvement (20/200-20/320) and an increase in letters on the Early Treatment Diabetic Retinopathy Study (ETDRS) visual acuity chart. The SMD patient improved from hand motion to counting fingers during the same period; by month 1 and 2 BCVA improved to 20/800. Before RPE transplantation, the patient was unable to read any letters on the ETDRS chart, but began reading letters at 2 weeks, which continued to improve during the study period (5 letters at 1 and 2 months).

The hESC-derived RPE cells have shown no signs of rejection or tumorigenicity at the time of this report. Visual measurements demonstrate improvement in both patients.

### Definitions

In order that the invention herein described may be fully understood, the following detailed description is set forth. Various embodiments of the invention are described in detail and may be further illustrated by the provided examples.

Unless defined otherwise, all technical and scientific terms used herein have the same meaning as those commonly understood by one of ordinary skill in the art to which this invention belongs. Although methods and materials similar or equivalent to those described herein can be used in the invention or testing of the present invention, suitable methods and materials are described below. The materials, methods and examples are illustrative only, and are not intended to be limiting. The following terms and definitions are provided herein.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

Throughout this specification, the word "comprise" or variations such as "comprises" or "comprising" will be understood to imply the inclusion of a stated integer or groups of integers but not the exclusion of any other integer or group of integers.

"Effective amount," as used herein, refers broadly to the amount of a compound or cells that, when administered to a patient for treating a disease, is sufficient to effect such treatment for the disease. The effective amount may be an amount effective for prophylaxis, and/or an amount effective for prevention. The effective amount may be an amount effective to reduce, an amount effective to prevent the incidence of signs/symptoms, to reduce the severity of the incidence of signs/symptoms, to eliminate the incidence of signs/symptoms, to slow the development of the incidence of signs/symptoms, to prevent the development of the incidence of signs/symptoms, and/or effect prophylaxis of the incidence of signs/symptoms. The "effective amount" may vary depending on the disease and its severity and the age, weight, medical history, susceptibility, and preexisting conditions, of the patient to be treated. The term "effective amount" is synonymous with "therapeutically effective amount" for purposes of this disclosure.

"Embryo" or "embryonic," as used herein refers broadly to a developing cell mass that has not implanted into the uterine membrane of a maternal host. An "embryonic cell" is a cell isolated from or contained in an embryo. This also includes blastomeres, obtained as early as the two-cell stage, and aggregated blastomeres.

"Embryonic stem cells" (ES cells), as used herein, refers broadly to cells derived from the inner cell mass of blastocysts or morulae that have been serially passaged as cell lines. The ES cells may be derived from fertilization of an egg cell with sperm or DNA, nuclear transfer, parthenogenesis, or by means to generate ES cells with homozygosity in the HLA region. ES cells may also refer to cells derived from a zygote, blastomeres, or blastocyst-staged mammalian embryo produced by the fusion of a sperm and egg cell, nuclear transfer, parthenogenesis, or the reprogramming of chromatin and subsequent incorporation of the reprogrammed chromatin into a plasma membrane to produce a cell. Embryonic stem cells, regardless of their source or the particular method used to produce them, can be identified based on the: (i) ability to differentiate into cells of all three germ layers, (ii) expression of at least Oct-4 and alkaline phosphatase, and (iii) ability to produce teratomas when transplanted into immunocompromised animals. The term also includes cells isolated from one or more blastomeres of an embryo, without destroying the remainder of the embryo (see, e.g., Chung et al., Cell Stem Cell. 2008 Feb 7;2(2):1 13-7; U.S. PGPub No. 20060206953; U.S. PGPub No. 2008/0057041. The term also includes cells produced by somatic cell nuclear transfer, even when non-embryonic cells are used in the process. ES cells may be derived from fertilization of an egg cell with sperm or DNA, nuclear transfer, parthenogenesis, or by means to generate ES cells with homozygosity in the HLA region. ES cells are also cells derived from a zygote, blastomeres, or blastocyst-staged mammalian embryo produced by the fusion of a sperm and egg cell, nuclear transfer, parthenogenesis, or the reprogramming of chromatin and subsequent incorporation of the reprogrammed chromatin into a plasma membrane to produce a cell. Human embryonic stem cells of the present disclosure may include, but are not limited to, MA01, MA09, ACT-4, No. 3, H1, H7, H9, H14 and ACT30 embryonic stem cells. In certain embodiments, human ES cells used to produce RPE cells are derived and maintained in accordance with GMP standards.

"Embryo-derived cells" (EDC), as used herein, refers broadly to morula-derived cells, blastocyst-derived cells including those of the inner cell mass, embryonic shield, or epiblast, or other pluripotent stem cells of the early embryo, including primitive endoderm, ectoderm, and mesoderm and their derivatives. "EDC" also including blastomeres and cell masses from aggregated single blastomeres or embryos from varying stages of development, but excludes human embryonic stem cells that have been passaged as cell lines.

"Macular degeneration," as used herein, refers broadly to diseases characterized by a progressive loss of central vision associated with abnormalities of Bruch's membrane, the neural retina, and the retinal pigment epithelium. Macular degeneration diseases include but are not limited to age- related macular degeneration, North Carolina macular dystrophy, Sorsby's fundus dystrophy, Stargardt's disease, pattern dystrophy, Best disease, malattia leventinese, Doyne's honeycomb choroiditis, dominant drusen, and radial drusen.

"Pluripotent stem cell," as used herein, refers broadly to a cell capable of prolonged or virtually indefinite proliferation in vitro while retaining their undifferentiated state, exhibiting a stable (preferably normal) karyotype, and having the capacity to differentiate into all three germ layers (i.e., ectoderm, mesoderm and endoderm) under the appropriate conditions.

"Pluripotent embryonic stem cells," as used herein, refers broadly cells that: (a) are capable of inducing teratomas when transplanted in immunodeficient (SCID) mice; (b) are capable of differentiating to cell types of all three germ layers (e.g., ectodermal, mesodermal, and endodermal cell types); and (c) express at least one molecular embryonic stem cell markers (e.g., express Oct-4, alkaline phosphatase, SSEA 3 surface antigen, SSEA 4 surface antigen, NANOG, TRA 1 60, TRA 1 81, SOX2, REX1). As an additional example, the pluripotent cells may express OCT-4, alkaline phosphatase, SSEA-3, SSEA-4, TRA-1-60, and/or TRA-1-80. Exemplary pluripotent stem cells can be generated using, for example, methods known in the art. Exemplary pluripotent stem cells include embryonic stem cells derived from the ICM of blastocyst stage embryos, as well as embryonic stem cells derived from one or more blastomeres of a cleavage stage or morula stage embryo without destroying the remainder of the embryo. Such embryonic stem cells can be generated from embryonic material produced by fertilization or by asexual means, including somatic cell nuclear transfer (SCNT), parthenogenesis, and androgenesis. Further exemplary pluripotent stem cells include induced pluripotent stem cells (iPS cells) generated by reprogramming a somatic cell by expressing or inducing expression of a combination of factors (herein referred to as reprogramming factors). iPS cells can be generated using fetal, postnatal, newborn, juvenile, or adult somatic cells. In certain embodiments, factors that can be used to reprogram somatic cells to pluripotent stem cells include, for example, a combination of Oct4 (sometimes referred to as Oct 3/4), Sox2, c-Myc, and Klf4. In other embodiments, factors that can be used to reprogram somatic cells to pluripotent stem cells include, for example, a combination of Oct-4, Sox2, Nanog, and Lin28. In other embodiments, somatic cells are reprogrammed by expressing at least 2 reprogramming factors, at least three reprogramming factors, or four reprogramming factors. In other embodiments, additional reprogramming factors are identified and used alone or in combination with one or more known reprogramming factors to reprogram a somatic cell to a pluripotent stem cell. iPS cells typically can be identified by expression of the same markers as embryonic stem cells, though a particular iPS cell line may vary in its expression profile.

"RPE cell," "differentiated RPE cell," "ES derived RPE cell," and as used herein, may be used interchangeably throughout to refer broadly to an RPE cell differentiated from a pluripotent stem cell, e.g., using a methods disclosed herein. The term is used generically to refer to differentiated RPE cells, regardless of the level of maturity of the cells, and thus may encompass RPE cells of various levels of maturity. RPE cells can be visually recognized by their cobblestone morphology and the initial appearance of pigment. RPE cells can also be identified molecularly based on substantial lack of expression of embryonic stem cell markers such as Oct-4 and NANOG, as well as based on the expression of RPE markers such as RPE 65, PEDF, CRALBP, and bestrophin. For example, a cell may be counted as positive for a given marker if the expected staining pattern is observed, e.g., PAX6 localized in the nuclei, Bestrophin localized in the plasma membrane in a polygonal pattern (showing localized Bestrophin staining in sharp lines at the cell's periphery), ZO-1 staining present in tight junctions outlining the cells in a polygonal pattern, and MITF staining detected confined to the nucleus. Unless otherwise specified, RPE cells, as used herein, refers to RPE cells differentiated in vitro from pluripotent stem cells.

"Mature RPE cell" and "mature differentiated RPE cell," as used herein, may be used interchangeably throughout to refer broadly to changes that occur following initial differentiating of RPE cells. Specifically, although RPE cells can be recognized, in part, based on initial appearance of pigment, after differentiation mature RPE cells can be recognized based on enhanced pigmentation.

"Seeding efficiency" as used herein refers to a to the fraction of recovered cells which, upon thawing, remain viable and can attach to a culture substrate. For example, seeding efficiency can be measured by thawing, washing, and plating cells (preferably on gelatin); with the total cell count determined prior to plating and the viable cell count being determined after plating; the seeding efficiency can then be computed as the fraction of total cells prior to plating which are viable and attached to the substrate after plating. As a more particular example, seeding efficiency is determined by thawing cells in a 37 degree C water bath with constant agitation (such as for one to two minutes or a sufficient time for the cells to thaw), followed by washing cells with phosphate buffered saline (or another suitable wash solution) three times, determining the total cell count (such as using a hemocytometer) including viable and non-viable cells, and plating the cells on gelatin with a growth medium (such as RPE-GM), incubating cells (preferably at 37 degrees C) and allowing cells to become attached to the gelatin for about 24 hours, then determining the viable cell count (e.g., using a hemocytometer and with trypan blue exclusion used to determine viability); seeding efficiency is then determined by dividing the viable cell count after plating by the total cell count prior to plating.

"Pigmented," as used herein refers broadly to any level of pigmentation, for example, the pigmentation that initial occurs when RPE cells differentiate from ES cells. Pigmentation may vary with cell density and the maturity of the differentiated RPE cells. The pigmentation of a RPE cell may be the same as an average RPE cell after terminal differentiation of the RPE cell. The pigmentation of a RPE cell may be more pigmented than the average RPE cell after terminal differentiation of the RPE cell. The pigmentation of a RPE cell may be less pigmented than the average RPE cell after terminal differentiation.

"Signs" of disease, as used herein, refers broadly to any abnormality indicative of disease, discoverable on examination of the patient; an objective indication of disease, in contrast to a symptom, which is a subjective indication of disease.

"Symptoms" of disease as used herein, refers broadly to any morbid phenomenon or departure from the normal in structure, function, or sensation, experienced by the patient and indicative of disease.

"Therapy," "therapeutic," "treating," "treat" or "treatment", as used herein, refers broadly to treating a disease, arresting or reducing the development of the disease or its clinical symptoms, and/or relieving the disease, causing regression of the disease or its clinical symptoms. Therapy encompasses prophylaxis, prevention, treatment, cure, remedy, reduction, alleviation, and/or providing relief from a disease, signs, and/or symptoms of a disease. Therapy encompasses an alleviation of signs and/or symptoms in patients with ongoing disease signs and/or symptoms (e.g., blindness, retinal deterioration.) Therapy also encompasses "prophylaxis" and "prevention". Prophylaxis includes preventing disease occurring subsequent to treatment of a disease in a patient or reducing the incidence or severity of the disease in a patient. The term "reduced", for purpose of therapy, refers broadly to the clinical significant reduction in signs and/or symptoms. Therapy includes treating relapses or recurrent signs and/or symptoms (e.g., retinal degeneration, loss of vision.) Therapy encompasses but is not limited to precluding the appearance of signs and/or symptoms anytime as well as reducing existing signs and/or symptoms and eliminating existing signs and/or symptoms. Therapy includes treating chronic disease ("maintenance") and acute disease. For example, treatment includes treating or preventing relapses or the recurrence of signs and/or symptoms (e.g., blindness, retinal degeneration).

The term "corticosteroid" is used herein to refer to the class of steroid hormones that bind to the glucocorticoid receptor, including natural and artificial corticosteroids, analogs, etc. Exemplary corticosteroids include, but are not limited to prednisolone, hydrocortisone, prednisone, methylprednisolone, dexamethasone, betamethasone, triamcinolone, beclometasone, fludrocortisone acetate, fluticasone (including fluticasone propionate (FP)), budesonide, ciclesonide, mometasone, and flunisolide.

### Preparations of RPE Cells and Combination Therapies

The present disclosure provides preparations of RPE cells, including RPE cells, substantially purified populations of RPE cells, pharmaceutical preparations comprising RPE cells, and cryopreserved preparations of the RPE cells. The RPE cells described herein may be substantially free of at least one protein, molecule, or other impurity that is found in its natural environment (e.g., "isolated".) The RPE cells may be mammalian, including, human RPE cells. The disclosure also provides human RPE cells, a substantially purified population of human RPE cells, pharmaceutical preparations comprising human RPE cells, and cryopreserved preparations of the human RPE cells. The preparation may be a preparation comprising human embryonic stem cell-derived RPE cells, human iPS cell-derived RPE cells, and substantially purified (with respect to non-RPE cells) preparations comprising differentiated ES derived RPE cells.

The RPE cells of the preparation may have a replicative lifespan that is greater than the replicative lifespan of RPE cells obtained from other sources (e.g., cultures derived from donated human tissue, such as fetal, infant, child, adolescent or adult tissue). Replicative lifespan may be assessed by determining the number of population doublings in culture prior to replicative senescence. For example, the RPE cells of the preparation may have a replicative lifespan that is at least 10 percent greater than that of an RPE population derived from donated human tissue, and preferably at least 20, 30, 40, 50, 60, 70 80, 90, 100 percent, or even greater, than that of an RPE population derived from donated human tissue.

The RPE cells of the preparation may have an average telomere length that is at least 30 percent of the telomere length of a hESC and/or human iPS cell (or the average of a population of hESC and/or human iPS cells), and preferably at least 40, 50, 60, 70 80 or even 90 percent of the telomere length of an hESC and/or human iPS cell (or of the average of a population of hESC and/or human iPS cells). For example, said hESC and/or human iPS cell (or said population of hESC and/or human iPS cells) may be a cell or cell population from which said RPE cells were differentiated.

The RPE cells of the preparation may have a mean terminal restriction fragment length (TRF) that is longer than 4 kb, and preferably longer than 5, 6, 7, 8, 9, 10, 11, 12 or even 13kb. In an exemplary embodiment, the RPE cells of the preparation may have a TRF that is 10kb or longer.

The RPE cells of the preparation may have an average lipofuscin content that is less than 50 percent of the average lipofuscin content of the equivalent number of RPE cells isolated from adult eyes (i.e., human adult patients from the age of 25-80, more preferably adults from the age of 50-80), and more preferably less than 40, 30, 20 or even 10 percent of the average lipofuscin content of the equivalent number of RPE cells isolated from adult eyes.

The RPE cells of the preparation may have an average N-retinylidene-N-retinylethanolamine (A2E) content that is less than 50 percent of the average A2E content of the equivalent number of RPE cells isolated adult eyes (e.g., human adult patients from the age of 25-80, more preferably adults from the age of 50-80), and more preferably less than 40, 30, 20 or even 10 percent of the average A2E content of the equivalent number of RPE cells isolated from adult eyes.

The RPE cells of the preparation may have an average N-retinylidene-N-retinylethanolamine (A2E) content that is less than 50ng per 10⁵ (100,000) cells, which may be determined from integrated peak intensities (such as described in Sparrow et al., Invest. Ophthalmol. Vis. Sci. November 1999 vol. 40 no. 12, pg. 2988-2995), and more preferably less than 40ng, 30ng, 20ng, 10ng or even 5ng per 10⁵ cells.

The RPE cells of the preparation may have a rate of phagocytosis of photoreceptor outer segments (POS) that is at least 50 percent greater than the rate of phagocytosis of POS for an equivalent number of RPE cells isolated adult eyes (i.e., human adult patients from the age of 25-80, more preferably adults from the age of 50-80), and more preferably at least than 75, 100, 150 or even 200 percent greater. POS phagocytosis can be measured, as one illustrative and non-limiting example, using the protocols described in Bergmann et al. FASEB Journal March 2004 vol. 18 pages 562-564.

The RPE cells of the preparation may have a rate of phagocytosis of photoreceptor outer segments (POS) that is at least 20 percent of the total concentration of POS after 24 hours, and more preferably at least than 25, 30, 25, 40 or even 50 percent of the total concentration of POS after 24 hours. POS phagocytosis can be measured, as one illustrative and non-limiting example, using the protocols described in Bergmann et al. FASEB Journal March 2004 vol. 18 pages 562-564.

The RPE cells may exhibit a decreased level of accumulated oxidative stress and/or DNA damage compared to RPE cells isolated from an adult host.

The RPE cells of the preparation may have an average proteasome activity that is at least 50 percent greater than the average proteosome activity of the equivalent number of RPE cells isolated adult eyes (i.e., human adult patients from the age of 25-80, more preferably adults from the age of 50-80), and more preferably at least 60, 70, 80, 90 or even 100 percent of the average proteosome activity of the equivalent number of RPE cells isolated from adult eyes. Proteosome activity can be measured using, as one illustrative and non-limiting example, Succinyl-Leu-Leu-Val-Tyr-amidomethylcoumarin (LLVY-AMC) for chymotrypsin-like activity, N-t-butyloxycarbonyl-Leu-Ser-Thr-Arg-amidomethylcoumarin (LSTR-AMC) for trypsin-like activity, and benzyloxycarbonyl-Leu-Leu-Glu-amidomethylcoumarin (LLE-AMC) for peptidylglutamyl-peptide hydrolase activity.

The RPE cells of the preparation may have an average accumulation of ubiquitin conjugates that is less than 50 percent of the average accumulation of ubiquitin conjugates for an equivalent number of RPE cells isolated adult eyes (i.e., human adult patients from the age of 25-80, more preferably adults from the age of 50-80), and more preferably less than 40, 30, 20 or even 10 percent of the average accumulation of ubiquitin conjugates of the equivalent number of RPE cells isolated from adult eyes. Accumulation of ubiquitin conjugates can be measured, as one illustrative and non-limiting example, using the protocols decribed in Zhang et al. Invest. Ophthalmol. Vis. Sci. August 2008 vol. 49 no. 8 3622-3630.

One or more angiogenesis inhibitors may be administered in combination with the preparation of RPE cells, preferably in a therapeutically effective amount for the prevention or treatment of ocular disease, such as an angiogenesis-associated ocular disease. Exemplary ocular diseases include macular degeneration (e.g., wet AMD or dry AMD), diabetic retinopathy, and choroidal neovascularization. Exemplary angiogenesis inhibitors include VEGF antagonists, such as inhibitors of VEGF and/or a VEGF receptor (VEGFR, e.g., VEGFR1 (FLT1, FLT), VEGFR2 (KDR, FLK1, VEGFR, CD309), VEGFR3 (FLT4, PCL)), such as peptides, peptidomimetics, small molecules, chemicals, or nucleic acids, e.g., pegaptanib sodium, aflibercept, bevasiranib, rapamycin, AGN-745, vitalanib, pazopanib, NT-502, NT-503, or PLG101, CPD791 (a di-Fab' polyethylene glycol (PEG) conjugate that inhibits VEGFR-2), anti-VEGF antibodies or functional fragments thereof (such as bevacizumab (AVASTIN^{®}) or ranibizumab (LUCENTIS^{®})), or anti-VEGF receptor antibodies (such as IMC-1121(B) (a monoclonal antibody to VEGFR-2), or IMC-18F1 (an antibody to the extracellular binding domain of VEGFR-1)). Additional exemplary inhibitors of VEGF activity include fragments or domains of VEGFR receptor, an example of which is VEGF-Trap (Aflibercept), a fusion protein of domain 2 of VEGFR-1 and domain 3 of VEGFR-2 with the Fc fragment of IgG1. Another exemplary VEGFR inhibitors is AZD-2171 (Cediranib), which inhibits VEGF receptors 1 and 2. Additional exemplary VEGF antagonists include tyrosine kinase inhibitors (TKIs), including TKIs that reportedly inhibit VEGFR-1 and/or VEGFR-2, such as sorafenib (Nexavar), SU5416 (Semaxinib), SU11248/Sunitinib (Sutent), and Vandetanib (ZD 6474). Additional exemplary VEGF antagonists include Ly317615 (Enzastaurin), which is though to target a down-stream kinase involved in VEGFR signaling (protein kinase C). Additional exemplary angiogenesis inhibitors include inhibitors of alpha5betal integrin activity, including and anti-alpha5betal integrin antibodies or functional fragments thereof (such as volociximab), a peptide, peptidomimetic, small molecule, chemical or nucleic acid such as 3-(2-{1-alkyl-5-[(pyridine-2-ylamino)-methyl]-pyrrolidin-3-yloxy}-acetylamino)-2-(alkyl-amino)-propionic acid, (S)-2-[(2,4,6-trimethylphenyl)sulfonyl]amino-3-[7-benzyloxycarbonyl-8-(2-pyridinylaminomethyl)-1-oxa-2,7-diazaspiro-(4,4)-non-2-en-3-yl]carbonylamino propionic acid, EMD478761, or RC*D(ThioP)C* (Arg-Cys-Asp-Thioproline-Cys; asterisks denote cyclizing by a disulfide bond through the cysteine residues). Additional exemplary angiogenesis inhibitors include 2-methoxyestradiol, alphaVbeta3 inhibitors, Angiopoietin 2, angiostatic steroids and heparin, angiostatin, angiostatin-related molecules, anti-alpha5betal integrin antibodies, anti-cathepsin S antibodies, antithrombin III fragment, bevacizumab, calreticulin, canstatin, carboxyamidotriazole, Cartilage-Derived Angiogenesis Inhibitory Factor, CDAI, CM 101, CXCL10, endostatin, IFN-α, IFN-β, IFN-γ, IL-12, IL-18, IL-4, linomide, maspin, matrix metalloproteinase inhibitors, Meth-1, Meth-2, osteopontin, pegaptanib, platelet factor-4, prolactin, proliferin-related protein, prothrombin (kringle domain-2), ranibizumab, restin, soluble NRP-1, soluble VEGFR-1, SPARC, SU5416, suramin, tecogalan, tetrathiomolybdate, thalidomide, lenalidomide, thrombospondin, TIMP, TNP-470, TSP-1, TSP-2, vasostatin, VEGFR antagonists, VEGI, Volociximab (also known as M200), a fibronectin fragment such as anastellin (*see* Yi and Ruoslahti, Proc Natl Acad Sci U S A. 2001 Jan 16;98(2):620-4) or any combination thereof. Said angiogenesis inhibitor is preferably in an amount sufficient to prevent or treat proliferative (neovascular) eye disease, such as choroidal neovascular membrane (CNV) associated with wet AMD and other diseases of the retina. Additional exemplary angiogenesis inhibitors include: Lenvatinib (E7080), Motesanib (AMG 706), Pazopanib (Votrient), and an IL-6 antagonist such as anti-IL-6 antibody. Additional exemplary angiogenesis inhibitors include fragments, mimetics, chimeras, fusions, analogs, and/or domains of any of the foregoing. Additional exemplary angiogenesis inhibitors include combinations of any of the foregoing. In an exemplary embodiment, the preparation of RPE cells comprises an anti-VEGF antibody, e.g., bevacizumab, such as between about 0.1 mg to about 6.0 mg, e.g., about 1.25 mg and about 2.5 mg bevacizumab, per injection into the eye. In further exemplary embodiments, the preparation of RPE cells comprises one or more inhibitors of VEGF activity and one or more inhibitors of alpha5betal integrin activity.

One or more anti-inflammatory agents may be administered in combination with the preparation of RPE cells. Exemplary anti-inflammatory agents include: glucocorticoids, non-steroidal anti-inflammatory drugs, aspirin, ibuprofen, naproxen, cyclooxygenase (COX) enzyme inhibitors, aldosterone, beclometasone, betamethasone, corticosteroids, cortisol, cortisone acetate, deoxycorticosterone acetate, dexamethasone, fludrocortisone acetate, fluocinolone acetonide (e.g., ILUVIEN^{®}), glucocorticoids, hydrocortisone, methylprednisolone, prednisolone, prednisone, steroids, and triamcinolone. Optionally, the anti-inflammatory agent may not be a corticosteroid. For example, the anti-inflammatory agent may be a non-steroidal anti-inflammatory agent.

Additionally, the patient may not receive a corticosteroid prior to, concurrently with, and/or subsequent to administration of the preparation of RPE cells. Without intent to be limited by theory, Applicants hypothesize that administration of a corticosteroid can interfere with RPE cell settling and/or engraftment. In certain preferred embodiments the patient is not treated with prednisolone or methylprednisolone prior to, concurrently with, and/or subsequent to administration of the preparation of RPE cells. In a more preferred embodiment the patient is not treated with prednisolone prior to, concurrently with, and/or subsequent to administration of the preparation of RPE cells. For example, the patient may not be administered prednisolone or methylprednisolone or another cortocosteroid within at least 3, 6, 12, 24, 48, 72, 96, or 120 hours, or more, prior to administration of the preparation of RPE cells. Additionally, the patient may not be administered prednisolone or methylprednisolone or another cortocosteroid within at least 3, 6, 12, 24, 48, 72, 96, or 120 hours, or more, subsequent to administration of the preparation of RPE cells.

The patient may be administered a non-corticosteroid immune suppressant prior to and/or subsequent to administration of the preparation of RPE cells. Exemplary non-corticosteroid immune suppressants include tacrolimus (FK-506 macrolid) and MMF (mycophenolic acid prodrug).

One or more antioxidants, antioxidant cofactors, and/or other factors contribute to increased antioxidant activity may be administered in combination with the preparation of RPE cells, examples of which may include OT-551 (Othera), vitamin C, vitamin E, beta carotene, zinc (e.g., zinc oxide), and/or copper (e.g., copper oxide).

One or more macular xanthophylls (such as lutein and/or zeaxanthin) may be administered in combination with the preparation of RPE cells.

One or more long-chain omega-3 fatty acids, such as docosahexaenoic acid (DHA) and/or eicosapentaenoic acid (EPA)), may be administered in combination with the preparation of RPE cells.

One or more amyloid inhibitors, such as fenretinide, Arc-1905, Copaxone (glatiramer acetate, Teva), RN6G (PF-4382923, Pfizer) (a humanized monoclonal antibody versus ABeta40 and ABeta42), GSK933776 (GlaxoSmithKline) (anti-amyloid antibody), may be administered in combination with the preparation of RPE cells.

One or more ciliary neurotrophic factor (CNTF) agonists (e.g., CNTF which may be delivered in an intraocular device such as NT-501 (Neurotech)) may be administered in combination with the preparation of RPE cells.

One or more inhibitors of RPE65, such as ACU-4429 (Aculea, Inc.) may be administered in combination with the preparation of RPE cells.

One or more factors that target A2E and/or lipofuscin accumulation, such as Fenretinide, and ACU-4429, may be administered in combination with the preparation of RPE cells.

One or more downregulators or inhibitors of photoreceptor function and/or metabolism, such as fenretinide and ACU-4429, may be administered in combination with the preparation of RPE cells.

One or more α2-adrenergic receptor agonists, such as Brimonidine tartrate, may be administered in combination with the preparation of RPE cells.

One or more selective serotonin 1A agonists, such as Tandospirone (AL-8309B), may be administered in combination with the preparation of RPE cells.

In combination with the preparation of RPE cells, one or more factors targeting C-5, membrane attack complex (C5b-9) and/or any other Drusen component may be administered, examples of which include inhibitors of complement factors D, C-3, C-3a, C5, and C5a, and/or agonists of factor H, such as ARC1905 (Ophthotec) (an anti-C5 Aptamer that selectively inhibits C5), POT-4 (Potentia) (a compstatin derivative that inhibits C3), complement factor H, Eculizumab (Soliris, Alexion) (a humanized IgG antibody that inhibits C5), and/or FCFD4514S (Genentech, San Francisco) (a monoclonal antibody against complement factor D).

One or more immunosuppressants, such as Sirolimus (rapamycin), may be administered in combination with the preparation of RPE cells.

One or more agents that prevent or treat the accumulation of lipofuscin, such as piracetam, centrophenoxine, acetyl-L-carnitine, Ginko Biloba or an extract or preparation thereof, and/or DMAE (Dimethylethanolamine), may be administered in combination with the preparation of RPE cells.

Where one or more agent (such as angiogenesis inhibitors, antioxidants, antioxidant cofactors, other factors contribute to increased antioxidant activity, macular xanthophylls, long-chain omega-3 fatty acids, amyloid inhibitors, CNTF agonists, inhibitors of RPE65, factors that target A2E and/or lipofuscin accumulation, downregulators or inhibitors of photoreceptor function and/or metabolism, α2-adrenergic receptor agonists, selective serotonin 1A agonists, factors targeting C-5, membrane attack complex (C5b-9) and/or any other Drusen component, immunosuppressants, agents that prevent or treat the accumulation of lipofuscin, etc.) is administered in combination with the preparation of RPE cells, said agent may be administered concurrently with, prior to, and/or subsequent to said preparation of RPE cells. For example, said agent may be administered to the eye of the patient during the procedure in which said preparation of RPE cells is introduced into the eye of said patient. Administration of said agent may begin prior to and/or continue after administration of said RPE cells to the eye of the patient. For example, said agent may be provided in solution, suspension, as a sustained release form, and/or in a sustained delivery system (e.g., the Allergan Novadur^{™} delivery system, the NT-501, or another intraocular device or sustained release system).

The RPE cell populations may include differentiated RPE cells of varying levels of maturity, or may be substantially pure with respect to differentiated RPE cells of a particular level of maturity. The RPE cells may be a substantially purified preparation comprising RPE cells of varying levels of maturity/pigmentation. For example, the substantially purified culture of RPE cells may contain both differentiated RPE cells and mature differentiated RPE cells. Amongst the mature RPE cells, the level of pigment may vary. However, the mature RPE cells may be distinguished visually from the RPE cells based on the increased level of pigmentation and the more columnar shape. The substantially purified preparation of RPE cells comprises RPE cells of differing levels of maturity (e.g., differentiated RPE cells and mature differentiated RPE cells). In such instances, there may be variability across the preparation with respect to expression of markers indicative of pigmentation. The pigmentation of the RPE cells in the cell culture may be homogeneous. Further, the pigmentation of the RPE cells in the cell culture may be heterogeneous, and the culture of RPE cells may comprise both differentiated RPE cells and mature RPE cells. Preparations comprising RPE cells include preparations that are substantially pure, with respect to non-RPE cell types, but which contain a mixture of differentiated RPE cells and mature differentiated RPE cells. Preparations comprising RPE cells also include preparations that are substantially pure both with respect to non-RPE cell types and with respect to RPE cells of other levels of maturity.

The percentage of mature differentiated RPE cells in the culture may be reduced by decreasing the density of the culture. Thus, the methods described herein may further comprise subculturing a population of mature RPE cells to produce a culture containing a smaller percentage of mature RPE cells. The number of RPE cells in the preparation includes differentiated RPE cells, regardless of level of maturity and regardless of the relative percentages of differentiated RPE cells and mature differentiated RPE cells. The number of RPE cells in the preparation refers to the number of either differentiated RPE cells or mature RPE cells. The preparation may comprise at least about 75%, 80%, 85%, 90%, 91%, 92%, 93%, 94%, 95%, 96%, 97%, 98%, 99%, or 100% differentiated RPE cells. The preparation may comprise at least about 75%, 80%, 85%, 90%, 91%, 92%, 93%, 94%, 95%, 96%, 97%, 98%, 99%, or 100% mature RPE cells. The RPE cell preparation may comprise a mixed population of differentiated RPE cells and mature RPE cells.

The disclosure provides a cell culture comprising human RPE cells which are pigmented and express at least one gene that is not expressed in a cell that is not a human RPE. For example, although such RPE cells may have substantially the same expression of RPE65, PEDF, CRALBP, and bestrophin as a natural human RPE cell. The RPE cells may vary, depending on level of maturity, with respect to expression of one or more of PAX2, Pax 6, MITF , and/or tyrosinase. Note that changes in pigmentation post-differentiation also correlate with changes in PAX2 expression. Mature RPE cells may be distinguished from RPE cells by the level of pigmentation, level of expression of PAX2, Pax 6, and/or tyrosinase. For example, mature RPE cells may have a higher level of pigmentation or a higher level of expression of PAX2, Pax 6, and/or tyrosinase compared to RPE cells.

The preparations may be substantially purified, with respect to non-RPE cells, comprising at least about 75%, 80%, 85%, 90%, 91%, 92%, 93%, 94%, 95%, 96%, 97%, 98%, 99%, or 100% RPE cells. The RPE cell preparation may be essentially free of non-RPE cells or consist of RPE cells. For example, the substantially purified preparation of RPE cells may comprise less than about 25%, 20%, 15%, 10%, 9%, 8%, 7%, 6%, 5%, 4%, 3%, 2%, or 1% non-RPE cell type. For example, the RPE cell preparation may comprise less than about 25%, 20%, 15%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% non-RPE cells.

The RPE cell preparations may be substantially pure, both with respect to non-RPE cells and with respect to RPE cells of other levels of maturity. The preparations may be substantially purified, with respect to non-RPE cells, and enriched for mature RPE cells. For example, in RPE cell preparations enriched for mature RPE cells, at least about 30%, 40%, 45%, 50%, 55%, 60%, 65%, 70%, 75%, 80%, 85%, 90%, 91%, 92%, 93%, 94%, 95%, 96%, 97%, 98%, 99%, 99%, or 100% of the RPE cells are mature RPE cells. The preparations may be substantially purified, with respect to non-RPE cells, and enriched for differentiated RPE cells rather than mature RPE cells. For example, at least about 30%, 40%, 45%, 50%, 55%, 60%, 65%, 70%, 75%, 80%, 85%, 90%, 91%, 92%, 93%, 94%, 95%, 96%, 97%, 98%, 99%, or 100% of the RPE cells may be differentiated RPE cells rather than mature RPE cells.

The RPE cell preparations may comprise at least about 1x10³, 2x10³, 3x10³, 4x10³, 5x10³, 6x10³, 7x10³, 8x10³, 9x10³, 1x10⁴, 2x10⁴, 3x10⁴, 4x10⁴, 5x10⁴, 6x10⁴, 7x10⁴, 8x10⁴, 9x10⁴, 1x10⁴, 2x10⁵, 3x10⁵, 4x10⁵, 5x10⁵, 6x10⁵, 7x10⁵, 8x10⁵, 9x10⁵, 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10⁹, 2x10¹⁰, 3x10¹⁰, 4x10¹⁰, 5x10¹⁰, 6x10¹⁰, 7x10¹⁰, 8x10¹⁰, or 9x10¹⁰, RPE cells. The RPE cell preparations may comprise at least about 5,000-10,000, 50,000-100,000, 100,000-200,000, 200,000-500,000, 300,000-500,000, or 400,000-500,000 RPE cells. The RPE cell preparation may comprise at least about 20,000-50,000 RPE cells. Also, the RPE cell preparation may comprise at least about 5,000, 10,000, 20,000, 30,000, 40,000, 50,000, 60,000, 70,000, 75,000, 80,000, 100,000, or 500,000 RPE cells.

The RPE cell preparations may comprise at least about 1x10³, 2x10³, 3x10³, 4x10³, 5x10³, 6x10³, 7x10³, 8x10³, 9x10³, 1x10⁴, 2x10⁴, 3x10⁴, 4x10⁴, 5x10⁴, 6x10⁴, 7x10⁴, 8x10⁴, 9x10⁴, 1x10⁵, 2x10⁵, 3x10⁵, 4x10⁵, 5x10⁵, 6x10⁵, 7x10⁵, 8x10⁵, 9x10⁵, 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, or 9x10¹⁰, RPE cells/mL. The RPE cell preparations may comprise at least about 5,000-10,000, 50,000-100,000, 100,000-200,000, 200,000-500,000, 300,000-500,000, or 400,000-500,000 RPE cells/mL. The RPE cell preparation may comprise at least about 20,000-50,000 RPE cells/mL. Also, the RPE cell preparation may comprise at least about 5,000, 10,000, 20,000, 30,000, 40,000, 50,000, 60,000, 75,000, 80,000, 100,000, or 500,000 RPE cells/mL.

The preparations described herein may be substantially free of bacterial, viral, or fungal contamination or infection, including but not limited to the presence of HIV 1, HIV 2, HBV, HCV, CMV, HTLV 1, HTLV 2, parvovirus B19, Epstein-Barr virus, or herpesvirus 6. The preparations described herein may be substantially free of mycoplasma contamination or infection.

The RPE cells described herein may also act as functional RPE cells after transplantation where the RPE cells form a monolayer between the neurosensory retina and the choroid in the patient receiving the transplanted cells. The RPE cells may also supply nutrients to adjacent photoreceptors and dispose of shed photoreceptor outer segments by phagocytosis. Additionally, the RPE cells described herein may have greater proliferative potential than cells derived from eye donors (e.g., the RPE cells are "younger" than those of eye donors). This allows the RPE cell described herein to have a longer useful lifespan than cells derived from eye donors.

The preparations comprising RPE cells may be prepared in accordance with Good Manufacturing Practices (GMP) (e.g., the preparations are GMP-compliant) and/or current Good Tissue Practices (GTP) (e.g., the preparations may be GTP-compliant.)

### RPE Cell Cultures

The present disclosure also provides substantially purified cultures of RPE cells, including human RPE cells. The RPE cultures described herein may comprise at least about 1,000; 2,000; 3,000; 4,000; 5,000; 6,000; 7,000; 8,000; or 9,000 RPE cells. The culture may comprise at least about 1x10⁴, 2x10⁴, 3x10⁴, 4x10⁴, 5x10⁴, 6x10⁴, 7x10⁴, 8x10⁴, 9x10⁴, 1x10⁵, 2x10⁵, 3x10⁵, 4x10⁵, 5x10⁵, 6x10⁵, 7x10⁵, 8x10⁵, 9x10⁵, 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, or 9x10¹⁰, RPE cells.

The RPE cells may be further cultured to produce a culture of mature RPE cells. The RPE cells may be matured, and the RPE cells may be further cultured in, for example RPE-GM/MM or MDBK MM medium until the desired level of maturation is obtained. This may be determined by monitoring the increase in pigmentation level during maturation. As an alternative to RPE-GM/MM or MDBK MM medium, a functionally equivalent or similar medium, may be used. Regardless of the particular medium used to mature the RPE cells, the medium may optionally be supplemented with a growth factor or agent. Both RPE cells and mature RPE cells are differentiated RPE cells. However, mature RPE cells are characterized by increased level of pigment in comparison to differentiated RPE cells. The level of maturity and pigmentation may be modulated by increasing or decreasing the density of the culture of differentiated RPE cells. Thus, a culture of RPE cells may be further cultured to produce mature RPE cells. Alternatively, the density of a culture containing mature RPE cells may be decreased to decrease the percentage of mature differentiated RPE cells and increase the percentage of differentiated RPE cells.

The RPE cells may be identified by comparing the messenger RNA transcripts of such cells with cells derived in vivo. An aliquot of cells is taken at various intervals during the differentiation of embryonic stem cells to RPE cells and assayed for the expression of any of the markers described above. These characteristic distinguish differentiated RPE cells.

The RPE cell culture may be a substantially purified culture comprising at least about 30%, 35%, 40%, or 45%, 50%, 55%, 60%, 65%, 70%, 75%, 80%, 85%, 90%, 91%, 92%, 93%, 94%, 95%, 96%, 97%, 98%, 99%, or 100% differentiated RPE cells. The substantially purified culture may comprise at least about 30%, 35%, 40%, or 45%, 50%, 55%, 60%, 65%, 70%, 75%, 80%, 85%, 90%, 91%, 92%, 93%, 94%, 95%, 96%, 97%, 98%, 99%, or 100% mature differentiated RPE cells.

The RPE cell cultures may be prepared in accordance with Good Manufacturing Practices (GMP) (e.g., the cultures are GMP-compliant) and/or current Good Tissue Practices (GTP) (e.g., the cultures may be GTP-compliant.)

### Cryopreserved Preparations of RPE Cells

The RPE cells may be stored by any appropriate method known in the art (e.g., cryogenically frozen) and may be frozen at any temperature appropriate for storage of the cells. For example, the cells may be frozen at about -20°C, -80°C, -120°C, -130°C, - 135°C, -140°C, -150°C, -160°C, -170°C, -180°C, -190°C, -196°C, at any other temperature appropriate for storage of cells. Cryogenically frozen cells may be stored in appropriate containers and prepared for storage to reduce risk of cell damage and maximize the likelihood that the cells will survive thawing. RPE cells may be cryopreserved immediately following differentiation, following in vitro maturation, or after some period of time in culture. The RPE cells may also be maintained at room temperature, or refrigerated at, for example, about 4°C.

Similarly provided are methods of cryopreserving RPE cells. The RPE cells may be harvested, washed in buffer or media, counted, concentrated (via centrifugation), formulated in freezing media (e.g., 90% FBS/10% DMSO), or any combination of these steps. For example, the RPE cells may be seeded in several culture vessels and serially expanded. As the RPE cells are harvested and maintained in FBS at about 4°C while several flasks of RPE cells are combined into a single lot. The RPE cells may be also washed with saline solution (e.g., DPBS) at least 1, 2, 3, 4, or 5 times. Further, the RPE cells may be cryopreserved after dystrophin is organized at the cell membrane and PAX6 expression is low. In addition, the vials may be labeled, with a primary and/or secondary label. The information on the label may include the type of cell (e.g., hRPE cells), the lot number and date, the number of cells (e.g., 1x10⁶, cells/mL), the expiration date (e.g., recommended date by which the vial should be used), manufacture information (e.g., name and address), warnings, and the storage means (e.g., storage in liquid nitrogen).

Cryopreserved RPE cell preparations described herein may comprise at least about 50,000-100,000 RPE cells. The cryopreserved RPE cell preparations may also comprise at least about 20,000-500,000 RPE cells. Also, the cryopreserved RPE cell preparations may comprise at least about 5,000, 10,000, 20,000, 30,000, 40,000, 50,000, 60,000, 75,000, 80,000, or 100,000 RPE cells. The cryopreserved RPE cell preparations may comprise at least about 1,000, 2,000, 3,000, 4,000, 5,000, 10,000, 20,000, 30,000, 40,000, 50,000, 60,000, 75,000, 80,000, 100,000, or 500,000 RPE cells. The cryopreserved RPE cell preparations may comprise at least about 1,000, 2,000, 3,000, 4,000, 5,000, 6,000, 7,000, 8,000, 9,000, 1x10⁴, 2x10⁴, 3x10⁴, 4x10⁴, 5x10⁴, 6x10⁴, 7x10⁴, 8x10⁴, 9x10⁴, 1x10⁵, 2x10⁵, 3x10⁵, 4x10⁵, 5x10⁵, 6x10⁵, 7x10⁵, 8x10⁵, 9x10⁵, 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, or 9x10¹⁰ RPE cells. The RPE cells of the cryopreserved RPE cell preparations may be mammalian RPE cells, including human RPE cells.

Further, the cryopreserved RPE cell preparations described herein may comprise at least about 50,000-100,000 RPE cells/mL. The cryopreserved RPE cell preparations may also comprise at least about 20,000-500,000 RPE cells/mL. Also, the cryopreserved RPE cell preparations may comprise at least about 5,000, 10,000, 20,000, 30,000, 40,000, 50,000, 60,000, 75,000, 80,000, and 100,000 RPE cells/mL. The cryopreserved RPE cell preparations may comprise at least about 1,000, 2,000, 3,000, 4,000, 5,000, 10,000, 20,000, 30,000, 40,000, 50,000, 60,000, 75,000, 80,000, 100,000, or 500,000 RPE cells/mL. The cryopreserved RPE cell preparations may comprise at least about 1,000, 2,000, 3,000, 4,000, 5,000, 6,000, 7,000, 8,000, 9,000, 1x10⁴, 2x10⁴, 3x10⁴, 4x10⁴, 5x10⁴, 6x10⁴, 7x10⁴, 8x10⁴, 9x10⁴, 1x10⁵, 2x10⁵, 3x10⁵, 4x10⁵, 5x10⁵, 6x10⁵, 7x10⁵, 8x10⁵, 9x10⁵, 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, or 9x10¹⁰ RPE cells/mL. The RPE cells of the cryopreserved RPE cell preparations may be mammalian RPE cells, including human RPE cells.

The RPE cells of the disclosure may be recovered from storage following cryopreservation. The RPE cells recovered from cryopreservation also maintain their viability and differentiation status. For example, at least about 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, or 100% of the RPE cells may retain viability and differentiation following cryopreservation. Further, the RPE cells of the disclosure may be cryopreserved and maintain their viability after being stored for at least about 1, 2, 3, 4, 5, 6, or 7 days. The RPE cells of the disclosure may also be cryopreserved and maintain their viability after being stored for at least about 1, 2, 3, 4, 5, 6, 7, 8, 9, 10, 11, or 12 months. The RPE cells of the disclosure may be cryopreserved and maintain their viability after being stored for at least about 1, 2, 3, 4, 5, 6, or 7 years. For example, the RPE cells of the disclosure may be cryopreserved for at least about 4 years and show at least about 80% viability. The cryopreservation preparation comprising RPE cells may be substantially free of DMSO.

### Methods of Producing RPE Cells

Cell populations analyzed by the subject methods may be produced from pluripotent stem cells. Cell types that may be produced include, but are not limited to, RPE cells, RPE progenitor cells, iris pigmented epithelial (IPE) cells, and other vision associated neural cells, such as internuncial neurons (e.g., "relay" neurons of the inner nuclear layer (INL)) and amacrine cells. Additionally, retinal cells, rods, cones, and corneal cells may be produced. Cells providing the vasculature of the eye may also be produced by the methods described herein.

Without being bound to a particular theory, the inventors found that the methods described herein may act through FGF, EGF, WNT4, TGF-beta, and/or oxidative stress to signal MAP-Kinase and potential C Jun terminal Kinase pathways to induce the expression of the Paired-box 6 (PAX6) transcription factor. PAX6 acts synergistically with PAX2 to terminally differentiate mature RPE via the coordination of MITF and Otx2 to transcribe RPE-specific genes such as Tyrosinase (Tyr), and downstream targets such as RPE 65, Bestrophin, CRALBP, and PEDF. See WO 2009/051671, Figure 1.

The RPE cells described herein may be differentiated from pluripotent stem cells, such as human embryonic stem cells, and may be molecularly distinct from embryonic stem cells, adult-derived RPE cells, and fetal-derived RPE cells. For example, the manufacturing process steps described herein may impart distinctive structural and functional characteristics to the final RPE cell product such that these cells closely resemble native RPE cells and are distinct from fetal derived RPE cells or RPE cell lines (e.g., ARPE19).

Applicants have previously disclosed methods for producing RPE from pluripotent cells. *See* U.S. Patent Nos. 7736896, 7795025 and 7794704, and published international applications WO/2012/012803 and WO 201 1/063005, each of which is incorporated by reference herein in its entirety. RPE may be produced from pluripotent cells cultured as multilayer populations or embryoid bodies. For example, embryoid bodies may be formed by culturing pluripotent cells under non-attached conditions, e.g., on a low-adherent substrate or in a "hanging drop." In these cultures, ES cells can form clumps or clusters of cells denominated as embryoid bodies. *See* Itskovitz-Eldor et al., Mol Med. 2000 Feb;6(2):88-95, which is hereby incorporated by reference in its entirety. Typically, embryoid bodies initially form as solid clumps or clusters of pluripotent cells, and over time some of the embryoid bodies come to include fluid filled cavities, the latter former being referred to in the literature as "simple" EBs and the latter as "cystic" embryoid bodies. *Id.* As Applicants have previously reported, the cells in these EBs (both solid and cystic forms) can differentiate and over time produce increasing numbers of RPE cells. Optionally EBs may then be cultured as adherent cultures and allowed to form outgrowths. Likewise, Applicants have previously reported that pluripotent cells that are allowed to overgrow and form a multilayer cell population can differentiate and form RPE cells over time. Once RPE have formed, they are readily identified based on their morphological characteristics, including pigmentation and cobblestone appearance, and can be isolated for further use.

The pluripotent cells may be propagated and maintained prior to RPE cell formation using any culture methods known in the art. For example, the pluripotent cells may be cultured in the presence of feeder cells, such as murine cells (e.g., murine embryo fibroblasts (MEFs)), human feeder cells (e.g., human adult skin cells, neonatal dermal fibroblasts (HNDFs), etc.). Pluripotent cells may be cultured in xeno-free culture, and/or under feeder-free conditions. *See* Klimanskaya et al., Lancet. 2005 May 7-13;365(9471):1636-41; Richards et al., Stem Cells. 2003;21(5):546-56; U.S. Pat. No. 7,410,798; Ilic et al., Stem Cells Dev. 2009 Nov; 18(9):1343-5; Xu et al. Nat Biotechnol. 2001 Oct;19(10):971-4. For example, pluripotent cells may be cultured on a matrix. The matrix may be selected from the group consisting of: laminin, fibronectin, vitronectin, proteoglycan, entactin, collagen, collagen I, collagen IV, collagen VIII, heparan sulfate, Matrigel(TM) (a soluble preparation from Engelbreth-Holm-Swarm (EHS) mouse sarcoma cells), CellStart, a human basement membrane extract, and any combination thereof. The matrix may be of human or non-human animal origin, such as of bovine, mouse or rat origin. The pluripotent cells may be cultured in a conditioned medium. For example, the conditioned medium may be conditioned by a pluripotent cells, such as ES cells, iPS cells, feeder cells, fetal cells, etc., any of which may or may not be human.

During RPE formation, the pluripotent cells may be cultured in the presence of an inhibitor of rho-associated protein kinase (ROCK). ROCK inhibitors refer to any substance that inhibits or reduces the function of Rho-associated kinase or its signaling pathway in a cell, such as a small molecule, an siRNA, a miRNA, an antisense RNA, or the like. "ROCK signaling pathway," as used herein, may include any signal processors involved in the ROCK-related signaling pathway, such as the Rho-ROCK-Myosin II signaling pathway, its upstream signaling pathway, or its downstream signaling pathway in a cell. An exemplary ROCK inhibitor that may be used is Stemgent's Stemolecule Y-27632, a rho-associated protein kinase (ROCK) inhibitor (see Watanabe et al., Nat Biotechnol. 2007 Jun;25(6):681-6) Other ROCK inhibitors include, e.g., H-1152, Y-30141, Wf-536, HA-1077, hydroxyl-HA-1077, GSK269962A and SB-772077-B. Doe et al., J. Pharmacol. Exp. Ther., 32:89-98, 2007; Ishizaki, et al., Mol. Pharmacol., 57:976-983, 2000; Nakajima et al., Cancer Chemother. Pharmacol., 52:319-324, 2003; and Sasaki et al., Pharmacol. Ther., 93:225-232, 2002. ROCK inhibitors may be utilized with concentrations and/or culture conditions as known in the art, for example as described in US PGPub No. 2012/0276063. For example, the ROCK inhibitor may have a concentration of about 0.05 to about 50 microM, for example, at least or about 0.05, 0.1, 0.2, 0.5, 0.8, 1, 1.5, 2, 2.5, 5, 7.5, 10, 15, 20, 25, 30, 35, 40, 45, or 50 microM, including any range derivable therein, or any concentration effective for promoting cell growth or survival.

For example, pluripotent cell viability may be improved by inclusion of a ROCK inhibitor. In an exemplary embodiment, the pluripotent cells may be maintained under feeder-free conditions, such as on Matrigel(TM) or another matrix. Thereafter, embryoid bodies may be formed from pluripotent cells which are dissociated without the use of trypsin, such as using EDTA, collagenase, or mechanically. The embryoid bodies may be formed in a culture medium comprising Y-27632 or another ROCK inhibitor. For example, the ROCK inhibitor may promote cell viability in embryoid bodies formed from pluripotent cells cultured on Matrigel(TM) or another matrix. RPE cell yield may be thereby improved.

An exemplary method for producing a RPE cell comprises: (a) providing pluripotent stem cells; (b) culturing the pluripotent stem cells as embryoid bodies in nutrient rich, low protein medium, wherein the medium optionally comprises serum free B 27 supplement; (c) culturing the embryoid bodies as an adherent culture in nutrient rich, low protein medium, wherein the medium optionally comprises serum free B 27 supplement; (d) culturing the adherent culture of cells of (c) in nutrient rich, low protein medium, wherein the medium does not comprise serum free B 27 supplement; (e) culturing the cells of (d) in medium capable of supporting growth of high-density somatic cell culture, whereby RPE cells appear in the culture of cells; (f) dissociating cells or clumps of cells from the culture of (e), preferably mechanically or chemically (e.g., using a protease or other enzyme, or another dissociation medium); (g) selecting the RPE cells from the culture and transferring the RPE cells to a separate culture containing medium supplemented with a growth factor to produce an enriched culture of RPE cells; and (g) propagating the enriched culture of RPE cells to produce a RPE cell. These method steps may be performed at least once to produce a substantially purified culture of RPE cells. Further, these method steps may be repeated at least 1, 2, 3, 4, 5, 6, 7, 8, 9, 10 or more times to produce more RPE cells.

Additionally, the disclosure also provides a method for producing a mature retinal pigment epithelial (RPE) cell comprising: (a) providing pluripotent stem cells; (b) culturing the pluripotent stem cells as embryoid bodies in nutrient rich, low protein medium, wherein the medium optionally comprises serum free B 27 supplement; (c) culturing the embryoid bodies as an adherent culture in nutrient rich, low protein medium, wherein the medium optionally comprises serum free B 27 supplement; (d) culturing the adherent culture of cells of step (c) in nutrient rich, low protein medium, wherein the medium does not comprise serum free B 27 supplement; (e) culturing the cells of (d) in medium capable of supporting growth of high-density somatic cell culture, whereby RPE cells appear in the culture of cells; (f) dissociating cells or clumps of cells from the culture of (e), preferably mechanically or chemically (e.g., using a protease or other enzyme, or another dissociation medium); (g) selecting the RPE cells from the culture and transferring the RPE cells to a separate culture containing medium supplemented with a growth factor to produce an enriched culture of RPE cells; (h) propagating the enriched culture of RPE cells; and (i) culturing the enriched culture of RPE cells to produce a mature RPE cell. These method steps may be performed at least once to produce a substantially purified culture of mature RPE cells. Further, these method steps may be repeated at least 1, 2, 3, 4, 5, 6, 7, 8, 9, 10 or more times to produce more mature RPE cells.

For any of the articulated steps, the cells may be cultured for at least about 1-10 weeks. For example, the cells may be cultured for at least about 3-6 weeks. For any of the articulated steps, the cells may be cultured for between about 1 days and 50 days, for example, for at least about 1-3, 3-4, 7, 4-9, 7-10, 7-12, 8-11, 9-12, 7-14, 14-21, and 3-45 days. The cells may be cultured for about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 days. The cells may be cultured for about 1, 2, 3, 4, 5, 6, 7, 8, 9, 10, 11, 12, 13, 14, 15, 16, 17, 18, 19, 20, 21, 22, 23, or 24 hours. For example, the cells may be cultured for 2-4 and 3-6 hours. For each of the above articulated method steps, the cells may be cultured for the same period of time at each step or for differing periods of time at one or more of the steps. Additionally, any of the above articulated method steps may be repeated to produce more RPE cells (e.g., scaled up to produce large numbers of RPE cells).

In the methods described herein, the RPE cells may begin to differentiate from amongst cells in the adherent culture of EBs. RPE cells may be visually recognized based on their cobblestone morphology and the initial appearance of pigmentation. As RPE cells continue to differentiate, clusters of RPE cells may be observed.

Mechanical or enzymatic methods may be used to select RPE cells from amongst clusters of non-RPE cells in a culture of embryoid body, or to facilitate sub culture of adherent cells. Exemplary mechanical methods include, but are not limited to, tituration with a pipette or cutting with a pulled needle. Exemplary enzymatic methods include, but are not limited to, any enzymes appropriate for disassociating cells (e.g., trypsin (e.g., Trypsin/EDTA), collagenase (e.g., collagenase B, collagenase IV), dispase, papain, mixture of collagenase and dispase, a mixture of collagenase and trypsin). A non-enzymatic solution may be used to disassociate the cells, such as a high EDTA-containing solution e.g., Hanks-based cell disassociation buffer.

The RPE cells may be differentiated from the embryoid bodies. Isolating RPE cells from the EBs allows for the expansion of the RPE cells in an enriched culture in vitro. For human cells, RPE cells may be obtained from EBs grown for less than 90 days. Further, RPE cells may arise in human EBs grown for at least about 7-14 days, 14-28 days, 28-45 days, or 45-90 days. The medium used to culture pluripotent stem cells, embryoid bodies, and RPE cells may be removed and/or replaced with the same or different media at any interval. For example, the medium may be removed and/or replaced after at least about 0-7 days, 7-10 days, 10-14 days, 14-28 days, or 28-90 days. Further, the medium may be replaced at least daily, every other day, or at least every 3 days.

To enrich for RPE cells and to establish substantially purified cultures of RPE cells, RPE cells may be dissociated from each other and from non-RPE cells using mechanical and/or chemical (including enzymatic) methods. A suspension of RPE cells may then be transferred to fresh medium and a fresh culture vessel to provide an enriched population of RPE cells.

RPE cells may be selected from the dissociated cells and cultured separately to produce a substantially purified culture of RPE cells. RPE cells are selected based on characteristics associated with RPE cells. For example, RPE cells can be recognized by cobblestone cellular morphology and pigmentation. In addition, there are several known markers of the RPE, including cellular retinaldehyde-binding protein (CRALBP), a cytoplasmic protein that is also found in apical microvilli; RPE65, a cytoplasmic protein involved in retinoid metabolism; bestrophin, the product of the Best vitelliform macular dystrophy gene (VMD2), and pigment epithelium derived factor (PEDF), a 48kD secreted protein with angiostatic properties. The messenger RNA transcripts of these markers may be assayed using PCR (e.g., RT-PCR) or Northern blots. Also, the protein levels of these markers may be assaying using immunoblot technology or Western blots.

The RPE cells may also be selected based on cell function, such as by phagocytosis of shed rod and cone outer segments (or phagocytosis of another substrate, such as polystyrene beads), absorption of stray light, vitamin A metabolism, regeneration of retinoids, and tissue repair. Evaluation may also be performed by testing in vivo function after RPE cell implantation into a suitable host animal (such as a human or non-human animal suffering from a naturally occurring or induced condition of retinal degeneration), e.g., using behavioral tests, fluorescent angiography, histology, tight junctions conductivity, or evaluation using electron microscopy.

The enriched cultures of RPE cells may be cultured in appropriate medium, for example, EGM 2 medium. This, or a functionally equivalent or similar medium, may be supplemented with a growth factor or agent (e.g., bFGF, heparin, hydrocortisone, vascular endothelial growth factor, recombinant insulin-like growth factor, ascorbic acid, or human epidermal growth factor). The RPE cells may be phenotypically stable over a long period of time in culture (e.g., >6 weeks).

Optionally, the RPE may be cultured in the presence of an inhibitor of rho-associated protein kinase (ROCK), such as Stemgent's Stemolecule Y-27632. For example the RPE may be cultured in the presence of a ROCK inhibitor prior to cryopreservation.

### Pluripotent stem cells

The methods described herein may use differentiated cells (such as RPE cells) produced from pluripotent stem cells. Suitable pluripotent stem cells include but are not limited to embryonic stem cells, embryo-derived stem cells, and induced pluripotent stem cells, regardless of the method by which the pluripotent stem cells are derived. Pluripotent stem cells may be generated using, for example, methods known in the art. Exemplary pluripotent stem cells include embryonic stem cells derived from the inner cell mass (ICM) of blastocyst stage embryos, as well as embryonic stem cells derived from one or more blastomeres of a cleavage stage or morula stage embryo without destroying the remainder of the embryo. Such embryonic stem cells may be generated from embryonic material produced by fertilization or by asexual means, including somatic cell nuclear transfer (SCNT), parthenogenesis, cellular reprogramming, and androgenesis. Further, suitable pluripotent stem cells include but are not limited to human embryonic stem cells, human embryo-derived stem cells, and human induced pluripotent stem cells, regardless of the method by which the pluripotent stem cells are derived.

The pluripotent stem cells (e.g., hES cells) may be cultured as a suspension culture to produce embryoid bodies (EBs). The embryoid bodies may be cultured in suspension for about 7-14 days. However, in certain embodiments, the EBs may be cultured in suspension for fewer than 7 days (less than 7, 6, 5, 4, 3, 2, or less than 1 day) or greater than 14 days. The EBs may be cultured in medium supplemented with B 27 supplement.

After culturing the EBs in suspension culture, the EBs may be transferred to produce an adherent culture. For example, the EBs may be plated onto gelatin coated plates in medium. When cultured as an adherent culture, the EBs may be cultured in the same type of media as when grown in suspension. The media may not supplemented with B 27 supplement when the cells are cultured as an adherent culture. Also, the medium is supplemented with B 27 initially (e.g., for less than or equal to about 7 days), but then subsequently cultured in the absence of B 27 for the remainder of the period as an adherent culture. The EBs may be cultured as an adherent culture for at least about 14-28. However, in certain embodiments, the EBs may be cultured as an adherent culture for fewer than about 14 days (less than 14, 13, 12, 11, 10, 9, 8, 7, 6, 5, 4, 3, 2, or less than 1 day) or greater than about 28 days.

### Human Embryonic Stem Cells

Human embryonic stem (hES) cells may be used as a pluripotent stem cell in the methods described herein. Human embryonic stem cells (hES) include progeny of the inner cell mass (ICM) of a blastocyst or cells derived from another source, and may remain pluripotent virtually indefinitely. The hES cells may be derived from one or more blastomeres of an early cleavage stage embryo, without destroying or without harming the embryo. The hES cells may be produced using nuclear transfer. The hES cells may also be induced pluripotent cells (iPS cells) which are described in further detail below. Also, cryopreserved hES cells may be used. The hES cells may be cultured in any way known in the art, such as in the presence or absence of feeder cells. For example, the hES cells may be cultured in EB-DM, MDBK GM, hESC Medium, INVITROGEN^{®} Stem Cell Media, OptiPro SFM, VP SFM, EGM 2, or MDBK MM. See Stem Cell Information (Culture of Human Embryonic Stem Cells (hESC)) [NIH website, 2010]. The hES cells may be used and maintained in accordance with GMP standards.

When grown in culture on a feeder layer in defined conditions hES cells maintain a specific morphology, forming flat colonies comprised of small, tightly packed cells with a high ratio of nucleus to cytoplasm, clear boundaries between the cells, and sharp, refractile colony borders. hES cells express a set of molecular markers, such as Octamer binding protein 4 (Oct-4, a.k.a., Pou5fl), stage specific embryonic antigens (SSEA) 3 and SSEA 4, tumor rejection antigen (TRA) 1 60, TRA 1 80, alkaline phosphatase, NANOG, and Rex 1. Similar to the cells of the ICM that differentiate into predetermined lineages, hES cells in culture may be induced to differentiate. For example, hES cells may be differentiated into human RPE under the defined conditions described herein.

Human embryonic stem cells that may be used include, but are not limited to, MA01, MA04, MA09, ACT 4, MA03, H1, H7, H9, and H14. Additional exemplary cell lines include NED1, NED2, NED3, NED4, and NED5. See also NIH Human Embryonic Stem Cell Registry. An exemplary human embryonic stem cell line that may be used is MA09 cells. The isolation and preparation of MA09 cells was previously described in Klimanskaya, et al. (2006) "Human Embryonic Stem Cell lines Derived from Single Blastomeres." Nature 444: 481-485.

The hES cells may be initially co cultivated with murine embryonic feeder cells (MEF) cells. The MEF cells may be mitotically inactivated by exposure to mitomycin C prior to seeding hES cells in co culture, and thus the MEFs do not propagate in culture. Additionally, hES cell cultures may be examined microscopically and colonies containing non hES cell morphology may be picked and discarded, e.g., using a stem cell cutting tool, by laser ablation, or other means. Typically, after the point of harvest of the hES cells for seeding for embryoid body formation no additional MEF cells are used in the process. The time between MEF removal and RPE cells described herein harvest may be a minimum of at least one, two, three, four, or five passages and at least about 5, 10, 20, 30, 40, 50, 60, 70, 80, 90, or 100 days in MEF-free cell culture. The time between MEF removal and harvesting the RPE cells may also be a minimum of at least about 3 passages and at least about 80-90 days in MEF-free cell culture. Due to the methods of production described herein, the RPE cell cultures and preparations described herein may be substantially free of mouse embryo fibroblasts (MEF) and human embryonic stem cells (hES).

### Induced Pluripotent Stem Cells (iPS cells)

Further exemplary pluripotent stem cells include induced pluripotent stem cells (iPS cells) generated by reprogramming a somatic cell by expressing or inducing expression of a combination of factors ("reprogramming factors"). iPS cells may be generated using fetal, postnatal, newborn, juvenile, or adult somatic cells. iPS cells may be obtained from a cell bank. Alternatively, iPS cells may be newly generated (by methods known in the art) prior to commencing differentiation to RPE cells or another cell type. The making of iPS cells may be an initial step in the production of differentiated cells. iPS cells may be specifically generated using material from a particular patient or matched donor with the goal of generating tissue-matched RPE cells. iPS cells can be produced from cells that are not substantially immunogenic in an intended recipient, e.g., produced from autologous cells or from cells histocompatible to an intended recipient.

The induced pluripotent stem cell may be produced by expressing or inducing the expression of one or more reprogramming factors in a somatic cell. The somatic cell is a fibroblast, such as a dermal fibroblast, synovial fibroblast, or lung fibroblast, or a non-fibroblastic somatic cell. The somatic cell is reprogrammed by expressing at least 1, 2, 3, 4, 5. The reprogramming factors may be selected from Oct 3/4, Sox2, NANOG, Lin28, c Myc, and Klf4. Expression of the reprogramming factors may be induced by contacting the somatic cells with at least one agent, such as a small organic molecule agents, that induce expression of reprogramming factors.

The somatic cell may also be reprogrammed using a combinatorial approach wherein the reprogramming factor is expressed (e.g., using a viral vector, plasmid, and the like) and the expression of the reprogramming factor is induced (e.g., using a small organic molecule.) For example, reprogramming factors may be expressed in the somatic cell by infection using a viral vector, such as a retroviral vector or a lentiviral vector. Also, reprogramming factors may be expressed in the somatic cell using a non-integrative vector, such as an episomal plasmid. *See*, *e.g.*, Yu et al., Science. 2009 May 8;324(5928):797-801. When reprogramming factors are expressed using non-integrative vectors, the factors may be expressed in the cells using electroporation, transfection, or transformation of the somatic cells with the vectors. For example, in mouse cells, expression of four factors (Oct3/4, Sox2, c myc, and Klf4) using integrative viral vectors is sufficient to reprogram a somatic cell. In human cells, expression of four factors (Oct3/4, Sox2, NANOG, and Lin28) using integrative viral vectors is sufficient to reprogram a somatic cell.

Once the reprogramming factors are expressed in the cells, the cells may be cultured. Over time, cells with ES characteristics appear in the culture dish. The cells may be chosen and subcultured based on, for example, ES morphology, or based on expression of a selectable or detectable marker. The cells may be cultured to produce a culture of cells that resemble ES cells-these are putative iPS cells.

To confirm the pluripotency of the iPS cells, the cells may be tested in one or more assays of pluripotency. For example, the cells may be tested for expression of ES cell markers; the cells may be evaluated for ability to produce teratomas when transplanted into SCID mice; the cells may be evaluated for ability to differentiate to produce cell types of all three germ layers. Once a pluripotent iPS cell is obtained it may be used to produce RPE cells.

### Retinal Pigment Epithelium (RPE) Cells

The present disclosure provides RPE cells that may be differentiated from pluripotent stem cells, such as human embryonic stem cells, and may be molecularly distinct from embryonic stem cells, adult-derived RPE cells, and fetal-derived RPE cells. RPE produced according to exemplary embodiments of the methods disclosed herein and in the above-identified related applications may be different than those attainable by previous methods and from other sources of RPE cells. For example, the manufacturing process steps described herein may impart distinctive structural and functional characteristics to the final RPE cell product such that these cells from isolated RPE cells obtained from other sources such as fetal derived RPE cells or RPE cell lines (*e.g.*, ARPE19).

Further, exemplary embodiments of the methods of producing RPE cells described herein are not permissive to ES cells, such that ES cells cannot persist and do not pose an unacceptable risk of contamination in the RPE cell cultures and preparations.

The cell types provided by this disclosure include, but are not limited to, RPE cells, RPE progenitor cells, iris pigmented epithelial (IPE) cells, and other vision associated neural cells, such as internuncial neurons (*e.g.*, "relay" neurons of the inner nuclear layer (INL)) and amacrine cells. The embodiments of the disclosure may also provide retinal cells, rods, cones, and corneal cells as well as cells providing the vasculature of the eye.

The RPE cells may be used for treating retinal degeneration diseases due to retinal detachment, retinal dysplasia, Angioid streaks, Myopic Macular Degeneration, or retinal atrophy or associated with a number of vision-altering ailments that result in photoreceptor damage and blindness, such as, choroideremia, diabetic retinopathy, macular degeneration (*e.g.*, age-related macular degeneration), retinitis pigmentosa, and Stargardt's Disease (fundus flavimaculatus).

The RPE cells may be stable, terminally differentiated RPE cells that do not de-differentiate to a non-RPE cell type. The RPE cells described herein may be functional RPE cells, characterized by the ability to integrate into the retina upon corneal, sub-retinal, or other administration into a human or a non-human animal.

The RPE cells may express RPE cell markers. For example, the level of expression of markers such as RPE65, PAX2, PAX6, tyrosinase, bestrophin, PEDF, CRALBP, Otx2, and MITF may be equivalent to that in naturally occurring RPE cells. The level of maturity of the RPE cells may assessed by measuring expression of at least one of PAX2, PAX6, and tyrosinase, or their respective expression levels.

In contrast, the RPE cells may not express ES cell markers. For example, the expression levels of the ES cell genes Oct-4, NANOG, and/or Rex-1 may be about 100-1000 fold lower in RPE cells than in ES cells. For example, the RPE cells may substantially lack expression of ES cell markers including but not limited to Octamer binding protein 4 (Oct-4, a.k.a., Pou5fl), stage specific embryonic antigens (SSEA)-3 and SSEA-4, tumor rejection antigen (TRA)-1-60, TRA-1-80, alkaline phosphatase, NANOG, Rex-1, Sox2, TDGF-1, DPPA2, DPPA3 (STELLA), DPPA4, and/or DPPA5. Thus, in comparison to ES cells, RPE cells preferably substantially lack expression of Oct-4, NANOG, and/or Rex-1.

The RPE cells described herein may also show elevated expression levels of alpha integrin subunits 1-6 or 9 as compared to uncultured RPE cells or other RPE cell preparations. The RPE cells described herein may also show elevated expression levels of alpha integrin subunits 1, 2, 3, 4, 5, or 9. The RPE cells described herein may be cultured under conditions that promote the expression of alpha integrin subunits 1-6. For example, the RPE cells may be cultured with integrin-activating agents including but not limited to manganese and the activating monoclonal antibody (mAb) TS2/16. *See* Afshari, et al. Brain (2010) 133(2): 448-464. The RPE cells may be plated on laminin (1 µg/mL) and exposed to Mn²⁺ (500 µM) for at least about 8, 12, 24, 36, or 48 hours. Also, the RPE cells may be cultured for several passages (*e.g*., at least about 4, 5, 6, 7, or 8 passages) which may increase alpha integrin subunit expression.

The RPE cells may exhibit a normal karyotype, express RPE markers, and not express hES markers.

The RPE cells described herein may also be identified and characterized based on the degree of pigmentation of the cell. Changes in pigment can be controlled by the density at which the RPE cells are cultured and maintained and the duration that RPE are maintained in culture. Differentiated RPE cells that are rapidly dividing are more lightly pigmented. In contrast, more slowly dividing or non-dividing RPE adopt their characteristic polygonal or hexagonal shape and increase pigmentation level by accumulating melanin and lipofuscin. For example, quiescent RPE cultures (e.g., due to confluence) typically increase their level of pigmentation over time. As such, accumulation of pigmentation serves as an indicator of RPE differentiation and increased pigmentation associated with cell density serves as an indicator of RPE maturity. For example, mature RPE cells may be subcultured at a lower density, such that the pigmentation decreases. In this context, mature RPE cells may be cultured to produce less mature RPE cells. Such RPE cells are still differentiated RPE cells that express markers of RPE differentiation.

The RPE cells described herein may maintain their phenotype for a long period of time *in vitro.* For example, the RPE cells may maintain their phenotype for at least 1, 2, 3, 4, 5, 6, 7, 8, 9, 10, 11, 12, 13, 14, 15, 16, 17, 18, 19, or 20 passages. The RPE cells may maintain their phenotype for at least about 1, 2, 3, 4, 5, 6, 7, 8, 9, 10, 11, 12, 13, 14, 15, 16, 17, 18, 19, or 20 days. The RPE cells may maintain their phenotype for at least about 1, 2, 3, 4, 5, 6, 7, 8, 9, or 10 weeks.

Moreover, the RPE cells described herein may maintain their phenotype following transplantation. The RPE cells may maintain their phenotype for the lifespan of the recipient after transplantation. For example, the RPE cells may maintain their phenotype following transplantation for at least about 1, 2, 3, 4, 5, 6, 7, 8, 9, 10, 11, 12, 13, 14, 15, 16, 17, 18, 19, or 20 days. Further, the RPE cells may maintain their phenotype following transplantation for at least about 1, 2, 3, 4, 5, 6, 7, 8, 9, or 10 weeks. The RPE cells may maintain their phenotype following transplantation for at least about 1, 2, 3, 4, 5, 6, 7, 8, 9, 10, 11, or 12 months. The RPE cells may maintain their phenotype following transplantation for at least about 1, 2, 3, 4, 5, 6, 7, 8, 9, 10, 11, 12, 13, 14, 15, 16, 17, 18, 19, 20, or more years.

### Melanin content of RPE cell populations

Exemplary embodiments of the disclosure provide an RPE cell population having a low or medium average level of pigmentation and a pharmaceutical preparation comprising RPE cells having a low or medium average level of pigmentation. As further described in the examples below, Applicants have shown that RPE cells having a relatively lower level of pigmentation performed better in an assay that measured the capacity of cells for attachment and survival. Without intent to be limited by theory, it is believed that as RPE cells become more mature they may become less able to form cell attachments, survive, and proliferate after cryopreservation, which may be due to the increased level of pigmentation (melanin) contained in more mature RPE cells and/or other phenotypes of mature RPE that tend to generally correlate with increased pigmentation (which may include, for example, changes in cytoskeleton, membrane composition, cell surface receptor expression, attachment strength, nuclear architecture, gene expression, or other phenotypes, or combinations of phenotypes). Also without intent to be limited by theory, it is believed that as RPE cells become more mature they may become less able to form cell attachments, survive, and proliferate when passaged and/or maintained even without cryopreservation; again, this may be due to the increased level of pigmentation (melanin) contained in more mature RPE cells and/or other phenotypes of mature RPE that tend to generally correlate with increased pigmentation.

The level of pigmentation may be measured as an average melanin per cell for a population, e.g., expressed as picograms per cell (pg/cell), as it will be appreciated that in general there may be some variation in the level of melanin in cells in a population. In accordance with the claims, the average melanin content is between 1-5 pg/cell, for example, between 1-4 pg/cell, between 1-3 pg/cell, between 1-2 pg/cell, between 3-5 pg/cell, or between 4-5 pg/cell, such as 4.2-4.8 pg/cell. In a further example, the average melanin content may be 1-5 pg/cell, 2-5 pg/cell, 3-5 pg/cell, 4-5 pg/cell, or 4.5-5 pg/cell.

Melanin content may be measured using a variety of methods, including methods utilizing cell extracts, FACS-based methods, and others. See, e.g., Boissy et al., Cytometry. 1989 Nov;10(6):779-87; Swope et al., J Invest Dermatol. 1997 Sep;109(3):289-95; Watts et al., Cancer Res 1981;41:467-472; Rosenthal et al., Anal Biochem. 1973 Nov;56(1):91-9. For example, to determine the average melanin content, the number of cells in a representative sample may be determined, the cells in the representative sample may be lysed, and the total melanin content of the cell lysate (e.g., from an NaOH-extracted cell pellet) determined (e.g., by spectrophotometry) and divided by the number of cells in the representative sample to yield the average melanin content per cell. Optionally, the number of cells in the representative sample may be determined by disregarding cells other than RPE in the culture (e.g., counting cells that are positive for one or more markers of RPE and/or exhibit a characteristic RPE cell morphology) thereby yielding the average melanin content per RPE cell in the representative sample.

The average melanin content may be determined for the cell population excluding the five percent of the most pigmented and the five percent of the least pigmented harvested RPE cells.

RPE populations having a desired average melanin content can be readily obtained. For example, the melanin content of non-dividing metabolically active RPE (e.g., in a confluent culture) tends to increase over time due to accumulation of synthesized melanin, whereas accumulated melanin is diluted by cell division such that melanin content is relatively lower in dividing cells. *See*, *e.g.*, Dunn et al., Exp Eye Res. 1996 Feb;62(2):155-69. Accordingly, an RPE population having a desired average melanin content can be obtained by selecting the appropriate growth history, e.g., maintenance as a quiescent population for a duration that results in the desired average melanin content, e.g., as a quiescent culture for 1, 2, 3, 4, 5, or 6 days, or for 1, 2, 3, 4, 5, 6, 7, 8, or more weeks. Additional growth histories that may also be used to control average melanin content include maintenance for a time as a quiescent population, followed by allowing the cells to divide again for a specified time or number of divisions (thereby decreasing the average melanin content from that attained in the quiescent population). Melanin content may also be controlled by use of varying culture media and/or media supplements, e.g., melanin accumulation in cultured RPE has been reported to be decreased in the presence of protein kinase inhibitors (e.g., H-7, W-7, H-8, and staurosporine) (Kishi et al., Cell Biol Int. 2000;24(2):79-83), and increase in the presence of all-trans retinoic acid (10(-5) to 10(-7) M) or TGF-beta 1 (1 to 100 U/ml) (Kishi et al., Curr Eye Res. 1998 May;17(5):483-6). Melanin content may also be increased by treatment of cells with zinc alpha-2-glycoprotein (ZAG) (see U.S. Pat. 7,803,750) and/or with an adenosine-1 receptor antagonist, an adenosine-2 receptor agonist, an adenosine-1 receptor agonist, an adenosine-2 receptor antagonist and a combination of an adenosine-1 receptor antagonist, adenosine-2 receptor agonist, or combination thereof (see U.S. Pat. 5,998,423). Each of the foregoing documents is incorporated by reference herein in its entirety.

Alternatively or in addition to the foregoing methods, RPE cells having a desired average melanin content may also be obtained through cell sorting, e.g., using a flow cytometer. For example, melanin-containing cells are detectable by their light-scattering characteristics, including, elevated side-scattering and decreased forward scattering; these characteristics may be used to sort a population by the level of pigmentation, thereby purifying a population having a desired average melanin content. Boissy et al., Cytometry. 1989 Nov;10(6):779-87; Swope et al., J Invest Dermatol. 1997 Sep;109(3):289-95, each of which is incorporated by reference herein in its entirety.

### Engineering MHC genes in human embryonic stem cells to obtain reduced-complexity RPE cells

Human embryonic stem (hES) cells (e.g., from which RPE may be derived as described herein) may be derived from a library of human embryonic stem cells. The library of human embryonic stem cells may comprise stem cells, each of which is hemizygous, homozygous, or nullizygous for at least one MHC allele present in a human population, wherein each member of said library of stem cells is hemizygous, homozygous, or nullizygous for a different set of MHC alleles relative to the remaining members of the library. The library of human embryonic stem cells may comprise stem cells that are hemizygous, homozygous, or nullizygous for all MHC alleles present in a human population. In the context of this disclosure, stem cells that are homozygous for one or more histocompatibility antigen genes include cells that are nullizygous for one or more (and in some embodiments, all) such genes. Nullizygous for a genetic locus means that the gene is null at that locus (*i.e.*, both alleles of that gene are deleted or inactivated.)

A hES cell may comprise modifications to one of the alleles of sister chromosomes in the cell's MHC complex. A variety of methods for generating gene modifications, such as gene targeting, may be used to modify the genes in the MHC complex. Further, the modified alleles of the MHC complex in the cells may be subsequently engineered to be homozygous so that identical alleles are present on sister chromosomes. Methods such as loss of heterozygosity (LOH) may be utilized to engineer cells to have homozygous alleles in the MHC complex. For example, one or more genes in a set of MHC genes from a parental allele can be targeted to generate hemizygous cells. The other set of MHC genes can be removed by gene targeting or LOH to make a null line. This null line can be used further as the embryonic cell line in which to drop arrays of the HLA genes, or individual genes, to make a hemizygous or homozygous bank with an otherwise uniform genetic background. Stem cells that are nullizygous for all MHC genes may be produced by standard methods known in the art, such as, for example, gene targeting and/or loss of heterozygosity (LOH). *See*, *for example*, United States Patent Application Publications 2004/0091936, 2003/0217374 and 2003/0232430, and U.S. Provisional Patent Application Number 60/729,173.

Accordingly, the present disclosure relates to methods of obtaining RPE cells, including a library of RPE cells, with reduced MHC complexity. RPE cells with reduced MHC complexity may be used to increase the supply of available cells for therapeutic applications as it may eliminate the difficulties associated with patient matching. Such cells may be derived from stem cells that are engineered to be hemizygous or homozygous for genes of the MHC complex.

The present disclosure also provides a library of RPE cells (and/or RPE lineage cells), wherein several lines of ES cells are selected and differentiated into RPE cells. These RPE cells and/or RPE lineage cells may be used for a patient in need of a cell-based therapy. The disclosure also provides a library of RPE cells, each of which is hemizygous, homozygous, or nullizygous for at least one MHC allele present in a human population, wherein each member of said library of RPE cells is hemizygous, homozygous, or nullizygous for a different set of MHC alleles relative to the remaining members of the library. The disclosure provides a library of human RPE cells that are hemizygous, homozygous, or nullizygous for all MHC alleles present in a human population.

### Culture Medium

Any medium that is capable of supporting cell cultures may be used in the methods described herein, such as medium for viral, bacterial, or eukaryotic cell culture. For example, the medium may be EB-DM or RPE-GM/MM. As a further example, the medium may be a high nutrient, protein-free medium or high nutrient, low protein medium. Further, the medium also may include nutrient components such as albumin, B-27 supplement, ethanolamine, fetuin, glutamine, insulin, peptone, purified lipoprotein material, sodium selenite, transferrin, vitamin A, vitamin C, or vitamin E. For example, nutrient rich, low protein medium may be any medium which supports the growth of cells in culture and has a low protein content. For example, nutrient rich, low protein media includes but is not limited to MDBK-GM, OptiPro SFM, VP-SFM, DMEM, RPMI Media 1640, IDMEM, MEM, F-12 nutrient mixture, F-10 nutrient mixture EGM-2, DMEM/F-12 media, media 1999, or MDBK-MM. *See also* Table 1. Further, the nutrient rich, low protein medium may be a medium that does not support the growth or maintenance of embryonic stem cells.

When low protein medium is used, the medium may contain at least about 20%, 19%, 18%, 17%, 16%, 15%, 14%, 13%, 12%, 11%, 10%, 9%, 8%, 7%, 6%, 5%, 4%, 3%, 2.5%, 2%, 1.5%, 1%, 0.75%, 0.5%, 0.25%, 0.20%, 0.10%, 0.05%, 0.02%, 0.016%, 0.015%, or 0.010% of a component containing animal-derived protein (*e.g.*, 10% FBS). Note that reference to the percentage of protein present in low protein medium refers to the medium alone and does not account for protein present in, for example, B-27 supplement. Thus, it is understood that when cells are cultured in low protein medium and B-27 supplement, the percentage of protein present in the medium may be higher.

The low protein or protein free medium are supplemented with serum free B-27 supplement. Nutrient components of B27 supplement may comprise biotin, L-carnitine, corticosterone, ethanolamine, D+-galactose, reduced glutathione, linoleic acid, linolenic acid, progesterone, putrescine, retinyl acetate, selenium, triodo-1-thyronine (T3), DL-alpha-tocopherol (vitamin E), DL-alpha-tocopherol acetate, bovine serum albumin, catalase, insulin, superoxide dismutase, and transferrin. When cells are cultured in protein free medium supplemented with B-27, protein free refers to the medium prior to addition of B-27.

Growth factors, agents, and other supplements described herein may be used alone or in combination with other factors, agents, or supplements for inclusion in media. Factors, agents, and supplements may be added to the media immediately, or any time during or after cell culture.

The medium may also contain supplements such as heparin, hydrocortisone, ascorbic acid, serum (*e.g.*, fetal bovine serum), or a growth matrix (*e.g*., extracellular matrix from bovine corneal epithelium, Matrigel(TM) (basement membrane matrix), or gelatin), fibronectin, proteolytic fragments of fibronectin, laminin, thrombospondin, aggrecan, and syndezan.

The culture media may be supplemented with one or more factors or agents.

Growth factors that may be used include, for example, EGF, FGF, VEGF, and recombinant insulin-like growth factor. Growth factors that may be used in the present disclosure also include 6Ckine (recombinant), activin A, α-interferon, alpha-interferon, amphiregulin, angiogenin, β-endothelial cell growth factor, beta cellulin, β-interferon, brain derived neurotrophic factor, cardiotrophin-1, ciliary neurotrophic factor, cytokine-induced neutrophil chemoattractant-1, endothelial cell growth supplement, eotaxin, epidermal growth factor, epithelial neutrophil activating peptide-78, erythropoiten, estrogen receptor-α, estrogen receptor-β, fibroblast growth factor (acidic/basic, heparin stabilized, recombinant), FLT-3/FLK-2 ligand (FLT-3 ligand), gamma-interferon, glial cell line-derived neurotrophic factor, Gly-His-Lys, granulocyte colony-stimulating factor, granulocyte macrophage colony-stimulating factor, GRO-alpha/MGSA, GRO-B, GRO-gamma, HCC-1, heparin-binding epidermal growth factor like growth factor, hepatocyte growth factor, heregulin-alpha (EGF domain), insulin growth factor binding protein-1, insulin-like growth factor binding protein-1/IGF-1 complex, insulin-like growth factor, insulin-like growth factor II, 2.5S nerve growth factor (NGF), 7S-NGF, macrophage inflammatory protein-1β, macrophage inflammatory protein-2, macrophage inflammatory protein-3 α, macrophage inflammatory protein-3β, monocyte chemotactic protein-1, monocyte chemotactic protein-2, monocyte chemotactic protein-3, neurotrophin-3, neurotrophin-4, NGF-β (human or rat recombinant), oncostatin M (human or mouse recombinant), pituitary extract, placenta growth factor, platelet-derived endothelial cell growth factor, platelet-derived growth factor, pleiotrophin, rantes, stem cell factor, stromal cell-derived factor 1B/pre-B cell growth stimulating factor, thrombopoetin, transforming growth factor alpha, transforming growth factor-β1, transforming growth factor-β2, transforming growth factor-β3, transforming growth-factor-β5, tumor necrosis factor (α and β), and vascular endothelial growth factor.

Agents that may be used according to the present disclosure include cytokines such as interferon-a, interferon-a A/D, interferon-β, interferon-y, interferon-γ-inducible protein-10, interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-15, interleukin-17, keratinocyte growth factor, leptin, leukemia inhibitory factor, macrophage colony-stimulating factor, and macrophage inflammatory protein-1α.

The culture media may be supplemented with hormones and hormone antagonists, including but not limited to 17B-estradiol, adrenocorticotropic hormone, adrenomedullin, alpha-melanocyte stimulating hormone, chorionic gonadotropin, corticosteroid-binding globulin, corticosterone, dexamethasone, estriol, follicle stimulating hormone, gastrin 1, glucagon, gonadotropin, hydrocortisone, insulin, insulin-like growth factor binding protein, L-3,3',5'-triiodothyronine, L-3,3',5'-triiodothyronine, leptin, leutinizing hormone, L-thyroxine, melatonin, MZ-4, oxytocin, parathyroid hormone, PEC-60, pituitary growth hormone, progesterone, prolactin, secretin, sex hormone binding globulin, thyroid stimulating hormone, thyrotropin releasing factor, thyroxine-binding globulin, and vasopressin. The culture media may be supplemented with antibodies to various factors including but not limited to anti-low density lipoprotein receptor antibody, anti-progesterone receptor, internal antibody, anti-alpha interferon receptor chain 2 antibody, anti-c-c chemokine receptor 1 antibody, anti-CD 118 antibody, anti-CD 119 antibody, anti-colony stimulating factor-1 antibody, anti-CSF-1 receptor/c-fins antibody, anti-epidermal growth factor (AB-3) antibody, anti-epidermal growth factor receptor antibody, anti-epidermal growth factor receptor, phospho-specific antibody, anti-epidermal growth factor (AB-1) antibody, anti-erythropoietin receptor antibody, anti-estrogen receptor antibody, anti-estrogen receptor, C-terminal antibody, anti-estrogen receptor-B antibody, anti-fibroblast growth factor receptor antibody, anti-fibroblast growth factor, basic antibody, anti-gamma-interferon receptor chain antibody, anti-gamma-interferon human recombinant antibody, anti-GFR alpha-1 C-terminal antibody, anti-GFR alpha-2 C-terminal antibody, anti-granulocyte colony-stimulating factor (AB-1) antibody, anti-granulocyte colony-stimulating factor receptor antibody, anti-insulin receptor antibody, anti-insulin-like growth factor-1 receptor antibody, anti-interleukin-6 human recombinant antibody, anti-interleukin-1 human recombinant antibody, anti-interleukin-2 human recombinant antibody, anti-leptin mouse recombinant antibody, anti-nerve growth factor receptor antibody, anti-p60, chicken antibody, anti-parathyroid hormone-like protein antibody, anti-platelet-derived growth factor receptor antibody, anti-platelet-derived growth factor receptor-B antibody, anti-platelet-derived growth factor-alpha antibody, anti-progesterone receptor antibody, anti-retinoic acid receptor-alpha antibody, anti-thyroid hormone nuclear receptor antibody, anti-thyroid hormone nuclear receptor-alpha 1/Bi antibody, anti-transfesferin receptor/CD71 antibody, anti-transforming growth factor-alpha antibody, anti-transforming growth factor-B3 antibody, anti-rumor necrosis factor-alpha antibody, and anti-vascular endothelial growth factor antibody.

Exemplary growth media potentially suitable for use in the methods described herein are listed in Table 1.

**TABLE 1 GROWTH MEDIA FORMULATIONS**

| **NAME OF MEDIUM** | **FORMULATION** |
|---|---|
| **MEF Growth (MEF-GM)** | 500 mL of IMDM |
| | 55 mL FBS |
| **hES Growth (hES-GM)** | 200 mL Knockout^{®} D-MEM |
| | 30 mL Knockout^{®} Serum Replacement |
| | 2 mL GlutaMAX^{®}-I |
| | 2 mL NEAA |
| | 200 µL 2-mercaptoethanol |
| | 10 ng/mL bFGF |
| | 10 ng/mL LIF |
| **EB Growth (EB-GM)** | 1 L EX-CELL^{®} MDBK-GM |
| | 16.5 mL GlutaMAX^{®}-I |
| | or |
| | 1 L OptiPRO-SFM |
| | 20 mL GlutaMAX^{®}-I |
| | or |
| | EB-DM (described in Example 4) |
| **EB Formation (EB-FM)** | 1 L EX-CELL^{®} MDBK-GM |
| | 16.5 mL GlutaMAX^{®}-I 20 mL B-27 Supplement |
| | or |
| | 1 L OptiPRO-SFM |
| | 20 mL GlutaMAX^{®}-I |
| | 20 mL B-27 Supplement or EB-DM (described in Example 4) |
| **RPE Maintenance (RPE-MM)** | 1 L EX-CELL^{®} MDBK-MM |
| | 20 mL GlutaMAX^{®}-I |
| | or |
| | 1 L VP-SFM |
| | 20 mL GlutaMAX^{®}-I |
| | or |
| | RPE-GM/MM (described in Example 4) |
| **RPE Growth (RPE-GM)** | 500 mL EBM^{®}-2 |
| | 10 mL FBS |
| | 0.2 mL hydrocortisone |
| | 2.0 mL rhFGF-B |
| | 0.5 mL R3-IGF-1 |
| | 0.5 mL ascorbic Acid |
| | 0.5 mL rhEGF |
| | 0.5 mL heparin |
| | 0.5 mL VEGF |
| | or |
| | RPE-GM/MM (described in Example 4) |

### Therapeutic Methods

The RPE cells and pharmaceutically preparations comprising RPE cells produced by the methods described herein may be used for cell-based treatments. The disclosure provides methods for treating a condition involving retinal degeneration comprising administering an effective amount of a pharmaceutical preparation comprising RPE cells, wherein the RPE cells are derived from pluripotent stem cells *in vitro.* Conditions involving retinal degeneration include, for example, choroideremia, diabetic retinopathy, retinal atrophy, retinal detachment, retinal dysplasia, retinitis pigmentosa, Angioid streaks, (also called Knapp streaks or Knapp striae, characterized by small breaks in Bruch's membrane that can become calcified and crack), and Myopic Macular Degeneration (also called Degenerative myopia). The RPE cells described herein may also be used in methods for treating macular degeneration including but are not limited to age related macular degeneration (dry or wet), North Carolina macular dystrophy, Sorsby's fundus dystrophy, Stargardt's disease, pattern dystrophy, Best disease, malattia leventinese, Doyne's honeycomb choroiditis, dominant drusen, and radial drusen. The RPE cells described herein may also be used in methods of treating Parkinson's disease (PD).

A common feature of cell transplantation described in prior publications is low graft survival, for example, in many cell transplantation studies there tends to be a loss of cells immediately following transplantation (*e.g.*, within the first week). This loss of cells does not appear to be due to rejection of the transplanted cells but rather an inability of a certain percentage of the cells to be retained at the transplant site. This lack of cell retention is most likely due to a number of factors such as the failure of the cells to attach to an underlying structure, a lack of sufficient nutrients, or physical stresses at the transplant site. Following this initial drop-off of cell number, the cell survival at various times after transplantation can vary considerably from study to study. Thus, although some studies show a steady decline in numbers, other show results where the grafted cells can reach a stable number. However, an important factor in considering the success of a transplantation is the percentage of recipients with surviving grafts following cell transplant.

In contrast with previous preparations, the RPE cells in the pharmaceutical preparations described herein may survive long term following transplantation. For example, the RPE cells may survive at least about 1, 2, 3, 4, 5, 6, 7, 8, 9, or 10 days. Additionally, the RPE cells may survive at least about 1, 2, 3, 4, 5, 6, 7, 8, 9, or 10 weeks; at least about 1, 2, 3, 4, 5, 6, 7, 8, 9, or 10 months; or at least about 1, 2, 3, 4, 5, 6, 7, 8, 9, or 10 years. Further, the RPE cells may survive throughout the lifespan of the receipt of the transplant. Additionally, at least 10, 20, 30, 40, 50, 60, 70, 80, 90, 95, 96, 97, 98, 99, or 100% of the receipts of RPE cells described herein may show survival of the transplanted RPE cells. Further, the RPE cells described herein may successfully incorporate into the RPE layer in the transplantation receipt, forming a semi-continuous line of cells and retain expression of key RPE molecular markers (*e.g.*, RPE65 and bestrophin). The RPE cells described herein may also attach to the Bruch's membrane, forming a stable RPE layer in the transplantation receipt. Also, the RPE cells described herein are substantially free of ES cells and the transplantation receipts do not show abnormal growth or tumor formation at the transplantation site.

The methods of treating a patient suffering from a condition associated with retinal degeneration may comprise administering a composition of the disclosure locally (*e.g.*, by intraocular injection or insertion of a matrix comprising the pharmaceutical preparation of the disclosure). Intraocular administration of pharmaceutical preparation of the disclosure include, for example, delivery into the vitreous body, transcorneally, sub-conjunctival, subretinal, submacular (e.g., by transfoveal submacular injection), juxtascleral, posterior scleral, and sub-tenon portions of the eye. *See*, for example, U.S. Patent Nos. 7,794,704; 7,795,025; 6,943,145; and 6,943,153.

The disclosure also provides a method of administering human RPE cells that have been derived from reduced-complexity embryonic stem cells to a patient. This method may comprise: (a) identifying a patient that needs treatment involving administering human RPE cells to him or her; (b) identifying MHC proteins expressed on the surface of the patient's cells; (c) providing a library of human RPE cells of reduced MHC complexity made by the method for producing RPE cells of the present disclosure; (d) selecting the RPE cells from the library that match this patient's MHC proteins on his or her cells; (e) administering any of the cells from step (d) to said patient. This method may be performed in a regional center, such as, for example, a hospital, a clinic, a physician's office, and other health care facilities. Further, the RPE cells selected as a match for the patient, if stored in small cell numbers, may be expanded prior to patient treatment.

The RPE cells may be cultured under conditions to increase the expression of alpha integrin subunits 1-6 or 9 as compared to uncultured RPE cells or other RPE cell preparations prior to transplantation. The RPE cells described herein may be cultured to elevate the expression level of alpha integrin subunits 1, 2, 3, 4, 5, 6, or 9. The RPE cells described herein may be cultured under conditions that promote the expression of alpha integrin subunits 1-6. For example, the RPE cells may be cultured with integrin-activating agents including but not limited to manganese and the activating monoclonal antibody (mAb) TS2/16. *See* Afshari, et al. Brain (2010) 133(2): 448-464.

The particular treatment regimen, route of administration, and adjuvant therapy may be tailored based on the particular condition, the severity of the condition, and the patient's overall health. Administration of the pharmaceutical preparations comprising RPE cells may be effective to reduce the severity of the symptoms and/or to prevent further degeneration in the patient's condition. For example, administration of a pharmaceutical preparation comprising RPE cells may improve the patient's visual acuity. Additionally, in certain embodiments, administration of the RPE cells may be effective to fully restore any vision loss or other symptoms. Further, the RPE cell administration may treat the symptoms of injuries to the endogenous RPE layer.

### Pharmaceutical Preparations of RPE Cells

The RPE cells may be formulated with a pharmaceutically acceptable carrier. For example, RPE cells may be administered alone or as a component of a pharmaceutical formulation. The subject compounds may be formulated for administration in any convenient way for use in medicine. Pharmaceutical preparations suitable for administration may comprise the RPE cells, in combination with one or more pharmaceutically acceptable sterile isotonic aqueous or nonaqueous solutions (*e.g.*, balanced salt solution (BSS)), dispersions, suspensions or emulsions, or sterile powders which may be reconstituted into sterile injectable solutions or dispersions just prior to use, which may contain antioxidants, buffers, bacteriostats, solutes or suspending or thickening agents. Exemplary pharmaceutical preparations comprises the RPE cells in combination with ALCON^{®} BSS PLUS^{®} (a balanced salt solution containing, in each mL, sodium chloride 7.14 mg, potassium chloride 0.38 mg, calcium chloride dihydrate 0.154 mg, magnesium chloride hexahydrate 0.2 mg, dibasic sodium phosphate 0.42 mg, sodium bicarbonate 2.1 mg, dextrose 0.92 mg, glutathione disulfide (oxidized glutathione) 0.184 mg, hydrochloric acid and/or sodium hydroxide (to adjust pH to approximately 7.4) in water).

Exemplary compositions of the present disclosure may be formulation suitable for use in treating a human patient, such as pyrogen-free or essentially pyrogen-free, and pathogen-free. When administered, the pharmaceutical preparations for use in this disclosure may be in a pyrogen-free, pathogen-free, physiologically acceptable form. The preparation comprising RPE cells used in the methods described herein may be transplanted in a suspension, gel, colloid, slurry, or mixture. Further, the preparation may desirably be encapsulated or injected in a viscous form into the vitreous humor for delivery to the site of retinal or choroidal damage. Also, at the time of injection, cryopreserved RPE cells may be resuspended with commercially available balanced salt solution to achieve the desired osmolality and concentration for administration by subretinal injection. The preparation may be administered to an area of the pericentral macula that was not completely lost to disease, which may promote attachment and/or survival of the administered cells.

Compositions of the present disclosure may include an inhibitor of rho-associated protein kinase (ROCK), such as Stemgent's Stemolecule Y-27632. For example exemplary compositions may include RPE and a ROCK inhibitor, which may be present in an amount sufficient to promote RPE survival and/or engraftment after administration to a patient.

The RPE cells of the disclosure may be delivered in a pharmaceutically acceptable ophthalmic formulation by intraocular injection. When administering the formulation by intravitreal injection, for example, the solution may be concentrated so that minimized volumes may be delivered. Concentrations for injections may be at any amount that is effective and non-toxic, depending upon the factors described herein. The pharmaceutical preparations of RPE cells for treatment of a patient may be formulated at doses of at least about 10⁴ cells/mL. The RPE cell preparations for treatment of a patient are formulated at doses of at least about 10³, 10⁴, 10⁵, 10⁶, 10⁷, 10⁸, 10⁹, or 10¹⁰ RPE cells/mL. For example, the RPE cells may be formulated in a pharmaceutically acceptable carrier or excipient.

The pharmaceutical preparations of RPE cells described herein may comprise at least about 1,000; 2,000; 3,000; 4,000; 5,000; 6,000; 7,000; 8,000; or 9,000 RPE cells. The pharmaceutical preparations of RPE cells may comprise at least about 1x10⁴, 2x10⁴, 3x10⁴, 4x10⁴, 5x10⁴, 6x10⁴, 7x10⁴, 8x10⁴, 9x10⁴, 1x10⁵, 2x10⁵, 3x10⁵, 4x10⁵, 5x10⁵, 6x10⁵, 7x10⁵, 8x10⁵, 9x10⁵, 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, or 9x10¹⁰ RPE cells. The pharmaceutical preparations of RPE cells may comprise at least about 1x10²-1x10³, 1x10²-1x10⁴, 1x10⁴-1x10⁵, or 1x10³-1x10⁶ RPE cells. The pharmaceutical preparations of RPE cells may comprise at least about 10,000, 20,000, 25,000, 50,000, 75,000, 100,000, 125,000, 150,000, 175,000, 180,000, 185,000, 190,000, or 200,000 RPE cells. For example, the pharmaceutical preparation of RPE cells may comprise at least about 20,000-200,000 RPE cells in a volume at least about 50-200 µL. Further, the pharmaceutical preparation of RPE cells may comprise about 50,000 RPE cells in a volume of 150 µL, about 200,000 RPE cells in a volume of 150 µL, or at least about 180,000 RPE cells in a volume at least about 150 µL.

In the aforesaid pharmaceutical preparations and compositions, the number of RPE cells or concentration of RPE cells may be determined by counting viable cells and excluding non-viable cells. For example, non-viable RPE may be detected by failure to exclude a vital dye (such as Trypan Blue), or using a functional assay (such as the ability to adhere to a culture substrate, phagocytosis, etc.). Additionally, the number of RPE cells or concentration of RPE cells may be determined by counting cells that express one or more RPE cell markers and/or excluding cells that express one or more markers indicative of a cell type other than RPE.

The RPE cells may be formulated for delivery in a pharmaceutically acceptable ophthalmic vehicle, such that the preparation is maintained in contact with the ocular surface for a sufficient time period to allow the cells to penetrate the affected regions of the eye, as for example, the anterior chamber, posterior chamber, vitreous body, aqueous humor, vitreous humor, cornea, iris/ciliary, lens, choroid, retina, sclera, suprachoridal space, conjunctiva, subconjunctival space, episcleral space, intracorneal space, epicorneal space, pars plana, surgically-induced avascular regions, or the macula.

The RPE cells may be contained in a sheet of cells. For example, a sheet of cells comprising RPE cells may be prepared by culturing RPE cells on a substrate from which an intact sheet of cells can be released, e.g., a thermoresponsive polymer such as a thermoresponsive poly(N-isopropylacrylamide) (PNIPAAm)-grafted surface, upon which cells adhere and proliferate at the culture temperature, and then upon a temperature shift, the surface characteristics are altered causing release the cultured sheet of cells (e.g., by cooling to below the lower critical solution temperature (LCST) (see da Silva et al., Trends Biotechnol. 2007 Dec;25(12):577-83; Hsiue et al., Transplantation. 2006 Feb 15;81(3):473-6; Ide, T. et al. (2006); Biomaterials 27, 607-614, Sumide, T. et al. (2005), FASEB J. 20, 392-394; Nishida, K. et al. (2004), Transplantation 77, 379-385; and Nishida, K. et al. (2004), N. Engl. J. Med. 351, 1187-1196. The sheet of cells may be adherent to a substrate suitable for transplantation, such as a substrate that may dissolve in vivo when the sheet is transplanted into a host organism, e.g., prepared by culturing the cells on a substrate suitable for transplantation, or releasing the cells from another substrate (such as a thermoresponsive polymer) onto a substrate suitable for transplantation. An exemplary substrate potentially suitable for transplantation may comprise gelatin (see Hsiue *et al.*, *supra*). Alternative substrates that may be suitable for transplantation include fibrin-based matrixes and others. The sheet of cells may be used in the manufacture of a medicament for the prevention or treatment of a disease of retinal degeneration. The sheet of RPE cells may be formulated for introduction into the eye of a subject in need thereof. For example, the sheet of cells may be introduced into an eye in need thereof by subfoveal membranectomy with transplantation the sheet of RPE cells, or may be used for the manufacture of a medicament for transplantation after subfoveal membranectomy.

The volume of preparation administered according to the methods described herein may be dependent on factors such as the mode of administration, number of RPE cells, age and weight of the patient, and type and severity of the disease being treated. If administered by injection, the volume of a pharmaceutical preparations of RPE cells of the disclosure may be from at least about 1, 1.5, 2, 2.5, 3, 4, or 5 mL. The volume may be at least about 1-2 mL. For example, if administered by injection, the volume of a pharmaceutical preparation of RPE cells of the disclosure may be at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67 ,68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 100, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, or 200 µL (microliters). For example, the volume of a preparation of the disclosure may be from at least about 10-50, 20-50, 25-50, or 1-200 µL. The volume of a preparation of the disclosure may be at least about 10, 20, 30, 40, 50, 100, 110, 120, 130, 140, 150, 160, 170, 180, 190, or 200 µL, or higher.

For example, the preparation may comprise at least about 1x10³, 2x10³, 3x10³, 4x10³, 5x10³, 6x10³, 7x10³, 8x10³, 9x10³, 1x10⁴, 2x10⁴, 3x10⁴, 4x10⁴, 5x10⁴, 6x10⁴, 7x10⁴, 8x10⁴, or 9x10⁴ RPE cells per µL. The preparation may comprise 2000 RPE cells per µL, for example, 100,000 RPE cells per 50 µL or 180,000 RPE cells per 90 µL.

The method of treating retinal degeneration may further comprise administration of an immunosuppressant. Immunosuppressants that may be used include but are not limited to anti-lymphocyte globulin (ALG) polyclonal antibody, anti-thymocyte globulin (ATG) polyclonal antibody, azathioprine, BASILIXIMAB^{®} (anti-IL-2Rα receptor antibody), cyclosporin (cyclosporin A), DACLIZUMAB^{®} (anti-IL-2Rα receptor antibody), everolimus, mycophenolic acid, RITUXIMAB^{®} (anti-CD20 antibody), sirolimus, and tacrolimus. The immunosuppressants may be dosed at least about 1, 2, 4, 5, 6, 7, 8, 9, or 10 mg/kg. When immunosuppressants are used, they may be administered systemically or locally, and they may be administered prior to, concomitantly with, or following administration of the RPE cells. Immunosuppressive therapy may continue for weeks, months, years, or indefinitely following administration of RPE cells. For example, the patient may be administered 5 mg/kg cyclosporin for 6 weeks following administration of the RPE cells.

The method of treatment of retinal degeneration may comprise the administration of a single dose of RPE cells. Also, the methods of treatment described herein may comprise a course of therapy where RPE cells are administered multiple times over some period. Exemplary courses of treatment may comprise weekly, biweekly, monthly, quarterly, biannually, or yearly treatments. Alternatively, treatment may proceed in phases whereby multiple doses are administered initially (*e.g.*, daily doses for the first week), and subsequently fewer and less frequent doses are needed.

If administered by intraocular injection, the RPE cells may be delivered one or more times periodically throughout the life of a patient. For example, the RPE cells may be delivered once per year, once every 6-12 months, once every 3-6 months, once every 1-3 months, or once every 1-4 weeks. Alternatively, more frequent administration may be desirable for certain conditions or disorders. If administered by an implant or device, the RPE cells may be administered one time, or one or more times periodically throughout the lifetime of the patient, as necessary for the particular patient and disorder or condition being treated. Similarly contemplated is a therapeutic regimen that changes over time. For example, more frequent treatment may be needed at the outset (*e.g.*, daily or weekly treatment). Over time, as the patient's condition improves, less frequent treatment or even no further treatment may be needed.

The methods described herein may further comprise the step of monitoring the efficacy of treatment or prevention by measuring electroretinogram responses, optomotor acuity threshold, or luminance threshold in the subject. The method may also comprise monitoring the efficacy of treatment or prevention by monitoring immunogenicity of the cells or migration of the cells in the eye.

The RPE cells may be used in the manufacture of a medicament to treat retinal degeneration. The disclosure also encompasses the use of the preparation comprising RPE cells in the treatment of blindness. For example, the preparations comprising human RPE cells may used to treat retinal degeneration associated with a number of vision-altering ailments that result in photoreceptor damage and blindness, such as, diabetic retinopathy, macular degeneration (including age-related macular degeneration, *e.g*., wet age-related macular degeneration and dry age-related macular degeneration), retinitis pigmentosa, and Stargardt's Disease (fundus flavimaculatus). The preparation may comprise at least about 5,000-500,000 RPE cells (*e.g*., 100,00 RPE cells) which may be administered to the retina to treat retinal degeneration associated with a number of vision-altering ailments that result in photoreceptor damage and blindness, such as, diabetic retinopathy, macular degeneration (including age-related macular degeneration), retinitis pigmentosa, and Stargardt's Disease (fundus flavimaculatus).

The RPE cells provided herein may be human RPE cells. Note, however, that the human cells may be used in human patients, as well as in animal models or animal patients. For example, the human cells may be tested in mouse, rat, cat, dog, or non-human primate models of retinal degeneration. Additionally, the human cells may be used therapeutically to treat animals in need thereof, such as in veterinary medicine.

### Modes of Administration

The pharmaceutical preparation may be formulated in a pharmaceutically acceptable carrier according to the route of administration. For example, the preparation may be formulated to be administered to the subretinal space of the eye. The preparation comprising RPE cells may be administered to one eye or both eyes in the same patient. The administration to both eyes may be sequential or simultaneous. For example, the preparation comprising RPE cells may be formulated as a suspension, solution, slurry, gel, or colloid.

RPE cells of the disclosure may be administered locally by injection (*e.g.*, intravitreal injection), or as part of a device or implant (*e.g*., an implant). As noted above, the RPE cells may have various possible arrangements such as individual cells, clumps, clusters, sheets, or any combination thereof, which may be contained in an aqueous carrier, gel, matrix, polymer, or the like. For example, the preparation may be administered by injection into the subretinal space of the eye. Also, the preparation may be administered transcorneally. For example, the cells of the present disclosure may be transplanted into the subretinal space by using vitrectomy surgery. Additionally, at the time of injection, RPE cells may be resuspended with commercially available balanced salt solution (e.g., Alcon BSS PLUS^{®}) to achieve the desired osmolality and concentration for administration by subretinal injection.

Optionally, the RPE cells may be administered by a method comprising pars plana vitrectomy surgery, such as a 3 port pars plana vitrectomy. The method may include a small retinotomy. Prior to cell administration, a subretinal bleb may be formed, e.g., of by injection of saline or another suitable fluid (a "pre-bleb"), which may then be removed prior to cell administration. However, the cells may also be administered without pre-bleb formation. The cells may be administered in a bleb in a temporal foveal position. For example, the bleb may optionally extend within the arcade blood vessels. The bleb may be positioned such that it does not detach the central macula fovea.

Depending on the method of administration, the RPE cells may be added to buffered and electrolyte balanced aqueous solutions, buffered and electrolyte balanced aqueous solutions with a lubricating polymer, mineral oil or petrolatum-based ointment, other oils, liposomes, cylcodextrins, sustained release polymers or gels.

### Matrices for use with RPE cells

The methods described herein may comprise a step of administering RPE cells of the disclosure as an implant or device. In certain embodiments, the device is bioerodible implant for treating a medical condition of the eye comprising an active agent dispersed within a biodegradable polymer matrix, wherein at least about 75% of the particles of the active agent have a diameter of less than about 10 µm. The bioerodible implant may be sized for implantation in an ocular region. The ocular region may be any one or more of the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina. The biodegradable polymer may be, for example, a poly(lactic-co-glycolic)acid (PLGA) copolymer, biodegradable poly(DL-lactic-co-glycolic acid) films, or PLLA/PLGA polymer substrates. The ratio of lactic to glycolic acid monomers in the polymer is about 25/75, 40/60, 50/50, 60/40, 75/25 weight percentage, more preferably about 50/50. The PLGA copolymer may be about 20, 30, 40, 50, 60, 70, 80 to about 90 percent by weight of the bioerodible implant. The PLGA copolymer may be from about 30 to about 50 percent by weight, preferably about 40 percent by weight of the bioerodible implant. The RPE cells may be transplanted in conjunction with a biocompatible polymer such as polylactic acid, poly(lactic-*co*-glycolic acid), 50:50 PDLGA, 85:15 PDLGA, and INION GTR^{®} biodegradable membrane (mixture of biocompatible polymers). *See* U.S. Patent No. 6,331,313; 7,462,471; and 7,625,582. *See also* Hutala, et al. (2007) "In vitro biocompatibility of degradable biopolymers in cell line cultures from various ocular tissues: Direct contact studies." Journal of Biomedical Materials Research 83A(2): 407-413; Lu, et al. (1998) J Biomater Sci Polym Ed 9: 1187-205; and Tomita, et al. (2005) Stem Cells 23: 1579-88.

In another aspect, the disclosure provides a composition comprising RPE situated on a membrane, and a method of using the same in the prevention or treatment of a disease, disorder, or condition of the retina. For example, the membrane may be a membrane as described in U.S. PGPub. No. 20110236464. The membrane may be substantially non-biodegradable and porous, the pores being between approximately 0.2µm and 0.5µm in diameter. For example, the pore diameter may be between 0.3 µm to 0.45µm. Use of a non-biodegradable membrane may ensure that it remains to support the cells once transplanted into the eye, for example for at least 5 years, at least 10 years, or at least 15 years following insertion into the body.

The pore density may be between approximately 1 x 10^7 and 3 x 10^8 pores per cm^2, such as between 5 x 107 and 1 x 10^8 pores per cm^2. This density may allow for the desired permeability levels and also may allow vascularization. In particular the size and density of the pores may allow the movement of nutrients from one side of the membrane to the other and also allow vascularization through the membrane, e.g., post-implantation. The polymer body can receive vascularization from the rich choroidal bed. This has been shown in rich vascular beds outside the eye (Cassell et al, 2002; Patrick et al, 1999; Saxena et al 1999, Peter et al 1998) but can only occur if the porosity is sufficient (Menger et al, 1990).

For example, the membrane hydraulic conductance may be more than 50 x 10^-10 m sec^-1 Pa^-1. Specifically, the membrane hydraulic conductance of the membrane may be approximately 33mL/min/cm^2. This is equal to = 801.21 x 10^-10 m sec^-1 Pa^-1 which is eight times the hydraulic conductivity of young macular cadaveric Bruch's membrane. This surplus conductivity is potentially useful since the artificial membrane may rely entirely on passive processes. As well as being able to meet the demands of the overlying cells in terms of nutrient diffusion, it preferably is not be a hindrance to fluid transport from the basal side of the RPE layer otherwise the RPE may detach from the polymer surface. Consistent with this expectation, the reduced hydraulic conductivity of Bruch's membrane in the elderly has been hypothesized to cause pigment epithelial detachments in AMD (Bird & Marshall, 1986).

Preferably, the membrane may be sterilized by gamma irradiation, ethylene oxide, autoclaving or UV sterilization without degrading.

Preferably the membrane may be sealed by ultrasonic sealing, radio frequency sealing or insert molding. The allows other layers to be attached to the membrane, for example attaching pharmaceutical or coating layers to the membrane. For example, one might wish to attach a more rigid biodegradable layer, such as PLGA, to provide rigidity to the membrane to aid delivery. Alternatively, layers may be attached which contain pharmacological or biological agents, or layers which support other cells.

The membrane preferably has a maximum thickness of approximately 11 µm. More preferably the membrane thickness is between 9 µm and 11 µm. The thickness of the membrane can be selected so as to allow diffusion of nutrients, to allow vascularization and also to allow the membrane to be easily inserted into the eye..

Accordingly, the RPE may be provided on or cultured on a membrane for supporting the growth of cells, the membrane being substantially non-biodegradable and porous and having a maximum thickness of approximately 11 µm. The membrane is preferably substantially planar and its smallest dimension is preferably less than approximately 11 µm. It may vary in thickness in that dimension, but is preferably between 9 µm and 11 µm thick.

The membrane may have a maximum weight of approximately 1.5mg/cm^2. More preferably the weight of the membrane is between 1.0 mg/cm^2 and 1.4 mg/cm^2. The minimum tensile strength of the membrane is preferably at least 100 bars, to provide enough strength to allow properly during surgery. The maximum tensile strength is preferably 300 bars, again to allow the membrane to be handled easily during surgery. The burst strength of the membrane is preferably at least 10 psi.

Preferably, the membrane is hydrophilic. This may give the membrane good wetting capability and allow attachment of cells and other desirable coatings with ease.

The membrane preferably has a physiologically acceptable pH, e.g., a pH of 4 to 8.

The membrane preferably comprises a coating on at least one side. The coating is preferably a protein or a glycoprotein, such as laminin, Matrigel(TM), collagen, fibronectin and/or PLGA poly(lactic-co-glycolic acid). The coating may also comprise a pharmacological or biological agent, bound to the coating component. For example, the coating may include a neurotrophic agent, an anti-inflammatory agent, or an antiangiogenic agent.

In particular the coating preferably contains laminin, especially laminin-1 or a fragment thereof, such as IgVAV. In particular, the coating may contain more laminin-1 than other protein or glycoprotein. Preferably the coating may comprise at least 30% or at least 40% laminin, such as laminin-1. The coating may be applied to produce a laminin-1 concentration on the membrane of approximately 40 - 45µg/cm^2.

Accordingly, the RPE may be provided or cultured a membrane for supporting the growth of cells, the membrane comprising a substantially non-biodegradable and porous support layer coated on at least one side with a coating comprising laminin-1.

The membrane may be made from a hydrophilic polymer. Also hydrophobic polymers that have been made hydrophilic by shining UV light onto that polymer may be used. Exemplary polymers include polyesters such as polyethylene terephthalate, polybutylene terephthalate; polyurethanes and poly urea-urethanes, in particular those containing polycarbonate and polysiloxane, and those that are polyester based or polyether based; polyamides such as nylon; polyether-esters such as Sympatex; polycarbonates such as Makrolon; polyacrylates such as Perspex; poly(tetrafluoroethene) (PTFE); polysiloxanes; polyolefins such as polyethylene and polypropylene; and polyoxymethylene (POM) commonly known under DuPont's brand name Delrin. It is particularly preferred that the membrane is made from polyethylene terephthalate or polybutylene terephthalate. In another preferred embodiment, the membrane is made from polyester.

The membrane may be used for growing a layer of the RPE cells of the present disclosure. The membrane may preferably comprise a layer of cells on the membrane. The cells may be any cells selected according to the intended use of the membrane and cells.

The membrane and layer of cells are preferably at least 3mm x 5mm in length and width. Preferably the membrane and layer of cells are at least 4mm x 6mm.

The membrane and layer of cells may be transplanted into the eye of a patient in need thereof, e.g., in the treatment of age related macular degeneration, retinal tears, macular distrophy, choroidemia, Leber Congenital Amarosis, Stargardt Disease, and other diseases or conditions of the retina.

### Screening Assays

The disclosure provides a method for screening to identify agents that modulate RPE cell maturity. For example, RPE cells differentiated from human ES cells may be used to screen for agents that promote RPE maturation. Identified agents may be used, alone or in combination with RPE cells, as part of a treatment regimen. Alternatively, identified agents may be used as part of a culture method to improve the survival of RPE cells differentiated *in vitro.*

The RPE cells may be used as a research tool in settings such as a pharmaceutical, chemical, or biotechnology company, a hospital, or an academic or research institution. Such uses include the use of RPE cells differentiated from embryonic stem cells in screening assays to identify, for example, agents that may be used to promote RPE survival *in vitro* or *in vivo*, or that may be used to promote RPE maturation, survival, and/or engraftment. Identified agents may be studied *in vitro* or in animal models to evaluate, for example, their potential use alone or in combination with RPE cells.

The disclosure provides a method for identifying agents that promote RPE maturation comprising providing a RPE cell, contacting said RPE cell with an agent, assessing said RPE cell for signs of maturity, and then identifying an agent that promotes RPE maturation when said agent causes RPE cell to show signs of maturity. The signs of maturity may be pigmentation level, gene expression levels, and morphology as discussed herein.

### Commercial Applications and Methods

Certain aspects of the present disclosure pertain to the production of RPE cells to reach commercial quantities. The RPE cells may be produced on a large scale, stored if desired, and supplied to hospitals, clinicians or other healthcare facilities.

Accordingly certain aspects of the present disclosure relate to methods of production, storage, and distribution of RPE cells produced by the methods disclosed herein. Following RPE production, RPE cells may be harvested, purified, and optionally stored prior to a patient's treatment. RPE cells may optionally be patient specific or specifically selected based on HLA or other immunologic profile. For example, once a patient presents with an indication such as, for example, diabetic retinopathy, macular degeneration (including age-related macular degeneration), retinitis pigmentosa, retinal atrophy, retinal detachment, retinal dysplasia, and Stargardt's Disease (fundus flavimaculatus), Angioid streaks, or Myopic Macular Degeneration, RPE cells may be ordered and provided in a timely manner. Accordingly, the present disclosure relates to methods of producing RPE cells to attain cells on a commercial scale, cell preparations comprising RPE cells derived from said methods, as well as methods of providing (*i.e*., producing, optionally storing, and selling) RPE cells to hospitals and clinicians. The production of differentiated RPE cells or mature differentiated RPE cells may be scaled up for commercial use.

The present disclosure also provides for methods of conducting a pharmaceutical business comprising establishing a distribution system for distributing the preparation for sale or may include establishing a sales group for marketing the pharmaceutical preparation.

The present disclosure provides methods of supplying RPE cells to hospitals, healthcare centers, and clinicians, whereby RPE cells produced by the methods disclosed herein are stored, ordered on demand by a hospital, healthcare center, or clinician, and administered to a patient in need of RPE cell therapy. A hospital, healthcare center, or clinician orders RPE cells based on patient specific data, RPE cells are produced according to the patient's specifications and subsequently supplied to the hospital or clinician placing the order. For example, after a particular RPE cell preparation is chosen to be suitable for a patient, it is thereafter expanded to reach appropriate quantities for patient treatment.

Further aspects of the disclosure relate to a library of RPE cells that can provide matched cells to potential patient recipients. Accordingly, the disclosure provides a method of conducting a pharmaceutical business, comprising the step of providing RPE cell preparations that are homozygous for at least one histocompatibility antigen, wherein cells are chosen from a bank of such cells comprising a library of RPE cells that may be expanded by the methods disclosed herein, wherein each RPE cell preparation is hemizygous or homozygous for at least one MHC allele present in the human population, and wherein said bank of RPE cells comprises cells that are each hemizygous or homozygous for a different set of MHC alleles relative to the other members in the bank of cells. As mentioned above, gene targeting or loss of heterozygosity may be used to generate the hemizygous or homozygous MHC allele stem cells used to derive the RPE cells.

The present disclosure also includes methods of obtaining or producing human ES cells (e.g., induced pluripotent (iPS) cells, or ES cells produced by somatic cell nuclear transfer, or ES cells produced by other reprogramming methods) from a patient or a histocompatible donor and then generating and expanding RPE cells derived from the ES cells. These RPE cells may be stored. In addition, these RPE cells may be used to treat the patient from which the ES were obtained or a relative of that patient or a histocompatible individual.

The present disclosure demonstrates that human RPE cells may be reliably differentiated and expanded from human ES cells under well-defined and reproducible conditions-representing an inexhaustible source of cells for patients with retinal degenerative disorders. The concentration of these cells would not be limited by availability, but rather could be titrated to the precise clinical requirements of the individual. Repeated infusion or transplantation of the same cell population over the lifetime of the patient would also be possible if deemed necessary by the physician. Furthermore, the ability to create banks of matching or reduced-complexity HLA hES lines from which RPE cells could be produced could potentially reduce or eliminate the need for immunosuppressive drugs and/or immunomodulatory protocols altogether.

### EXAMPLES

The invention now being generally described, it will be more readily understood by reference to the following examples, which are included merely for purposes of illustration of certain aspects and embodiments of the present invention, and are not intended to limit the invention.

Further information concerning results presented examples and/or additional experimental results is included in the attached manuscript which is included immediately preceding the claims in the present application.

### EXAMPLE 1

### METHODS

### GENERATION OF hESC MASTER CELL BANK

The hESC line used in these studies was previously described MA09 (22), derived from an unused in vitro fertilization (IVF) embryo obtained with full informed consent and used in compliance with Advanced Cell Technology's Ethics Advisory Board and Institutional Review Board. MA09 seed stock was thawed and expanded through four serial passages on mitotically-inactivated mouse embryonic fibroblasts (MEF) using current Good Manufacturing Practices. The clinical hESC master cell bank (hESC-MCB) was cryopreserved, and confirmed to have normal female (46, XX) karyotype and to be free of bacterial and mycoplasmal contaminants as well as human, bovine, porcine and murine viruses. PCR analysis showed no changes or mutations in genes associated with macular degeneration, including CTRP5, EVLV4, RPE-65, VMD2, and ABCA4 (Table 3 below).

### MANUFACTURE OF RETINAL PIGMENT EPITHELIUM

Vials of hESC-MCB were thawed and expanded on Mitomycin C-treated MEF for three passages. Since the hESCs were co-cultured with animal cells, the differentiated derivatives are classified as a xenotransplantation product and subject to FDA guidelines for donor animal and product processing, testing, and archiving, as well as patient, monitoring and registration (further described in Example 2 below). After hESC expansion, the cells were sequentially induced to form embryoid bodies followed by cellular outgrowth and localized differentiation into pigmented RPE patches. The production of RPE used in this Example is further described in Example 4 below. The pigmented patches were isolated with collagenase, and after purification and trypsinization, the dissociated cells were seeded, grown to confluence, and induced to redifferentiate for a total of three serial passages. Passage 2 RPE were cryopreserved and served as the starting material for formulating cells for clinical use.

### PRECLINICAL STUDIES

Human ESC-derived RPE cells were injected subretinally into NIH III immune-nude mice (tumorigenicity and biodistribution studies) and dystrophic RCS rats and ELOV4 mice (efficacy studies) as previously described (8). Detection of human cells in the injected eyes and other organs was performed by DNA Q-PCR designed to amplify human Alu Y DNA sequences and by immunostaining of paraffin sections for human mitochondria and human bestrophin (further described in Example 2).

### CELL CHARACTERIZATION AND SAFETY TESTING

The RPE cells were assessed for safety and characterized for a number of RPE-specific attributes at various times, including in-process testing and testing performed after thaw, final product formulation, and culturing to maturity to simulate the fate of the transplanted cells *in vitro.* Safety assessment for potential bacteria, mycoplasma, murine viruses, and residual murine DNA were performed according to standard protocols by WuXi Apptec, Inc. St. Paul, MN. Cytogenetic analysis for karyotyping, DNA fingerprinting for cell line certification, and fluorescence *in situ* hybridization (FISH) were performed by Cell Lines Genetics, Madison, WI. Endotoxin testing was performed on cryopreserved RPE formulated as final product for clinical injection by Cape Cod Associates, Inc, East Falmouth, MA. Quantitative immunohistochemical staining was conducted using standard methods with the percentage of positive stained cells normalized to the number of DAPI stained nuclei inspected. Assessment of RPE purity and the extent of differentiation were based on the percentage of bestrophin, Pax6, ZO-1 and/or MITF stained cells. Screening to confirm the absence of pluripotency markers was performed by staining for OCT-4 and Alkaline Phosphatase. Phagocytosis (potency assay) was assessed by quantitative fluorescence activated cell sorting (FACS) analysis of RPE cultures exposed to PhRodo^{™} (Invitrogen) fluorescent bioparticles. Quantitative reverse transcription (q-RT) PCR assays were performed to confirm up-regulation of RPE-specific genes (RPE-65, PAX-6, MITF, bestrophin) and down-regulation of hESC-specific genes (OCT-4, NANOG, SOX-2). The melanin content per cell was measured spectrophotometrically in NaOH extracted pellets with known cell numbers (further described in example 2).

### CELL FORMULATION AND INJECTION

Vials of cryopreserved MA09-RPE were thawed, washed 3X by centrifugation, and resuspended at a density of 2 X 10³ viable cells/µL of BSS PLUS^{®} (Alcon). A vial containing the appropriate volume of formulated RPE and a paired vial containing the appropriate volume of BSS PLUS^{®} at 2-8 C were delivered to the OR. Immediately prior to injection, the two vials were reconstituted in a 1 mL syringe to obtain a loading cell density that would result in delivery of the desired number of RPE (50,000 viable RPE cells into the subretinal space of each patient's eye). To ensure accurate delivery of the intended dosage, the loading cell density was increased to offset the expected loss of viable RPE encountered during mixing, loading, and delivery through the cannula. This viable cell loss was measured as described in Example 3 below and was shown to be dependent on the cannula used. In these examples, the MEDONE POLYTIP^{®} Cannula 25/38 (a 0.50mm (25g) x 28mm cannula with 0.12mm (38g) x 5mm tip) was used, and the loading cell density was 444 viable cells / µL to yield an expected delivery of 336 +/- 40 viable cells / µL (N=6), yielding an expected delivery of 50,400 viable RPE in a volume of 150 µL into the subretinal space of each patient's eye.

### PATIENT SELECTION

Patients were selected based on a number of inclusion and exclusion criteria (Table 7 and Table 8, below), including end stage disease, central visual loss, the absence of other significant ophthalmic pathology, a cancer free medical history, current cancer screening, absence of contraindications for systemic immunosuppression, ability to undergo a vitreoretinal surgical procedure under monitored anesthesia care and psychological suitability to participate in a first in human clinical trial involving hESC derived transplant tissue.

### TRANSPLANTATION AND RATIONALE

Pars plana vitrectomy including surgical induction of posterior vitreous separation from the optic nerve anteriorly to the posterior border of the vitreous base was carried out. Submacular injection of 5 X 10⁴ hESC-RPE cells in a volume of 150µl was delivered into a pre-selected area of the pericentral macula that was not completely lost to disease. Transplantation sites were carefully chosen based on the presence of native, albeit compromised, RPE and overlying photoreceptors to optimize the chances of transplant integration and potential for photoreceptor cell rescue. Transplant attachment within a completely atrophic central macular pathoanotomic complex is unlikely and does not mimic central macular status in earlier stages of degeneration which may be the ultimate therapeutic target of a stem cell based regenerative transplant strategy.

Immunosuppression regimen includes low-dose tacrolimus (target blood levels 3-7 ng/mL) and mycophemolate mofetil (MMF ranging 0.25g - 2g orally/day) one week prior to the surgical procedure and continued for a period of 6 weeks. At week 6, the regimen calls for discontinuation the tacrolimus and a continuation of the MMF for an additional six weeks.

### RESULTS

### CHARACTERIZATION OF RPE

Controlled hESC differentiation resulted in near-100% pure RPE (Fig. 1). A single (9.6 cm2) 6-well plate of pigmented patches (Fig. 1A) produced approximately 1.5 X 10⁸ RPE cells (e.g., sufficient to treat 50 patients at a dosage of up to 3 X 10⁶ cells per patient). The cells displayed typical RPE behavior, losing their pigmented cobblestone morphology during proliferation (after trypsinization); once confluence was reestablished, they re-differentiated into a monolayer of polygonal cuboidal pigmented epithelium. Q-PCR showed that markers of pluripotency (Oct-4, NANOG, and SOX2) were significantly downregulated, whereas RPE markers RPE65, bestrophin, Pax6, MITF were expressed at high levels (Fig. 1B-F and Table 5). Immunostaining of mature cultures showed that bestrophin, a late marker of differentiated RPE, was organized in a membrane fashion in the majority of the cells prior to harvest; all (>99%) of the cells were positive for bestrophin and/or PAX6 (PAX6 became weaker or disappearing in more mature cells) and for ZO-1, a component of tight junctions (not shown). After cryopreservation, vials of cells were thawed and formulated for transplantation. Staining for retinal marker Pax6 and/or MITF (a marker of pigmented cells) confirmed 100% RPE purity (Fig. 1C). To further test the formulated cells, they were cultured for 2-3 weeks to allow for growth and maturation until the RPE morphology was established. Pax6/bestrophin (Fig.1E) and ZO-1 (Fig. 1G) immunostaining was similar to pre-harvest cultures, and a potency assay showed >85% of the cells phagocytized fragments of bioparticles (Fig. 1J).

### SAFETY STUDIES

Since the hESCs were exposed to animal cells and products, the MCB and RPE were extensively tested for animal and human pathogens. The cells were confirmed to be free of microbial contaminants at all stages, including animal and human viral pathogens (Table 3 below). The final RPE product had normal female (46, XX) karyotype (Fig. 1K) and a DNA fingerprint profile matching hESC-line MA09. Although the RPE manufacturing process was carried out under conditions that were non-supportive for pluripotent cells, a high sensitivity assay was performed to rule out the presence of any contaminating hESCs in the final RPE product. Examination of 2/9 million cell RPE samples (at P1/P2) stained for Oct-4 and alkaline phosphatase showed no presence of pluripotent cells. Tumorigenicity, biodistribution, and spiking studies carried out in NIH-III mice showed no adverse or safety issues in any of the animals. Additionally, no tumors were observed in animals injected with 50,000-100,000 RPE cells spiked with either 0.01%, 0.1%, or 1% undifferentiated hES cells. Survival of the human RPE cells was confirmed in the eyes of 100% of the animals up to 3 months after injection, and in 92% of the animals at 9 month (Table 6, below). Human RPE survived for the lifetime of the animals and integrated into the mouse RPE layer; although morphologically almost indiscernible from the host RPE cells (Fig. 2), they could be identified by immunostaining and expressed bestrophin in a typical baso-lateral fashion (Fig. 2B). Ki-67 staining showed a low level of proliferation 1 to 3 months after transplantation, but no Ki-67-positive cells were found at nine months indicating that the hESC-derived RPE had formed mature quiescent monolayers.

### STAGE OF DIFFERENTIATION IMPACTS CELL ATTACHMENT AND SURVIVAL

Attachment of the transplanted cells to Bruch's membrane, and their subsequent survival and integration into the host RPE layer is thought to be critical to the success of this therapeutic strategy. A distinguishing feature of hESC technology is that the degree of differentiation can be controlled *in vitro.* The extent of RPE differentiation is manifest in an array of modulated genotypic and phenotypic expression including the level of pigmentation. Cells maintained under similar conditions but harvested and cryopreserved at different time points display varying levels of pigmentation. Figure 3 shows two representative lots of cryopreserved RPE harvested at visibly different levels of pigmentation (melanin content was 4.8 ± 0.3 SD pg/cell and 10.4 ± 0.9 SD pg/cell for the lighter and more heavily pigmented lots, respectively). Cells from both RPE lots were processed and formulated using the protocol for clinical transplantation. After extrusion through the injection cannula, the cells were seeded in gelatin-coated tissue culture plates and monitored for attachment and subsequent growth. RPE cells from the lighter pigmented lot showed a minimal number of floating cells in overnight cultures; most of the cells had attached and spread, displaying typical RPE behavior and morphology for this stage of growth (Fig. 3A). After three days in culture, the number of RPE cells had increased from 4.0 X 10⁴ seeded to 10.6 X 10⁴ cells (Fig. 3C and Fig. 1G). In stark contrast, the more heavily pigmented RPE showed large numbers of floating cells; only a small percentage of the cells attached and survived, with a significantly decreased number of cells (less than one-tenth of that [9.0 X 103] seen in the lighter lot) after three days in culture (Fig. 3F and Fig. 3G). These results suggest a strong correlation between the stage of RPE differentiation and the ability to adhere and thrive *in vitro.* The RPE lot used in the current clinical study had a melanin content of 4.1 pg/cell and showed comparable attachment and growth to that of the lighter pigmented lot. Stresses associated with the freeze-thaw cycle, post-thaw washings, centrifugation, and formulation, as well as, extrusion through the injection cannula may account in part for the observed differences between lightly and heavily pigmented cells.

### CLINICAL RESULTS

The SMD patient is a 26 year old Caucasian female with baseline best corrected visual acuity (BCVA) of hand motion (HM) and was unable to read any letters on the Early Treatment Diabetic Retinopathy Study (ETDRS) visual acuity chart. At no point following transplantation were any signs of intraocular inflammation or hyperproliferation detected. Absence of clinically detectable inflammation was corroborated with slit lamp biomicroscopic photography, fundus photography, IVFA, and SD-OCT (further described in Example 2 and FIG. 8 and FIG. 9). Clinically increasing pigmentation at the level of the RPE was observed beginning at postoperative week 1, which appears to have spread outside the surgical transplant site (Fig. 4). Goldmann visual fields improved from baseline to two months post-transplantation (preoperative and postoperative fields are shown in FIG. 10 and FIG. 11). At week 2 BCVA was counting fingers (CF)(1 ETDRS letter), which continued to improve during the study period (5 ETDRS letters [BCVA 20/800] at 1 and 2 months) (Table 2). The patient is very reliable and worked for years as a graphic artist. She reported subjectively improved color vision and improved contrast and dark adaptation out of the operated eye with no change to the fellow eye.

**Table 2. Change in Visual Acuity After hESC-RPE Transplantation in the Operated Eye**

| **Dry AMD** | **BCVA*** | **ETDRS (# letters)**** | | **Stargardt's** | **BCVA** | **ETDRS (# letters)** |
|---|---|---|---|---|---|---|
| Baseline | 20/500 | 21 | | Baseline | Hand motion | 0 |
| 1 Week | 20/320 | 21 | | 1 Week | Counting fingers | 0 |
| 2 Weeks | 20/200 | 33 | | 2 Weeks | Counting fingers | 1 |
| 3 Weeks | 20/200 | 32 | | 3 Weeks | Counting fingers | 3 |
| 4 Weeks | 20/250 | 30 | | 4 Weeks | 20/800 | 5 |
| 6 Weeks | 20/250 | 28 | | 6 Weeks | 20/800 | 5 |
| 8 Weeks | 20/320 | 25 | | 8 Weeks | 20/800 | 5 |
| * BCVA = Best Corrected Visual Acuity | | | | | | |
| ** ETDRS = Early Treatment Diabetic Retinopathy Study (ETDRS) visual acuity chart | | | | | | |

The AMD patient is a 77 year old Caucasian female with baseline BCVA of 21 ETDRS letters (20/500). At no point following transplantation were any signs of intraocular inflammation or hyperproliferation detected despite moderate noncompliance with the immunosuppressive regimen. Absence of clinically detectable inflammation was corroborated with slit lamp biomicroscopic photography, fundus photography, IVFA, and SD-OCT (further described in Example 2 and FIG. 8 and FIG. 9). OCT images are shown in FIG. 4 and FIG. 7. At week 2 ETDRS BCVA was 33 letters (20/200). By week 6 BCVA was 28 ETDRS letters (20/320), and remained stable through week 8. Central scotoma measured by Goldmann visual field was slightly reduced in size at eight weeks compared to baseline.

### DISCUSSION

The therapeutic use of human embryonic stem cells poses daunting translational challenges. This report provides the first clinical evidence suggesting that hESC-derived cells can be safely transplanted into human patients. In the current study, a low dose (5 x 10⁴ cells) of RPE cells generated from hESCs was transplanted into the eyes of two patients with different forms of macular degeneration - dry AMD and SMD, the leading causes of adult and juvenile blindness in the developed world, respectively.

In order to improve the chances the cells would attach to Bruch's membrane, a submacular injection site was selected where the macular complex (photoreceptors, Bruch's membrane and RPE) was still present and potentially viable, thus increasing the expected likelihood that the transplanted cells would integrate with the native RPE and potentially rescue compromised peri-macular tissue. Both patients tolerated the transplant well and there were no signs postoperative inflammation, rejection, or tumorigenicity at the time of this report. Clinical and laboratory findings suggest that the transplanted RPE cells may have attached, integrated, and begun to influence the compromised native RPE.

Ongoing monitoring and assessment of the patients may determine whether the transplanted hESC-RPE have reduced immunogenicity, whether they might undergo rejection in the absence of immunosuppression in the long-term, and whether the visual gains observed will persist. It is expected that immune reactions, if any, can be managed through methods known in the art including immunosuppressive and/or tolerizing regimens. It is also expected that greater visual gains may be attainable through administration of greater numbers of RPE cells. Moreover, it is expected that administration of RPE cells will slow or arrest visual loss associated with conditions of retinal degeneration including AMD, SMD, and others.

Although the transplantation of intact sheets and suspensions of primary RPE cells has been previously attempted (11-19), RPE derived from adult organ donors are restricted in both their capacity to proliferate (23) and in their ability to differentiate *in vitro,* including the failure to express genes required for melanin biosynthesis using standard culture conditions (24). Clinically, sheets of adult RPE engrafted into the subretinal space of AMD patients have failed to improve visual function (25). Although RPE derived from pre- and post-natal tissue has been successfully dissociated and induced to grow and mature *in vitro* with attributes suggestive of fully differentiated RPE (26-28), such sources are extremely limited and variable with regard to quality and expansion capacity. In contrast to adult and fetal tissue, a feature of hESCs is that they have the capacity to proliferate indefinitely without undergoing senescence, providing a virtually unlimited source of 'youthful' cells as starting material for differentiation. Another expected advantage to using progeny obtained from hESCs is that the stage of *in vitro* differentiation can be controlled to maximize survival and functionality post-transplantation. Indeed, the data presented here shows that the extent of RPE maturity and pigmentation dramatically impacts subsequent attachment and growth of the cells *in vitro.*

The starting material for the RPE used in this study was a well-characterized hESC master cell bank generated using procedures optimized to reliably produce large quantities of pluripotent stem cells under controlled conditions. Although the RPE differentiation procedure is non-permissive for supporting hESC survival, extensive preclinical safety studies confirmed that the transplanted hESC-RPE did not cause ectopic tissue formation or tumors during the lifetime of the animals. An immunofluoresence-based assay capable of detecting less than one undifferentiated hES cell in over a million cells, confirmed that the clinical lot of RPE used in this study had no detectable pluripotent cells, representing a level of detection five orders of magnitude lower than the dose of hESCs shown to cause tumors in *in vivo* spiking studies. The generation of the hESC-MCB and the manufacture of each lot of RPE cells involved propagation on primary mouse embryo fibroblasts feeder layers. The hESC-RPE is therefore classified as a xenotransplantation product and was subject to all the testing and monitoring mandated by the FDA xenotranplantation guidelines to ensure that the cells were free of murine pathogens. The RPE also underwent an extensive battery of safety tests to confirm the absence of microbial contaminants and viruses, and was characterized by a variety of RPE-specific attributes including the ability to phagocytose, gene-expression, morphological evaluations, and immunohistochemical staining for RPE-specific markers. Prior to initiating these clinical trials, transplantation of hESC-RPE into dystrophic animals showed that the cells were capable of rescuing photoreceptors and visual function in a dose dependent fashion.

The current study is designed to test the safety and tolerability of hESC-RPE in patients with advanced-stage SMD and dry-AMD. To-date, the cells appear to have transplanted into both patients without abnormal proliferation, teratoma formation, graft rejection or other untoward pathological reactions. Continued follow-up and further study is indicated. However, the ultimate therapeutic goal will be to treat patients earlier in the disease processes, potentially increasing the likelihood of photoreceptor and central visual rescue.

### EXAMPLE 2

This example provides supplemental information and methods relating to Example 1.

Characteristics of the clinical hESC master cell bank (hESC-MCB) (from which the RPE cells that were used in Example 1 were produced) are shown in Table 3.

**Table 3 Characterization of MA09 hESC Master Cell Bank**

| **Test** | **Test method for MCB** | **hESC MCB** |
|---|---|---|
| Sterility | USP <71> inoculation method (WuXi SOP 30744) | Negative |
| Mycoplasma | Indirect culture with Hoechst stain and direct culture (WuXi SOP 30055) | Negative |
| Retroviruses: | Co-cultivation with *Mus dunni* and PG-4 (S⁺L⁻) cells for detection of retrovirus (WuXi SOP 30201) | Negative |
| | PCR-based viral reverse transcriptase detection (WuXi SOP 30357) | Negative |
| | Ultrastructural electron microscopy of cellular morphology and detection of viral particles (WuXi SOP 30610) | Negative |
| *In vitro* detection of viruses on cells | Incubation with MRC-5, VERO, NIH3T3 and HeLa cells (WuXi SOP 37000E) - cytopathic effect | Negative |
| | - haemadsorption | Negative |
| | - haemagglutination | Negative |
| *In vivo* detection of inapparent viruses | Inoculation into suckling and adult mice (WuXi SOP 30194) | Negative |
| | Inoculation into guinea pigs | Negative |
| | Inoculation into embryonated hen eggs - allantoic and yolk sac routes | Negative |
| Minute virus of mice (MVM) | Detection of MVM DNA by qPCR (WuXi SOP 30910) | Negative |
| Mouse antibody production | Antibody titers on inoculated mice for 19 viruses, LDHEV and LCMV (WuXi SOP 30001) | Negative |
| XC plaque assay | *In vitro* detection of murine ecotropic virus (extended duration) (WuXi SOP 30024) | Negative |
| Bovine viruses | Detection of adventitious bovine viruses (WuXi SOP 30236) | Negative |
| Porcine viruses | Detection of adventitious porcine viruses (WuXi SOP 30129) | Negative |
| Hepatitis B virus | Detection of HBV DNA by qPCR (WuXi SOP 32827) | Negative |
| Hepatitis C virus | Detection of HCV RNA by qPCR (WuXi SOP 30730) | Negative |
| Herpes simplex 6A and 6B | Detection of human HSV6A and HSV6B DNA by qPCR (WuXi SOP 30863) | Negative |
| Human immunodeficiency virus (HIV) type 1 | Detection of HIV-1 DNA by qPCR (WuXi SOP 30768) | Negative |
| HIV type 2 | Detection of HIV-2 DNA by qPCR (WuXi SOP 30798) | Negative |
| Human T-cell lymphotropic virus (HTLV) type 1 | Detection of HTLV-1 DNA by qPCR (WuXi SOP 32491) | Negative |
| HTLV type 2 | Detection of HTLV-2 DNA by qPCR (WuXi SOP 32492) | Negative |
| Human cyto-megalovirus (hCMV) | Detection of hCMV DNA by qPCR (WuXi SOP 30705) | Negative |
| Human Epstein Barr virus (hEBV) | Detection of hEBV DNA by qPCR (WuXi SOP 30713) | Negative |
| Human parvovirus B19 | Detection of human parvovirus B19 DNA by qPCR (WuXi SOP 30761) | Negative |
| DNA fingerprinting | Short tandem repeat (STR) profile (CLG SOP 401) | Conforms to expected pattern |
| Karyotype with G-banding | Cytogenetic analysis of 20 G-banded metaphase cells (CLG SOP 100) | 46 normal female |
| FISH analysis | 200 interphase nuclei assayed by FISH for chromosomes 12 and 17 (CLG SOP 201) | Normal signal patterns |
| Expression of hES specific markers | qPCR for hESC markers *OCT-4, REX-1, NANOG* and SOX-2 (ACT Quality SOP-0022) | Within 1 log₁₀ of control reference bank values |
| Absence of retinal degeneration gene mutations | Screening for mutant forms of *ABCA4, ELOVL4, VMD2, RPE-*65 and *CTRP5* genes by PCR and sequence analysis (Ophthalmic Molecular Diagnostic Laboratory at the University of California) | No mutations detected |
| Morphology | Microscopic evaluation of cells and colonies (ACT BR-009A) | Conforms to hESC morphology |

### Mouse Embryo Fibroblast (MEF) Master Cell Bank

In accordance to the April 2003 Guidance for Industry "Source Animal, Product, Preclinical, and Clinical Issues Concerning the Use of Xenotransplantation Products in Humans" and "Points to Consider on Xenogeneic Cell Therapy Medicinal Products" (EMEA/CHMP/CPWP/83508/2009)] the MA09-hRPE cells are defined as a xenotransplantation product since these human cells have had *ex vivo* contact with nonhuman (murine) cells. The breeding colony at Charles River Laboratories (Kingston Facility, Stoneridge, NY, USA) was used as the source of MEF cells. This AAALAC accredited facility (Association for Assessment and Accreditation of Laboratory Care International) houses a closed colony of CD-1, Specific Pathogen Free (SPF) mice in a barrier room under extensive health monitoring. The donor animals were time-mated and segregated during pregnancy. Twelve days post-mating prior to sacrifice, physical health examinations were performed on all mice by a veterinarian; animals were euthanized, and blood was collected from each donor mouse: for leukocyte and plasma preparation to be archived and serological testing for murine pathogens by Charles River Laboratories, Wilmington, MA. A board-certified veterinary pathologist performed a necropsy on the carcass and uterus of each donor animal and on one embryo from each litter. Organs from each animal were archived for at least 30 years along with plasma and cryopreserved leukocytes (as required by EMEA/CHMP/CPWP/83508/2009). MEF were isolated and cultures as previously described (Klimanskaya and McMahon, 2005), frozen at P1 and used at P2 after Mitomycin C inactivation. To minimize the risk of introducing murine viruses and other pathogens, MEF were tested and characterised by WuXi AppTec, Inc. The specifications and results for testing of lot MEF-08 used in the preparation of the hESC-MCB and the hRPE clinical lot are presented in Table 4.

**Table 4 Characterization of MEF Master Cell Bank**

| **Test** | **Method** | **Specification** | **Lot MEF-08** |
|---|---|---|---|
| Sterility | USP - inoculation method (WuXi SOP 30744) | Negative | Negative |
| Mycoplasma | Indirect culture with Hoechst stain and direct culture (WuXi SOP 30055) | Negative | Negative |
| Retroviruses: | Co-cultivation with *Mus dunni* and PG-4 (S⁺L⁻) cells for detection of retrovirus (WuXi SOP 30201) | Negative | Negative |
| | PCR-based viral reverse transcriptase detection (WuXi SOP 30357) | Negative | Negative |
| | Ultrastructural electron microscopy of cellular morphology and detection of viral particles (WuXi SOP 30610) | Negative | Negative |
| *In vitro* detection of viruses on cells | Incubation with MRC-5, VERO and NIH3T3 cells (WuXi SOP C30177) - cytopathic effect | Negative | Negative |
| | - haemadsorption | Negative | Negative |
| | - hemagglutination | Negative | Negative |
| *In vivo* detection of inapparent viruses | Inoculation into suckling and adult mice (WuXi SOP 30194) | Negative | Negative |
| | Inoculation into guinea pigs | Negative | Negative |
| | Inoculation into embryonated her eggs - allantoic and yolk sac routes | Negative | Negative |
| Minute virus of mice (MVM) | Detection of MVM DNA by qPCR (WuXi SOP 30910) | Negative | Negative |
| Mouse antibody production | Antibody titers on inoculated mice for 19 viruses, lactate dehydrogenase elevating virus (LDHEV) and lymphocytic choriomeningitis virus (LCMV) (WuXi SOP 30001) | Negative | Negative |
| *In vitro* tumorigenicity | Colony formation in soft agar (WuXi SOP 30006) | Negative | Negative |
| Cell line ID testing | Isoenzyme electrophoresis mobility profiles (WuXi SOP 30330) | Mouse isoenzymes | Isoenzyme pattern representative of mouse cells |
| XC plaque assay | *In vitro* detection of murine ecotropic virus (extended duration) (WuXi SOP 30024) | Report results | Positive: ecotropic MuLV detected¹ |
| MEF performance qualification | Test ability of MEF to support growth and attributes of MA-09 hES cells in culture (ACT SOP QCS-0004) | Comparable to control MEF | Pass |

¹ Murine cell lines are inherently capable of producing infectious murine retroviruses and evidence has been presented indicating the murine leukaemia (MuLV) virions produced by MEFs are non-infectious and are replication incompetent to human cells (Amit *et al* 2004).

### DNA Q-PCR for Human DNA

Detection of human DNA content in mouse tissues was performed by AltheaDX, Inc., San Diego, CA, using Taqman assay for Alu Y sequence with sensitivity 1 human cell per 150,000 mouse cells.

**Table 5 RPE Cell Characterization and Safety Testing**

| **Test** | **Specification** | **Lot 0211-B1A** | |
|---|---|---|---|
| Sterility | Negative | Negative | |
| Mycoplasma | Negative | Negative | |
| Cell density | 1-2 million viable cells/mL (post dilution) | 2 x 10⁶ viable cells/mL | |
| Cell viability | Final harvest: > 85% | 99% | |
| | Post-thaw: >70% | 95% | |
| Morphology | Confluent, cobblestone epithelium, medium pigmentation | Pass | |
| Karyotype | 46, XX, normal | 46, XX, normal | |
| DNA fingerprinting | Conforms with hESC MCB | Conforms | |
| hRPE mRNA for: *BEST-1* | Up-regulated by a minimum of 1 log₁₀ compared to hESC | RPE-6 | 1.32 |
| *RPE-65* | | PAX6 | 2.80 |
| *PAX6* | | MITF | 2.89 |
| *MITF* | | BEST-1 | 3.81 |
| | Down-regulated compared to hESC (log₁₀): | | |
| hESC mRNA for: *OCT-4* | *OCT-4* ≤ -2.13 | OCT-4 | -3.18 |
| *NANOG* | *NANOG* ≤ -1.95 | NANOG | -2.49 |
| *SOX-2* | *SOX-2* ≤ -0.63 | SOX-2 | -2.07 |
| Maturity by bestrophin staining | > 70% staining | 71% | |
| Purity by immunostaining | > 95% PAX6 and/or MITF | 100% | |
| | > 95% PAX6 and/or bestrophin | 100% | |
| | > 95% ZO-1 | 100% | |
| hESC protein markers | < 2 cells staining with OCT-4 and AP in 9 million cells examined | 0 | |
| Residual murine DNA | Negative | Negative | |
| Murine viruses by MAP | Negative | Negative | |
| Retroviruses by *Mus dunni* co-cultivation | Negative | Negative | |
| Ecotropic murine viruses | Negative | Negative | |
| Endotoxin | < 0.50 EU/mL | 0.312 EU/mL | |
| Potency by phagocytosis | Positive | Positive | |

### Immunostaining of Cells

Cells in 4-well or 6-well plates were fixed with 2% paraformaldehyde (Electron Microscopy Sciences) in PBS for 10 minutes, permeabilization for ten minute in 0.1% NP-40 substitute (Sigma) in PBS, blocked with 10% Goat Serum in PBS for 1 h or longer, and incubated with primary antibodies overnight at 4°C. Cells were then washed 3 x15 minutes in 0.1% Tween/PBS, incubated with secondary antibodies for 1 h at RT, washed as above and mounted using Vectashield with DAPI (Vector laboratories, Burlingame, CA). Stained cells were examined under an inverted fluorescence microscope (Nikon). Antibodies used were: bestrophin (Novus Biologicals), Pax6 (Covance), MITF (Abcam), ZO-1-FITC (Invitrogen), Oct-4 (Santa Cruz Biotechnologies), anti-mouse-Alexa594 (Invitrogen), anti-rabbit-FITC (Jackson Immunoresearch), anti-mouse-Alexa-488 (Invitrogen), anti-rabbit-Alexa-594 (Invitrogen). Alkaline phosphatase activity was detected using Vector blue kit (Vector Laboratories).

### Immunostaining of Mouse Tissue Sections

Deparaffinized sections were incubated in 0.1M citrate buffer (pH 6.0) in a steamer for 40 minutes for antigen retrieval (bestrophin and human mitochondria), or for 30 minutes in a pressure cooker for Ki67. Antibody staining was performed as described above, but biotin-conjugated secondary antibodies were used in some instances after blocking endogenous biotin using a kit from Vector (Burlingame, CA). Antibodies used were anti-bestrophin (Abcam, rabbit), anti-human mitochondria (mouse ,Spring Bioscience), anti-ki67 (rabbit, Abcam). Secondary antibodies were anti-mouse-biotin, anti-mouse-Cy3 (Jackson Immunoresearch), anti-rabbit-Alexa488 (Invitrogen), and Streptavidn-Cy3 was from Jackson Immunoresearch. Sections of mouse teratoma formed by hESC were used as a positive control for anti-human mitochondria and Ki67, and sections of an hRPE pellet fixed and embedded in paraffin were used as a positive control for bestrophin. Negative controls were mouse rabbit and mouse IgG (Novus Biologicals).

### q-RT-PCR

The RNeasy RNA isolation kit from Qiagen was used to extract RNA from the cell mixtures resulting in a final volume of 30 µL RNA per sample. cDNA was then synthesized from 10 µL of RNA with the Quantitect cDNA synthesis kit from Qiagen resulting in a final volume of 20 µL cDNA. One µL of cDNA was then tested for relative gene expression in triplicate replicates normalized to the beta actin signal present in each sample. Gene expression profiling was performed using the Applied Biosystems StepOne Plus with software version 2.1 and TaqMan gene expression assays from Life Technologies following the manufacturer's recommended cycle conditions for comparative Ct relative quantification. qRT-PCR assays for hES markers: Nanog, OCT4 and SOX2 and hRPE markers: RPE-65, PAX-6, MITF and bestrophin were normalized to the level of expression observed in the 100% hES cell sample (RQ = Relative Quantitation) which serves as the zero set point. Relative gene expression was assayed in triplicate replicates normalized to the beta actin signal present in each sample. Data are expressed as the mean +/- SD for three replicates.

### Phagocytosis Assay

Phagocytosis is assessed by a FACS-based assay using pHrodo^{™} *e coli* fluorescent bioparticles (Invitrogen) which fluoresce when internalized in the reduced pH environment of intracellular phagosomes. Bioparticles were prepared according to the manufacturer's instructions. Confluent RPE were incubated with 50-200µL bioparticles per one well of a 4-well plate in CO₂-independent medium (Invitrogen) for 16-20 hours at 37°C. Negative control plates were incubated at 4°C. Cells were examined under the microscope, harvested by trypsin and analyzed by FACS counting 10,000 events on a C6 Flow Cytometer.

### Melanin Determinations

RPE cell suspensions were centrifuged at 160 X G for 5 minutes at room temperature and samples were removed for hemocytometer cell counts. Pellets were resuspended in 1N NaOH and heated to 80°C for 10 minutes, vortexed, and absorbances measured at 475nm against a synthetic melanin (Sigma Cat# 8631) standard curve ranging from 5 to 180 µg/mL. Samples were assessed in triplicate and data normalized to the total cell number extracted.

**Table 6 Survival of RPE in the Subretinal Space of NIH-III Mice**

| Data in Table 6 below were compiled from three studies: 1) a tumorigenicity study in which 100,000 hES-RPE were injected into the eye, and the animals were terminated at 4, 12, and 40 weeks 2) a spiking study in which 100,000 hES-RPE spiked with 0.01%, 0.1%, and 1% of pluripotent hES cells were injected into the eye and the animals were terminated at 2 and 9 months and 3) a tissue distribution study in which 50,000 and 100,000 hES-RPE were injected into the eye, and the animals were terminated at 1, 3, and 9 months. The Table includes data obtained by both Q-PCR of the whole eye for human DNA and immunostaining of paraffin sections for human mitochondria. | | | | |
|---|---|---|---|---|
| **Survival Time (weeks)** | **Total Number of Animals** | **Number of Animals with Human Cells Found in the Eye** | **% of Animal with Human Cells Surviving in the Eye** | |
| 4 | 26 | 26 | 100% | |
| 8 | 19 | 19 | 100% | |
| 12 | 28 | 28 | 100% | |
| 36-40 | 52 | 48 | 92% | |

**Table 7. Inclusion/Exclusion Criteria for AMD Study**

| | | | | |
|---|---|---|---|---|
| **INCLUSION CRITERIA** | • Adult male or female over 55 years of age. | | | |
| | • Patient should be in sufficiently good health to reasonably expect survival for at least four years after treatment | | | |
| | • Clinical findings consistent with advanced dry AMD with evidence of one or more areas of >250microns of geographic atrophy (as defined in the Age-Related eye Disease Study [AREDS] study) involving the central fovea. | | | |
| | • GA defined as attenuation or loss of RPE as observed by biomicroscopy, OCT, and FA. | | | |
| | • No evidence of current or prior choroidal neovascularization | | | |
| | • The visual acuity of the eye to receive the transplant will be no better than 20/400. | | | |
| | • The visual acuity of the eye that is NOT to receive the transplant will be no worse than 20/400. | | | |
| | • Electrophysiological findings consistent with advanced dry AMD. | | | |
| | • Medically suitable to undergo vitrectomy and subretinal injection. | | | |
| | • Medically suitable for general anesthesia or waking sedation, if needed. | | | |
| | • Medically suitable for transplantation of an embryonic stem cell line: | | | |
| | | Any laboratory value which falls slightly outside of the normal range will be reviewed by the Medical Monitor and Investigators to determine its clinical significance. If it is determined not to be clinically significant, the patient may be enrolled into the study. | | |
| | | | • Normal serum chemistry (sequential multi-channel analyzer 20 [SMA-20]) and hematology (complete blood count [CBC], prothrombin time [PT], and activated partial thromboplastin time [aPTT]) screening tests. (NOTE: With the exception of abnormalities specifically identified in the exclusion crieteria) | |
| | | | • Negative urine screen for drugs of abuse. | |
| | | | • Negative human immunodeficiency virus (HIV), hepatitis B (HBV), hepatitis C (HCV) serologies. | |
| | | | • No history of malignancy (with the exception of successfully treated (excised) basal cell carcinoma [skin cancer] or successfully treated squamous cell carcinoma of the skin). | |
| | | | • Negative cancer screening within previous 6 months: | |
| | | | | • complete history & physical examination; |
| | | | | • dermatological screening exam for malignant lesions; |
| | | | | • negative fecal occult blood test & negative colonoscopy within previous 7 years; |
| | | | | • negative chest roentgenogram (CXR); |
| | | | | • normal CBC & manual differential; |
| | | | | • negative urinalysis (U/A); |
| | | | | • normal thyroid exam; |
| | | | | • if male, normal testicular examination; digital rectal examination (DRE) and prostate specific antigen (PSA); |
| | | | | • if female, normal pelvic examination with Papanicolaou smear; and |
| | | | | • If female, normal clinical breast exam and, negative mammogram. |
| | • If female and of childbearing potential, willing to use two effective forms of birth control during the study. | | | |
| | • If male, willing to use barrier and spermicidal contraception during the study. | | | |
| | • Willing to defer all future blood, blood component or tissue donation. | | | |
| | | • Able to understand and willing to sign the informed consent. | | |

| | | | | **Exclusion Criteria** |
|---|---|---|---|---|
| | | | | • Presence of active or inactive CNV. |
| | | | | • Presence or history of retinal dystrophy, retinitis pigmentosa, chorioretinitis, central serious choroidopathy, diabetic retinopathy or other retinal vascular or degenerative disease other than ARMD. |
| | | | | • History of optic neuropathy. |
| | | | | • Macular atrophy due to causes other than AMD. |
| | | | | • Presence of glaucomatous optic neuropathy in the study eye, uncontrolled IOP, or use of two or more agents to control IOP (acetozolamide, beta blocker, alpha-1-agonist, antiprostaglandins, anhydrous carnonic inhibitors). |
| | | | | • Cataract of sufficient severity likely to necessitate surgical extraction within 1 year. |
| | | | | • History of retinal detachment repair in the study eye. |
| | | | | • Axial myopia of greater than -8 diopters |
| | | | | • Axial length greater than 28 mm. |
| | | | | • History of malignancy (with the exception of successfully treated [excised] basal cell carcinoma [skin cancer] or successfully treated squamous cell carcinoma of the skin). |
| | | | | • History of myocardial infarction in previous 12 months. |
| | | | | • History of diabetes mellitus. |
| | | | | • History of cognitive impairments or dementia which may impact the patient's ability participate in the informed consent process and to appropriately complete evaluations. |
| | | | | • Any immunodeficiency. |
| | | | | • Any current immunosuppressive therapy other than intermittent or low dose corticosteroids. |
| | | | | • Alanine transaminase/aspartate aminotransferase (ALT/AST) >1.5 times the upper limit of normal or any known liver disease. |
| | | | | • Renal insufficiency, as defined by creatine level ≥1.3 mg/dL. |
| | | | | • A hemoglobin concentration of less than 10gm/dL, a platelet count of less than 100k/mm³ or an absolute neutrophil count of less than 1000/mm³ at study entry. |
| | | | | • Serologic evidence of infection with Hepatitis B, Hepatitis C, or HIV. |
| | | | | • Current participation in any other clinical trial. |
| | | | | • Participation within previous 6 months in any clinical trial of a drug by ocular or systemic administration. |
| | | | | • Any other sight-threatening ocular disease. |
| | | | | • Any history of retinal vascular disease (compromised blood-retinal barrier. |
| | | | | • Glaucoma. |
| | | | | • Uveitis or other intraocular inflammatory disease. |
| | | | | • Significant lens opacities or other media opacity. |
| | | | | • Ocular lens removal within previous 3 months. |
| | | | | • Ocular surgery in the study eye in the previous 3 months |
| | | | | • If female, pregnancy or lactation. |
| | | | | • Any other medical condition, which, in the Investigator's judgment, will interfere with the patient's ability to comply with the protocol, compromises patient safety, or interferes with the interpretation of the study results. |

**Table 8. Inclusion/Exclusion Criteria for SMD Study**

| | | | | |
|---|---|---|---|---|
| **INCLUSION CRITERIA** | | | | • Adult male or female over 18 years of age. |
| | | | | • Clinical diagnosis of advanced SMD. |
| | | | | • If known, the patient's genotype will be recorded in the medical history, if unknown, patient will allow for the submission of a sample for genotyping. Clinical findings consistent with SMD. |
| | | | | • The visual acuity of the eye to receive the transplant will be no better than hand movement. |
| | • The visual acuity of the eye that is not to receive the transplant will be no better than 24 (20/320) Early Treatment of Diabetic Retinopathy Study (ETDRS) letters. | | | |
| | • Peripheral visual field constriction documented on standard visual field testing. | | | |
| | • Electrophysiological findings consistent with SMD. | | | |
| | • Medically suitable to undergo vitrectomy and subretinal injection. | | | |
| | • Medically suitable for general anesthesia or waking sedation, if needed. | | | |
| | • Medically suitable for transplantation of an embryonic stem cell line: | | | |
| | | | • Normal serum chemistry (sequential multi-channel analyzer 20 [SMA-20]) and hematology (complete blood count [CBC], prothrombin time [PT], and activated partial thromboplastin time [aPTT]) screening tests. | |
| | | | • Negative urine screen for drugs of abuse. | |
| | | | • Negative human immunodeficiency virus (HIV), hepatitis B (HBV), hepatitis C (HCV) serologies. | |
| | | | • No history of malignancy. | |
| | | | • Negative cancer screening within previous 6 months: | |
| | | | | • complete history & physical examination; |
| | | | | • dermatological screening exam for malignant lesions; |
| | | | | • negative fecal occult blood test & if over age 50 years, negative colonoscopy within previous 7 years; |
| | | | | • negative chest roentgenogram (CXR); |
| | | | | • normal CBC & manual differential; |
| | | | | • negative urinalysis (U/A); |
| | | | | • normal thyroid exam; |
| | | | | • if male, normal testicularexamination; if over age 40, digital rectal examination (DRE) and prostate specific antigen (PSA); |
| | | | | • if female, normal pelvic examination with Papanicolaou smear; and |
| | | | | • if female, normal clinical breast exam and if 40 years of age or older, negative mammogram. |
| | • If female and of childbearing potential, willing to use two effective forms of birth control during the study. | | | |
| | | • If male, willing to use barrier and spermicide contraception during the study. | | |
| | • Willing to defer all future blood, blood component or tissue donation. | | | |
| | • Able to understand and willing to sign the informed consent. | | | |
| | **Exclusion Criteria** | | | |
| | • History of malignancy. | | | |
| | • History of myocardial infarction in previous 12 months. | | | |
| | • History of diabetes mellitus. | | | |
| | • Any immunodeficiency. | | | |
| | • Any current immunosuppressive therapy other than intermittent or low dose corticosteroids. | | | |
| | • Serologic evidence of infection with Hepatitis B, Hepatitis C, or HIV. | | | |
| | • Current participation in any other clinical trial. | | | |
| | • Participation within previous 6 months in any clinical trial of a drug by ocular or systemic administration. | | | |
| | • Any other sight-threatening ocular disease. | | | |
| | • Any chronic ocular medications. | | | |

| | | | | **Exclusion Criteria** |
|---|---|---|---|---|
| | | | | • Any history of retinal vascular disease (compromised blood-retinal barrier. |
| | | | | • Glaucoma. |
| | | | | • Uveitis or other intraocular inflammatory disease. |
| | | | | • Significant lens opacities or other media opacity. |
| | | | | • Ocular lens removal within previous 3 months. |
| | | | | • If female, pregnancy or lactation. |
| | | | | • Any other medical condition, which, in the Investigator's judgment, will interfere with the patient's ability to comply with the protocol, compromises patient safety, or interferes with the interpretation of the study results. |

### EXAMPLE 3

### Adjustment of cell density to ensure accurate dosage delivery

This study describes determination of the impact of steps in the loading and injection process on delivery of viable RPE. Specifically, in this example it was shown that the loading and injection process results in some loss of viable cells, that this loss can be readily measured (and may vary with the delivery protocol, e.g., depending on the specific injection cannula used), and that this loss can be accounted for by increasing the concentration of cells, allowing delivery of the expected number of cells. Additionally, it was shown that cell seeding and growth was not significantly adversely impacted following loading and extrusion through two cannulas.

These studies incorporated the entire loading and injection process including: (1) Final addition of cold BSS-Plus to concentrated final product RPE cells at 2000 viable cell/µL to obtain the desired density of cells to be injected. (2) Gentle mixing of the RPE cells and BSS-Plus using a 18g blunt fill needle (BD) attached to the 1 ml injection syringe (BD LUER-LOK (TM). (3) Extrusion of 150 µL of formulated RPE cells from the filled syringe through the injection cannula.

Maintenance of RPE cells in Alcon BSS BSS-Plus(R) on ice was demonstrated constant over 4 hours provided that the cells are formulated to a concentration of 1000 cells/µL or more. Under these conditions, there is no detectable loss in viable cell number. To ensure cell integrity, an exact volume RPE final product cells will be delivered to the operating room at 2000 viable cells/µL. Each tube of RPE cells will be accompanied by a second tube containing the exact volume of cold BSS-Plus to be added to the cells and mixed just prior to injection. The predispensed RPE cells and BSS-Plus will be delivered to the OR at 2-8 degrees C in sterile microcentrifuge tubes.

### Study 1 - MEDONE POLYTIP (R) Cannula 23/38

RPE cells similar in characteristics to the intended clinical RPE lot were thawed, processed and formulated in cold BSS-Plus as described in Example 1. A total of 4.1 million viable cells were recovered post-thaw and formulation. The starting viability was 91% and the number of cells recovered post-thaw and formulation were typical for this lot. Cells were diluted to the indicated starting concentrations in cold BSS-Plus and stored on ice. Cells were then gently triturated using a 18g blunt fill needle (BD) attached to a 1 mL syringe (BD LUER-LOK TM). Approximately 200 µL of cells were transferred into the syringe through the fill needle. The fill needle was removed and a MEDONE POLYTIP (R) Cannula 23/38 was attached to the syringe containing cells. The plunger of the syringe was gently tapped to administer 150 L of cells through the injection cannula. The total time of the injection was 2-3 minutes. Cells were collected in a sterile tube and assessed for viable cell number.

Study 1 demonstrated that RPE cells loaded and extruded through the MedOne cannula results in a predictable loss in cell density delivered over the range of cell densities tested (295 to 1144 viable cells/µL). The mean loss in viable cell density was 22.8 +/- 7.0% (N=6). Results are shown in Table 9 below.

**Table 9. Loss of viable cells after delivery through the MEDONE POLYTIP (R) Cannula 23/38. Mean decrease in the percent of cell delivered was 22.8 +/- 7.0 % viable cells/ L (N=6).**

| **Starting cells / µL** | **Extruded cells / µL** | **% Decrease after loading and cannula delivery** |
|---|---|---|
| 1144 | 883 | 23 |
| 830 | 615 | 26 |
| 668 | 495 | 26 |
| 532 | 440 | 18 |
| 335 | 296 | 12 |
| 295 | 199 | 32 |

Decreases in the number of cells delivered through the injection cannula were observed at all the cell densities tested ranging from 295 to 1144 viable cells/µL. The percentage decrease in cell density appears generally constant over the range tested. The percent decreases observed at the two lowest densities tested (199 and 296) are more variable and probably reflect the accuracy of cell counting are these lower cell densities.

Cells extruded through the MedOne cannula, control cells formulated but not extruded through the cannula were centrifuged, resuspended in RPE growth medium and seeded in gelatin coated full area 96-well plates at 10,000 cells per well. For comparison, portions of the same cell preparation were loaded and passed through the Synergetics cannula (39ga Rigid Micro Injection Cannula, Angled) and processed and seeded as above. Four days post-seeding, cells were trypsinized and counted. Table 10 below shows the mean cell number +/- SD for three cells counts.

**Table 10. Cell seeding and growth after loading and extrusion through a cannula.**

| **Cell Number after 4 Days in Culture (cells were seeded at 10,000 cells per well)** | |
|---|---|
| Control Cells | 20533 +/ 3085 (N=3) |
| Extruded Synergetics | 21047 +/- 1702 (N=3) |
| Extruded MedOne | 24460 +/5207 (N=3) |

Subsequent seeding and growth of cells extruded through either cannulas were comparable to control cells not extruded through the cannula.

### Study 2 - Synergetics, Inc. Injection Cannula, Angled, 39g

RPE cells from a lot similar in characteristics to the intended clinical RPE lot were thawed, processed and formulated in cold BSS-Plus as described in Example 1. A total of 2.6 million viable cells were recovered post-thaw and formulation. The starting viability was 97% and the number of cells recovered post-thaw and formulation were typical for this lot. Cells were diluted to a starting concentration of 375 viable cells/µL in cold BSS-Plus and stored on ice. Cells were then gently triturated using a 18g blunt fill needle (BD) attached to a 1mL syringe (BD LUER-LOK^{™}). Approximately 200 L of cells were transferred into the syringe through the fill needle. The fill needle was removed and a Synergetics, Inc. 39ga Rigid Micro Injection Cannula, Angled was attached to the syringe containing cells. The plunger of the syringe was gently tapped to administer 150 L of cells through the injection cannula. The total time of the injection was 2-3 minutes. Cells were collected in a sterile tube and assessed for viable cell number. Over a series of eight injections, the mean viable cells delivered was 238 +/- 25 viable cells/µL or approximately 100 viable cells less than the delivery intended for the lowest cell dose in this study (50,000 cells per eye).

Thus, Study 2 demonstrated that RPE cells loaded and extruded through the Synergetics cannula resulted in a predictable loss in cell density in range of the lowest intended cell dose (a loading density of 375 viable cells/µL was tested). The mean loss in viable cell density was 38.4 +/- 6.8%. (N=8). The loading cell density can be increased accordingly to compensate for the anticipated losses, thus ensuring accurate delivery of the intended number of viable RPE (such as 50,000 cells/eye as in the present study).

### Study 3 - MedOne POLYTIP(R) Cannula 23/38 and Synergetics 39ga Rigid Micro Injection Cannula, Angled

Study 3 was conducted with the RPE lot used for patient administration in Example 1 above. In this study, RPE cells were loaded at 25% higher than the dose-to-deliver cell density to compensate for anticipated losses in loading the syringe and injection through the MedOne cannula. The same 25% compensated loading density was used to test the Synergetics cannula.

When loaded with cells formulated 25% higher than the low target dose (444 viable cells/µL to deliver 333 cells/µL), the MedOne cannula delivered 336 +/- 40 viable cells/µL. Similarly, when loaded with cells formulated 25% higher than the target dose (1776 viable cells/µL to deliver 1,333 cells/µL), the MedOne cannula delivered 1433 +/- 187 viable cells/µL. Results for the lowest cell dose injections using the Synergetics cannula confirmed that an addition increase in loading cell density of 100 viable cells/µL would achieve the target dose at the low density.

Eight vials (total 16 million cells) were thawed and processed as described above (3 centrifugations), all processing was performed at RT. Yield was 3.78 million cells (23.6% recovery, similar with previous thaws) @ 95% viability. Cells were resuspended to storage and transport density of 2 million viable cells/ml (2,000 viable cells /µL) in cold BSS-Plus and kept thereafter on ice. A cell density of greater than 1 million cell/mL was selected to promote cell survival during cold-storage in BSS-Plus. Twenty one aliquots of 89 µL containing 177,600 total viable cells were dispensed into the final product closure microcentrifuge tubes.

Cell aliquots were stored on ice until the final dilution was performed at the time of syringe loading and extrusion through the cannula. For low dose deliveries (50,000 viable RPE/eye), 311 µL of cold BSS-Plus was dispensed into a tube containing cells to bring the final volume to 400 µL @ 444 cells/µL. This density is 25% higher than the intended delivery density of 333 cells/ µL to compensate for anticipated losses that occur when mixing with the fill needle, syringe loading, and delivery through the MedOne cannula.

For high dose deliveries (200,000 viable RPE /eye), two 89 µL aliquots of cells were pooled into one tube (356,000 cells) and 22 µL of cold BSS-Plus was dispensed into the tube containing the cells to bring the final volume to 200 µL @ 1,776 cells/ µL. This density is 25% higher than the intended delivery density of 1,333 cells/ L to compensate for anticipated losses that occur when mixing with the fill needle, syringe loading, and delivery through the MedOne cannula.

Microcentrifuge tubes containing diluted cell were capped and gently tapped with one finger to promote mixing. The blunt fill needle (void volume of 90 µL) was attached to the 1 mL BD syringe and cells were gently triturated 1-2 times in the blunt fill needle taking care to minimize contact with the syringe. The syringe was filled with approximately 200 µL of cells. The blunt needle was removed and the injection cannula was attached (MedOne 38g or Synergenic 39g). Approximately 150 µL of cells were dispensed into a microcentrifuge tube. Each dispensed aliquot was assessed for cell density and viability by trypan blue exclusion. These results are summarized in Tables 11 and 12 below.

**Table 11. Effect of reconstitution and delivery through the MedOne cannula on RPE cell number and viability.**

| **MedOne Cannula** | | | |
|---|---|---|---|
| Loading Density viable cells / µL | Target Density viable cells / µL | Mean Density Delivered viable cells / µL | Percent Viability |
| 444 | 333 | 336 +/-40 (N=6) | 95.2 +/- 3.2 (N=5) |
| 1776 | 1333 | 1433 +/- 187 (N=3) | 94.3 +/ -5.1 (N=3) |

**Table 12. Effect of reconstitution and delivery through the Synergetics cannula on RPE cell number and viability.**

| **Synergenics Cannnula** | | | |
|---|---|---|---|
| Loading Density viable cells / µL | Target Density viable cells / µL | Mean Density Delivered viable cells / µL | Percent Viability |
| 444 | 333 | 232 +/- 238 (N=3) | 89.0 +/- 9.6 (N=3) |
| 1776 | 1333 | 1296 (N=1) | 89 (N=1) |

Increasing the initial loading density by 25% above the targeted dose effectively compensated for the loss in cell density encountered during loading and extrusion through the MedOne cannula. At the lowest dose to be administered, the MedOne cannula delivered a mean cell density of 336 +/- 40 viable cells/ µL (N=6) for a targeted delivery of 333 viable cells/ µL. At the highest cell density to be delivered (1333 viable cells/ µL), the MedOne cannula delivered 1433 +/- 187 viable cells/ µL (N=3).

After the lowest dose delivery through the MedOne or Synergetics cannulae, cells were diluted in RPE growth medium, centrifuged, and seeded in gelatin-coated full-area 96 well plates at 40,000 cells per well. Non-cannula injected control cells taken from the same tubes as cells extruded through the cannula were processed and seeded in the same way. Twenty four hours post-seeding, all cells had attached and no floating cells indicative of cell death or impaired seeding efficiency were observed under any of the conditions tested.

Cells extruded through the MedOne cannula, the Synergetics cannula, and control cells formulated but not extruded through either cannula were centrifuged, resuspended in RPE growth medium and seeded in gelatin coated full area 96-well plates at 40,000 cells per well. Three days post-seeding, cells were trypsinized and counted. Table 13 below shows the mean cell number +/- SD. These results demonstrate that subsequent seeding and growth were not adversely impacted by extrusion through either of the cannulae. Control and MedOne cannula-injected cells were examined microscopically two-days post-seeding in culture and showed typical RPE morphology with actively dividing cells. No differences between control and cannula-injected cells were observed.

**Table 13. Cell seeding and growth after loading and extrusion through a cannula.**

| **Cell Number after 3 Days in Culture (cells were seeded at 40,000 cells per well)** | |
|---|---|
| Control Cells | 86117 +/ 3301 (N=3) |
| Extruded Synergetics | 98300 +/- 4554 (N=5) |
| Extruded MedOne | 82960 +/- 9368 (N=3) |

In summary, since RPE cells in cold BSS-Plus are more stable at concentrations greater than 1000 cells/µL, final product can be resuspended in the final product closure microcentrifuge tube in cold BSS-Plus at 2000 cells/µL, allowing the cannula to be loaded with doses up to 300,000 cells in a 150 µL volume. After processing in the GMP cleanroom, two microcentrifuge tubes at 2-8 degrees C can be delivered to the operating room: one vial containing the exact volume of RPE cells at 2000 viable cell/µL and one vial containing the exact volume of cold BSS-Plus to be added to the cells to bring the cell density to the density to be injected (i.e., the density that accounts for loss of viable cells during loading and extrusion through the cannula, e.g., a density 25% higher than the final targeted dose to account for the loss of viable cells with the MedOne cannula). When concentrations higher than 1,000 cells/µL or higher than 2,000 cells/µL are to be loaded into the cannula, the dilution step may be omitted and instead the cells may be delivered to the operating room in cold BSS-Plus at the desired concentration.

The formulated loading densities customized to the MedOne cannula and corresponding doses are shown in Table 14. Similar customization could readily be determined for the Synergetics cannula or another cannula or delivery system.

**Table 14. Loading cell densities used to deliver the target dosages of viable RPE, accounting for loss of viable cells during mixing, loading, and delivery with the MedOne cannula.**

| Loading Density viable cells / µL | Target Density viable cells / µL | Injection Volume | Dose Viable cells |
|---|---|---|---|
| 444 | 333 | 150 µL | 50,000 |
| 888 | 666 | 150 µL | 100,000 |
| 1333 | 999 | 150 µL | 150,000 |
| 1776 | 1333 | 150 µL | 200,000 |

### EXAMPLE 4

### RPE Differentiation from ES cells

This example describes the differentiation of RPE from hESC. The resulting RPE were used in the studies described in Example 1.

Embryoid Body Differentiation Medium (EB-DM) was composed of Knockout^{™} DMEM supplemented with Glutamax, nonessential amino acids, 2-mercaptothanol and Knockout^{™} Serum Replacement, and was used at the onset of embryoid body formation up to the time that pigmented patches are harvested and dissociated, i.e., through during embryoid body formation, outgrowth and subsequent pigmented patch formation. Each batch of EB-DM was made up of 250 mL Knockout^{™} DMEM, 3 mL Glutamax-I, 3 ml nonessential amino acids, 0.3 mL 2-mercaptothanol and 38 mL Knockout^{™} Serum Replacement.

RPE Growth/Maintenance Medium (RPE-GM/MM) was composed of one part EB-DM (as described in the preceding paragraph) and one part DMEM (high glucose), FBS and Glutamax. This medium was used after derivation of RPE cells from pigmented patches and during subsequent RPE growth and maintenance during passages 0 through passage 2 up to the point of final bulk product harvest. Each batch of RPT-GM/MM was made up of 100 mL EB-DM, 90 mL DMEM high glucose, 10 ML fetal bovine serum (FBS) (Hyclone), and 1 mL Glutamax-I.

RPE cells derived and cultured in these media expressed the molecular markers of RPE bestrophin, CRALBP, RPE65. PEDF, were capable of phagocytosis, and rescued visual function in RCS rats.

RPE lots generated using the above media have passed all in-process quality testing including: morphological evaluations, immunohistochemical staining and q-RT-PCR for the up-regulation of RPE genes and the down-regulation of hES cell gene expression. Yields and cell purity are comparable to the RPE cells previously prepared using MDBK-GM and MDBK-MM media (Sigma Aldrich), OptiPRO-SFM, or VP-SFM.

Lots of RPE were manufactured using EB-DM from the time of embryo body formation up to the point of harvesting pigmented patches (instead of MDBK-GM or OptiPRO-SFM). After harvesting and trypsinizing pigmented patches, passage 0, RPE cells were subsequently seeded in RPE-GM (EGM-2 medium) as defined above) and then switched to RPE Growth/Maintenance Media instead of MDBK-MM or VP-SFM. Alternatively, RPE may be seeded directly in RPE-GM/MM and allowed to grow and differentiate for the entire duration of the passage. After the appropriate level of differentiation is observed, passage 0 RPE cells were harvested and split two additional times in these media until final harvest and cryopreservation of bulk product at passage 2.

The data below show a summary of in-process testing for five sublots of RPE that were maintained in EB-DM from the time of embryo body formation up to the point of harvesting pigmented patches. At this time pigmented patches were harvested from different wells on different days, trypsinized and seeded as passage 0 RPE. Lots B1A, B2A and B2B were seeded in EGM-2 medium until confluent followed by switching to RPE Growth/Maintenance Media to promote differentiation for passages 0, 1 and 2. Lots B3B and B3A were treated the same way except for 1 or 2 passages, respectively, when they were maintained exclusively in RPE-GM/MM for the entire duration of the passage. All lots were thus maintained RPE-GM/MM upon reaching confluence until the appropriate level of differentiation was observed. After the termination of passage 2, RPE cells were cryopreserved as bulk product. Lots maintained in EGM-2 for the initial growth phase followed by switching to RPE-GM/MM or kept in RPE Growth/Maintenance Media for the entire duration of several passages were similar except for a slightly faster growth rate observed in the EGM-2 medium. All lots passed morphological evaluation at passages 0, 1, and 2, with passing specifications including typical epithelial, cobblestone morphology and medium pigmentation. RPE marker expression was detected by indirect immunofluorescence using the following primary antibodies (dilutions were between about 1:100 and 1:1000 and were empirically determined for each antibody batch): Bestrophin - mouse monoclonal; Novus Biologicals (# NB 300-164); PAX6 - Covance, rabbit polyclonal (PRB-278P); ZO-1 - Invitrogen; mouse monoclonal (339100); ZO-1 - Invitrogen; rabbit polyclonal (61-7300); ZO-1 - FITC- Invitrogen; mouse monoclonal (339111); MITF - mouse monoclonal, Abcam (ab3201).

Secondary antibodies were used at 1:500 dilution (or other dilution as indicated) in blocking solution and were as follows: Alexa Fluor 488 anti-mouse, Invitrogen # A 11001; Alexa Fluor 488 anti-rabbit, Invitrogen # A11008; Alexa Fluor 594 anti-mouse, Invitrogen # A11032; Alexa Fluor 594 anti-rabbit, Invitrogen # A 11012; goat anti-mouse Cy3-conjugated (Jackson Immunoresearch Cat. # 115-165-146), used at 1:200.

Immunostaining of RPE markers was performed to assess purity by combinations of: PAX6 and MITF; Bestrophin and PAX6; and ZO-1 alone. RPE maturation was assessed by determining the percentage of Bestrophin positive staining RPE. Immunostaining was performed at 4 points during the manufacture of RPE cells: (1) Prior to Harvest of Passage 1 and Seeding Passage 2 RPE were stained for Bestrophin, PAX6 and ZO-1; (2) Prior to Harvest of Passage 2 and Cryopreservation RPE were stained for Bestrophin, PAX6 and ZO-1; (3) RPE bulk product was thawed and formulated as described in Example 1 and re-suspended at 1,000 viable cells/µL in BSS-PLUS. Cells were then diluted in RPE-GM, centrifuged at 1000 RPM, re-suspended and seeded in gelatin coated four-well plates at 100,000-300,000 cells per well and incubated one to two days prior to staining for MITF and PAX6; (4) RPE bulk product was thawed and formulated as described in Example 1 and re-suspended at 1,000 viable cells/µL in BSS-PLUS. Cells were then diluted in RPE-GM, centrifuged at 1000 RPM, re-suspended and seeded in gelatin coated four-well plates at 50,000-200,000 cells per well and maintained until confluent prior to staining. At this time, cultures were switched to RPE-MM and maintained until medium pigmentation and cobblestone morphology are observed at which time cultures were stained for PAX6, Bestrophin and ZO-1. In brief, cells were rinsed 2-3 times with PBS without Ca2+, Mg2+ (Gibco # 14190), fixed with 2% paraformaldehyde for 10 minutes, rinsed with 2x PBS, incubated with 0.1 % NP-40 Substitute solution (Sigma # 74388) in PBS for 15 minutes, rinsed 2x with PBS, incubated with blocking solution (10% Normal Goat Serum (Jackson Immunoresearch # 005-000-121), 16% Paraformaldehyde (Electron Microscopy Sciences #15710) prepared at working concentration of 2% in PBS (Freshly made or frozen aliquots)) between 30 minutes and overnight. Cells were then incubated with primary antibodies (up to two antibodies per well using primary antibodies from different species) in blocking solution and incubated 1-2 hrs at room temperature or overnight at 4 degrees C, rinsed with PBS, washed three times in PBS-Tween solution (PBS without Ca2+, Mg2+ (Gibco # 14190) with 0.5% Tween-20 (Sigma # P7949)), with agitation (10-15 minutes each wash). Samples were then incubated with secondary antibodies, and washed as with the primary antibodies. After removal of the last wash, 1-2 drops of Vectashield with DAPI were added and the cells were examined and counted on an inverted fluorescence microscope. Photographs were taken of three to six random fields at 20x magnification in all channels, containing a minimum of 1000 nuclei. Photographs were merged and images were adjusted as needed to permit visualization of which cells were negative for Bestrophin and PAX6, or negative for PAX6 and MITF or negative for ZO-1. A cell was counted as positive for a given marker if the expected staining pattern was observed, e.g., PAX6 localized in the nuclei, Bestrophin localized in the plasma membrane in a polygonal pattern (showing localized Bestrophin staining in sharp lines at the cell's periphery), ZO-1 staining present in tight junctions outlining the cells in a polygonal pattern, and MITF staining detected confined to the nucleus. The percentage of cells positive for each marker or marker combination was determined by counting positive cells in the merged images and determining the total number of cells by counting nuclei from the unmerged DAPI-stained images.

**Table 15. RPE markers expressed by RPE cells differentiated from hES cells.**

| RPE markers were detected by indirect immunofluorescence straining. | | | | | | | |
|---|---|---|---|---|---|---|---|
| | Passage 1 markers | | | | Passage 2 markers | | |
| Lot | ZO-1 | Bestrophin | PAX6 and/or Bestrophin | | ZO-1 | Bestrophin | PAX6 and/or Bestrophin |
| B1A | 100% | 81% | 100% | | 100% | 81% | 100% |
| B2A | 100% | 90% | 100% | | 100% | 82% | 100% |
| B2B | 100% | 86% | 100% | | 100% | 89% | 100% |
| B3A | 100% | 98% | 100% | | 100% | 81% | 100% |
| B3B | 100% | 88% | 100% | | 100% | 99% | 100% |
| Specification | >/ =95% | >/=70% | >/=95% | | >/ =95% | >/=70% | >/=95% |

Additionally, mRNA expression was detected by q-RT-PCR, as described in Example 1. Results obtained from each lot are shown in Table 16 and demonstrate that RPE genes were up-regulated and ES cell genes were down-regulated as expected.

**Table 16. Up-regulation of RPE genes and down-regulation of ES cell genes in RPE cells differentiated from hES cells.**

| | Passage 2 (log up-regulation) | | | | | Passage 2 (down-regulation) | | |
|---|---|---|---|---|---|---|---|---|
| Lot | Bestrophin | PAX6 | MITF | RPE-65 | | NANOG | OCT-4 | SOX2 |
| B1A | 3.4 | 1.9 | 2.06 | 3.02 | | -2.78 | -3.29 | -2.68 |
| B2A | 4.2 | 1.79 | 2.5 | 1.6 | | -2.53 | -2.89 | -2.72 |
| B2B | 3.5 | 2.33 | 2.82 | 1.54 | | -2.08 | -3.24 | -1.86 |
| B3A | 3.39 | 2.34 | 2.77 | 1.55 | | -2.54 | -2.89 | -1.85 |
| B3B | 3.73 | 1.96 | 2.48 | 3.25 | | -2.76 | -3.33 | -4.01 |
| Specification | >1 | >1 | >1 | >1 | | <-1.95 | <-2.13 | <-0.63 |

RPE manufactured using the above-described media formulations (RPE-GM/MM and EB-DM), as well cryopreserved RPE cells previously manufactured using other media (MDBK-GM and MDBK-MM) were tested for their ability to phagocytose. In this study, the cryopreserved RPE were thawed and seeded in RPE Growth/Maintenance Media. RPE cells from current lots generated using EB-DM during embryoid body formation and pigmented patch formation and using RPE-GM/MM during RPE maturation were trypsinized and likewise seeded in RPE-GM/MM. Both cultures were grown to confluence and maintained until differentiated in RPE-GM/MM prior to testing for their ability to phagocytose fluorescent bioparticles (Invitrogen Cat. No. P35361), which fluoresce when internalized in the acidic environment of RPE cells' phagosomes. Cells were incubated with the fluorescent bioparticles at 37 degrees C to permit phagocytosis, or at 4 degrees C as a negative control. Shifts in fluorescence intensity were detected by FACS for the cells incubated at 37 degrees C (FIG. 12), indicating phagocytosis of the bioparticles. Statistical integration of the peaks yield the percentages of phagocytic positive cells for each lot and incubation temperature.

**Table 17. Phagocytosis by RPE cells produced using MDBK media (MDBK-GM and MDBK-MM) or EB-DM and RPE-GM/MM. Phagocytosis was detected by incubating cells with particles that become fluorescent in the acidic phagosome environment. Percentages of phagocytic positive cells are shown for cells incubated with at 37 degrees C or at 4 degrees C (negative control), as detected by FACS. Cells produced in EB-DM and RPE-GM/MM compared favorably to cells produced in MDBK media, further confirming suitability of these media formulations.**

| | MDBK media | EB-DM and RPE-GM/MM |
|---|---|---|
| 4 degrees C | 8% | 18% |
| 37 degrees C | 64% | 77% |

These results show phagocytosis in a high percentage of cells in both lots of RPE cells maintained in RPE-GM/MM, and further demonstrate the suitability of using RPE-GM/MM for RPE cell growth and maturation.

### Example 5

### Additional exemplary methods for RPE derivation

The methods in this example were used to produce RPE differentiated from additional hESC lines that were produced without embryo destruction, specifically, iPS cells (specifically, iPS cells produced using nonintegrating episomal vectors) and NED ("no embryo destruction") hES cells produced from biopsied blastomeres, wherein the embryo from which the blastomere was obtained remained viable and was subsequently cyropreserved. The NED cells were produced as described in Chung et al. (Cell Stem Cell. 2008 Feb 7;2(2): 113-7).

The hESC were propagated on Matrigel(TM) diluted per the manufacturer's instructions on mTESR-1 medium (Stem Cell Technologies, Inc.). RPE were produced from embryoid body ("EB") or multilayer hESC cultures as previously described (Klimanskaya et al., Cell Stem Cells 6:217-245 (2004), after suspension culture the EBs were plated for outgrowths prior to RPE harvest. However, it was observed that EB formation was less efficient from hESC cultured on Matrigel(TM), with cells exhibiting lower rates of successful aggregation and reduced viability. The following protocol modifications were utilized to improve EB formation efficiency:

The hESC were allowed to overgrow beyond the time when they would normally be passaged, so the colonies got "thicker," i.e., a little raised and /or multilayered. For EB formation, hESC were dissociated without being permitted to dissociate into single cell suspensions, using mechanical scraping, collagenase I, accutase, collagenase with or followed by accutase, EDTA-based dissociation buffer. These methods allowed hESC colonies to be lifted without dissociating into single cells. Trypsin (which tends to readily produce single cell suspensions under ordinary use conditions) was not utilized.

The dissociated hESC were then cultured on ultra-low attachment plates to allow EB formation. Optionally other methods, such as hanging drop, may be used for EB formation. Typically hESC from 1-3 wells of 6-well dish were cultured in 1-2 wells of low adherence plates in 2-7 ml of culture media. The cells were cultured in EB medium (knockout high glucose DMEM, 1% non-essential amino acids solution, 2 mM GlutaMAX I, 0.1 mM beta-mercaptoethanol, and 13% of Serum Replacement (SR, Invitrogen)). During the first 2-3 days of culture in EB medium while the EB are forming, the EB medium was supplemented with 10 microMolar Stemgent's Stemolecule Y-27632, a rho-associated protein kinase (ROCK) inhibitor (see Watanabe et al., Nat Biotechnol. 2007 Jun;25(6):681-6, which is hereby incorporated by reference in its entirety). Use of the ROCK inhibitor improved cell viability, particularly for hES cells obtained using EDTA or enzymatic dissociation. Use of the ROCK inhibitor was optional for mechanically scraped hESC, which survived well even without it.

Between 7-12 days after EB formation, the EBs were plated on gelatin coated plates for outgrowth. RPE were readily identified by their epithelial morphology (cobblestone appearance) and pigmentation.

RPE were also produced from multilayer cultures of hESC grown on Matrigel(TM) essentially as previously described (Klimanskaya et al., 2004, *supra*), except that the cells were cultured on Matrigel(TM) instead of feeder cells. In brief, hESC were allowed to overgrow on Matrigel(TM) in mTESR-1 media until the hESC colonies became multilayered (approximately 10-14 days of culture), at which time the culture media was replaced with EB media (as described above). ROCK inhibitor was optionally included in the culture media but was not necessary for efficient RPE formation and recovery. RPE were readily identified by their epithelial morphology (cobblestone appearance) and pigmentation. The medium was changed every 1-2 days until pigmented RPE cells were observed (typically within 4-5 weeks).

Resulting EB or multilayer cultures exhibited a "freckled" appearance containing darker regions visible to the naked eye. Microscopic examination confirmed that these darker regions were made up of RPE cells identifiable by their characteristic pigmentation and cobblestone, epithelial morphology. Resulting RPE cell culture are shown in FIG. 19. After differentiation from hESC, the RPE cells were isolated by either mechanical or enzymatic dissociation.

### EXAMPLE 6

### RPE Transplantation Methods

The following methods were used for cell transplantation into dry age-related macular degeneration (AMD) and Stargardt's Macular Dystrophy (SMD) patients.

No corticosteroids were administered to the patient immediately prior to surgery. Surgery was performed under general or local anesthesia with or without waking sedation at the surgeon's discretion.

Cells for transplantation were provided as a frozen suspension stored in the vapor phase of a liquid nitrogen storage system (approximately -140 °C). To formulate the cells for administration, the vials were removed from the liquid nitrogen freezer then placed in a water bath at 37 °C and constantly agitated for 1-2 minutes until thawed. The vial was then sprayed with 70% isopropanol and dried. The contents of each vial (1 mL of cryopreservation medium containing 1 million cells at the time of freezing) was transferred to a 50 mL conical tube and rinsed with 40 mL of serum-free DMEM. The cells were centrifuged and each pellet was resuspended in 40 mL BSS-PLUS. The cell suspension was centrifuged again and the pellet(s) were pooled together if more than one cryovial has been thawed. The volume was brought up to a final volume of 10 mL in BSS PLUS and centrifuged a third time. The supernatant was aspirated completely and the cells were brought to a final volume of approximately 150 µL BSS PLUS for each 1mL of cells thawed (lower volumes can be used if a more concentrated suspension is desired). Samples were removed and a viable cell count was performed. The total viable cell number was determined and the appropriate volume of BSS-PLUS was added to obtain the target viable cell concentration such as 2,000 viable cells per µL. The appropriate volume of formulated product was transferred to a 0.5 mL sterile microcentrifuge tube and samples were removed for archiving, viability determinations, Gram staining and sterility testing. Paired 0.5 mL sterile microcentrifuge tube containing the exact volume of BSS-Plus were also prepared and labeled. Paired vials were permitted to be stored at 2-8C for not more than 4 hours awaiting final mixing and transplantation in the operating room.

A standard 3 port pars plana vitrectomy was performed on the patient. A small retinotomy was made and infusion of BSS Plus into the subretinal space was then performed using the fluid injection system through the vitrectomy machine until a small neurosensory retinal detachment was created. The surgeon ensured that the bleb was created in a temporal foveal position. The bleb optionally can extend within the arcade blood vessels but did not detach the central macula/fovea. If the bleb was observed to be extending towards the central macula, the surgeon had the option to stop and create another retinotomy, observing the same rules as to its location. The BSS Plus injected subretinally was then removed.

A pre-loaded cannula was then introduced and the cells were infused into the space created over approximately one minute in a volume of 150 µL. Monitoring by direct viewing was undertaken to ensure correct cannula positioning. The exact position of the bleb was recorded through the operating microscope by photography or (preferably) video in order that postoperative findings could be correlated exactly with the position of the bleb.

A suspension containing the desired number of hESC-derived RPE cells (e.g., 50,000, 100,000, 150,000, or 200,000) in 150 uL of BSS Plus was implanted. The cells were be infused over approximately one minute. The cannula was held in position for an additional minute to avoid reflux. At the surgeon's discretion, for example if the retinotomy enlarges, an air-fluid exchange was optionally performed. Standard procedures were then used to close the incisions. The patient was then recovered from anesthesia, but kept in a supine position for 6 hours.

No corticosteroids were permitted to be administered for 48 hours following the procedure. Topical or systemic non-steroidal anti-inflammatory agents were permitted to be used to manage post-operative discomfort, if needed.

### EXAMPLE 7

### Stability of Cryopreserved RPE Cell Preparations

This example demonstrates that cryopreserved RPE cells passed release criteria and remained suitable for use when tested at time points 6 and 12 months after freezing. Based thereon, it is shown that cryopreserved RPE retain their function and product attributes for 12 month post-cryopreservation. It is anticipated that cryopreserved RPE cells will remain suitable for transplantation for years after freezing (e.g., at least 2, 3, 4, 5, 6, 7, 8, 9, 10, or more years).

Cryopreserved RPE samples were produced as described in Example 4, above, and frozen in liquid nitrogen in a cryopreservation medium (90% FBS and 10% DMSO) and stored in the vapor phase of a liquid nitrogen storage system (approximately -140 °C). After 6 or 12 months of storage, the cryopreserved cells were thawed and washed as described in Example 6 (in brief, thawed in a water bath at 37 degrees C, the outside of the container was washed with 70% ethanol, and the cells were washed to remove cryopreservation medium). After thawing, the cells were subjected to tests to confirm product stability. Each of the release criteria was passed, as shown in Table 18 below.

**Table 18. Stability of Cryopreserved RPE. Cells cryopreserved for 6 or 12 months**

| **Test Description** | **Method** | **Specification** | **Result (6 mo.)** | **Result (12 mo.)** |
|---|---|---|---|---|
| Karyotype | G-banding | Normal 46 XX (post-thaw, formulation, and culturing) | Pass | Pass |
| FISH | FISH | Normal FISH Signal (12 and 17) | Pass | Pass |
| Potency | Phagocytosis Assay using fluorescent particles | Internalization of fluorescent particles by RPE cells detected by FACS analysis with a shift in fluorescent peak for cells cultured with particles at 37 degrees C (post-thaw, formulation, and culturing) | Pass | Pass |
| Cell Count | Trypan Blue Exclusion | Report Recovery (post-thaw and formulation) | 32.6% | 29% |
| Viability | Trypan Blue Exclusion | At least 70% (post-thaw and formulation) | 95% | 97% |
| Sterility | Immersion Method (USP/21 CFR 610.12) | Negative (thawed vials) | Negative | Negative |
| Morphology | Morphological Evaluation | Acceptable cobblestone morphology, cubiodal cells (post-thaw, formulation and culturing) | Pass | Pass |
| Purity | Immunostaining of RPE markers | At least 95% positive for Bestrophin and/or | 97% | 96% |
| | | Pax6 | Bestrophin+ 100% Pax6+ 100% ZO-1+ | Bestrophin+ 100% Pax6+ 100% ZO-1+ |
| | | At least 95% positive for ZO-1 (post-thaw, formulation and culturing) | | |

### EXAMPLE 8

### Stability of Formulated RPE Cells

This example demonstrates that RPE cells remained suitable for use for at least 4 hours after thawing and preparation for administration when maintained between 2-6 degrees C.

Cryopreserved cells were thawed and formulated as described in Example 6, above and stored at 2-8°C in the final product container (0.5 mL sterile microcentrifuge tube with gasket). Table 19 shows the mean percent viability ± SD as assessed by trypan blue exclusion for these two lots tested at the time of formulation and after four hours in cold storage (3 final formulations per lot).

**Table 19. Initial and 4-Hour Viability of RPE Cells in Clinical Product**

| | **0-Hour Viability (%)** | **4-Hour Viability (%)** |
|---|---|---|
| **Lot A (N=3)** | 82.7 ± 6.7 | 82.3 ± 1.5 |
| **Lot B (N=3)** | 84.3 ± 1.5 | 85.3 ± 4.1 |

These data showed that the formulated RPE cells maintain cellular viability out to 4 hours post-preparation.

In additional experiments the RPE cell final product was formulated at 2,000 viable cells/µL and stored in the cold for various times prior to extrusion through the MedOne REF 3233 POLYTIP^{R} Cannula 23/38. In this study (data shown in Table 20) an RPE cell lot (which had passed bulk-product release testing for clinical use) was thawed and processed as above. Cells were resuspended and stored at a density of 2,000 viable cells/µL in BSS-Plus and kept thereafter on ice. A cell density of greater than 1,000 viable cells/µL was selected to promote cell survival during cold-storage in BSS-Plus. At this time twenty one, 89µL aliquots of cells containing 177,600 total viable cells were dispensed into the final product closure microcentrifuge tubes. Cell aliquots were stored on ice until the final dilution was performed at the time of syringe loading and extrusion through the cannula. For low dose deliveries, 311µL of cold BSS-Plus was dispensed into a tube containing cells to bring the final volume to 400µL @ 444 cells/µL. This density is 25% higher than the intended delivery density of 333 cells/µL to compensate for anticipated losses that occur when mixing with the fill needle, syringe loading, and delivery through the MedOne cannula.

For high dose deliveries, two 89µL of cells were pooled into one tube (356,000) and 22µL of cold BSS-Plus was dispensed into the tube containing the cells to bring the final volume to 400µL @ 1,776 cells/µL. This density is 25% higher than the intended delivery density of 1,333 cells/µL to compensate for anticipated losses that occur when mixing with the fill needle, syringe loading, and delivery through the MedOne cannula.

Microcentrifuge tubes containing diluted cell were capped and gently tapped with one finger to promote mixing. The blunt fill needle (void volume of 90µL) was attached to the 1mL BD syringe and cells were gently triturated 1-2 time in the blunt fill needle taking care to minimize contact with the syringe. The syringe was filled with approximately 200µL of cells. The blunt needle was removed and the MedOne 38g injection cannula was attached. (Approximately 150µL of cells were dispensed into a microcentrifuge tube. Each dispensed aliquot was assessed for cell density and viability by trypan blue exclusion. The time post-formulation are the minutes elapsed from resuspending the cells in cold BSS-Plus at 2,000 viable cells/µL. These data are shown in Table 20 for cells delivered at the indicated concentrations.

**Table 20. Viability of cannula-delivered RPE cells stored after formulation**

| **Low Dose (Formulated to 444 viable cells/µL: Target 333 viable cells/µL)** | | |
|---|---|---|
| **Minutes Post Formulation** | **Cell Density Delivered (Viable Cells/µL)** | **% Viability** |
| 22 | 331 | 99 |
| 85 | 346 | 97 |
| 91 | 374 | 91 |
| 187 | 363 | ND |
| 193 | 339 | 93 |
| 207 | 260 | 96 |
| | | |

| **Mean 336 +/- 40 (N=6)** | | |
|---|---|---|
| **Intermediate Dose (Formulated to 1561 viable cells/µL: Target 1172 viable cells/µL)** | | |
| **Minutes Post Formulation** | **Cell Density Delivered (Viable Cells/µL)** | **% Viability** |
| 248 | 1178 | 90 |
| | | |

| **High Dose (Formulated to 1776 viable cells/µL: Target 1333 viable cells/µL)** | | |
|---|---|---|
| **Minutes Post Formulation** | **Cell Density Delivered (Viable Cells/µL)** | **% Viability** |
| 48 | 1308 | 100 |
| 176 | 1648 | 93 |
| 293 | 1343 | 90 |
| | | |
| **Mean 1433 +/- 187 (N=3)** | | |

The data show that the viable cell number of final product RPE cells extruded through the injection cannula does not decrease when stored in the cold over the times tested. The viable cell densities observed at times exceeding 240 minutes (4 hours) post-formulation support an expiration time of at least 4 hours. In this study, RPE cells in BSS-Plus were stored in final product closures on ice. The temperature of BSS-Plus in microcentrifuge tubes store on ice has been subsequently measured using a calibrated probe and found to be 3°C.

Further experiments tested the viability of formulated RPE cells for up to six hours. Viability was assessed at the time of formulation (0 hours) and after 4 and 6 hours in cold storage (2-8°C). The RPE Lot used in this study was manufactured and cryopreserved using procedures, processes, and materials as described for GMP manufacture. Cryopreserved vials of RPE cells were thawed and formulated following the procedures described in Example 6, above. Cells were assessed for viable cell number at the time of formulation (0 Hours) and after 4 and 6 hours in cold-storage (2-8°C). In addition, 0, 4 and 6 hour cells were seeded and cultured for subsequent purity and potency assessments. For each time point seeded (0, 4 and 6 hours), purity was assessed by MITF and PAX6 immunostaining and for phagocytosis of fluorescent particles, by FACS analysis.

The viable cell density was determined by counting trypan blue excluding cells in a hemacytometer. The data are the mean +/- SD of counts performed on 4 hemocytometer chambers. Results are shown in Table 21 below.

**Table 21. Viability of cells stored between 2-8 degrees C after formulation**

| **Experiment # 1** | | |
|---|---|---|
| | **Viable Cells/µL** | **Viability** |
| 0 hour | 2590 +/- 332 | 86% |
| 4 hours | 2850 +/- 148 | 79% |
| 6 hours | 2875 +/- 145 | 89% |

| **Experiment # 2** | | |
|---|---|---|
| | | |

| | **Viable Cells/µL** | **Viability** |
|---|---|---|
| 0 hour | 1700 +/- 78 | 88% |
| 4 hours | 1680 +/- 123 | 82% |
| 6 hours | 1550 +/- 248 | 85% |

Temperature readings for the GMP storage refrigerator where the formulated cells were stored confirmed that the temperature remained at 6 degrees C throughout the experiments.

The 0 hour starting viable cell densities of 2,590/µL and 1,700/µL bracket the 2,000 viable cells/µL targeted for some clinical formulations. No loss in viable cell number over the range of starting cell densities tested was observed out to six hours in cold storage.

### Example 9

This example provides initial treatment results for two additional Startardt's disease patients. The two patients were each treated with 50,000 RPE cells derived from an hESC source (as described in Examples 1) using the RPE Transplantation Methods described in Example 6 above. Fundus photographs including the retina, optic disc, macula, and posterior pole for two the Stargardt's patients indicate the site of injection and the area of the bleb created upon injection of the solution containing the RPE cells (FIG. 15).

Further fundus photographs show the establishment of areas within the injection bleb which have increasing patches of pigmented RPE cells for two SMD patients (FIGS. 16 and 17). These results suggest engraftment and resurfacing of areas of the retina with a new RPE layer.

Visual acuity was also measured in the treated eye of the patient shown in FIG. 16. The vertical axis indicates Early Treatment Diabetic Retinopathy Study (ETDRS score and the horizontal axis shows the number of days postsurgery.

These results indicate stable engraftment of RPE cells persisting for at least 3 months after treatment. Visual acuity in the treated eye had returned to baseline levels at 14 days post-treatment and remained above baseline until 84 days at the final time point shown.

### EXAMPLE 10

### One year patient evaluation

The AMD patient and the SMD patient were evaluated over a period of one year after the RPE treatment described in Example 1 above.

Fundus photography of the SMD patient's eye demonstrated the presence of pigmented cells in the treated eye at one year post-treatment (FIG. 20B). In contrast, pigmented cells were not detectable at baseline prior to treatment (FIG. 20A). These results indicate long-term engraftment of RPE which were sustained for at least one year after treatment.

For the AMD patient, the Peripheral? ETDRS/BVCA Score is illustrated graphically in FIG. 21. From an initial baseline value of 21, the patient's Peripheral ERTDS-BVCA Score decreased to zero on days 1 and 3 post surgery but returned to at least baseline levels on the seventh day after surgery and thereafter remained above baseline. At one year post-treatment the patient's Peripheral ERTDS-BVCA Score was 34.

For the SMD patient, one year after treatment the Central ETDRS/BVCA Score was 15. The peripheral score is illustrated graphically in FIG. 22. From an initial baseline value of 0, two weeks after surgery the patient's Peripheral ERTDS-BVCA Score increased to 1 and thereafter continued to increase to a value of 15 at one year post-treatment.

These results indicate improvement in visual acuity in both AMD and SMD patients resulting from the administration of the RPE cells that were sustained for at least one year post-treatment.

### REFERENCES CITED

1. Thomson JA, Itskovitz-Eldor J, Shapiro SS, et al. Embryonic stem cell lines derived from human blastocysts. Science 2008; 282: 1145-1147.
2. Fink DW, Bauer SR. Stem cell-based therapies: Food and Drug Administration product and pre-clinical regulatory considerations. In: Lanza R, Hogan B, Melton D, Pedersen R, Thomas, ED, Thomson J, Wilmut I, eds. Essentials of Stem Cell Biology. San Diego: Academic Press/Elsevier, 2009: 619-630.
3. Lanza RP, Chung HY Yoo JJ, et al. Generation of histocompatible tissues using nuclear transplantation. Nature Biotechnology 2002; 20, 689 - 696.
4. Takahashi K, Tanabe K, Ohnuki M, et al. Induction of pluripotent stem cells from adult human fibroblasts by defined factors. Cell 2007; 131, 861-872.
5. Kim D, Kim, CH, Moon JI, et al. Generation of human induced pluripotent stem cells by direct delivery of reprogramming proteins. Cell Stem Cells 2009; 4, 472-476.
6. Kaplan HJ, Tezel TH, Berger AS, Del Priore LV. Retinal transplantation. In: Streilein JW, ed. Immune Response and the Eye. Chem Immunol. Basel: Karger, 1999: 207-219.
7. Lund RD, Wang S, Klimanskaya I, et al. Human embryonic stem cell-derived cells rescue visual function in dystrophic rats. Cloning and Stem Cells 2006; 8,189-199.
8. Lu B, Malcuit C, Wang S, et al. Long-term safety and function of RPE from human embryonic stem cells in preclinical models of macular degeneration. Stem Cells 2009; 21, 2125-2135.
9. Sparrow JR, Hicks D, Hamel CP. The retinal pigment epithelium in health and disease. Curr Mol Med 2010; 10, 802-823.
10. Strauss O. The retinal pigment epithelium in visual function. Physiol Rev 2005; 85, 845-881.
11. Binder S., et al. Outcome of transplantation of autologous retinal pigment epithelium in age-related macular degeneration: a prospective trial. Invest Ophthalmol Vis Sci 2004; 45, 4151-4160.
12. Algvere PV, Berglin L, Gouras P, Sheng Y. Transplantation of fetal retinal pigment epithelium in age-related macular degeneration with subfoveal neovascularization. Graefes Arch Clin Exp Ophthalmol 1994; 232, 707-716.
13. Kaplan HJ, Tezel TH, Berger AS, Del Priore LV. Retinal transplantation. Chem Immunol 1999;73, 207-219.
14. Binder S, Stolba U, Krebs I, et al. Transplantation of autologous retinal pigment epithelium in eyes with foveal neovascularization resulting from age-related macular degeneration: a pilot study. Am J Ophthalmol 2002; 133, 215-225.
15. MacLaren RE, Bird AC, Sathia PJ, Aylward GW. Long-term results of submacular surgery combined with macular translocation of the retinal pigment epithelium in neovascular age-related macular degeneration. Ophthalmology 2005; 112, 2081-2087.
16. Lappas A, Weinberger AW, Foerster AM, Kube T, Rezai KA, Kirchhof B. Iris pigment epithelial cell translocation in exudative age-related macular degeneration. A pilot study in patients. Graefes Arch Clin Exp Ophthalmol 2000; 238, 631-641.
17. Aisenbrey S, Lafaut BA, Szurman P, et al. Iris pigment epithelial translocation in the treatment of exudative macular degeneration: a 3-year follow-up. Arch Ophthalmol 2006;124, 183-188.
18. Thumann G, Aisenbrey S, Schraermeyer U, et al. Transplantation of autologous iris pigment epithelium after removal of choroidal neovascular membranes. Arch Ophthalmol 2000;118, 1350-1355.
19. Berger AS, Tezel TH, Del Priore LV, Kaplan HJ. Photoreceptor transplantation in retinitis pigmentosa: short-term follow-up. Ophthalmology 2003;110, 383-391.
20. Drukker M, Katchman H, Katz G, et al. Human embryonic stem cells and their differentiated derivatives are less susceptible to immune rejection than adult cells Stem Cells 2006; 24, 221-229.
21. Okamura RM, Lebkowski J, Au M, Priest CA, Denham J, Majumdar AS. Immunological properties of human embryonic stem cell-derived oligodendrocyte progenitor cells. J Neuroimmunol 2007; 192, 134-144.
22. Klimanskaya I, Chung Y, Becker S, et al. Human embryonic stem cell lines derived from single blastomeres. Nature 2006; 444, 481-485.
23. Tezel TH, Del Priore LV. Serum-free media for culturing and serially-passaging of adult human retinal pigment epithelium. Exp Eye Res 1998; 66, 807-815.
24. Lu F, Zhou X, Hu DN, et al. Expression of melanin-related genes in cultured adult retinal pigment epithelium and uveal melanoma cells. Mol Vis 2007; 13, 2066-2072.
25. Tezel TH, Del Priore LV, Berger AS, et al. Adult retinal pigment epithelial transplantation in exudative age-related macular degeneration. Am J. Ophthalmol 2007; 142, 584-595.
26. Song MK, Lui GM. Propagation of fetal human RPE cells: preservation of original culture morphology after serial passage. J Cell Physiol 1990; 143, 196-203.
27. Gamm DM, Wright LS, Capowski EE, et al. Regulation of prenatal human retinal neurosphere growth and cell fate potential by retinal pigment epithelium and Mash1. Stem Cells 2008; 26, 3182-3193.
28. Maminishkis A, Chen S, Jalickee S, et al. Confluent monolayers of cultured human fetal retinal pigment epithelium exhibit morphology and physiology of native tissue. Invest Ophthalmol Vis Sci. 2006; 47, 3612-3624.

Those skilled in the art will recognize, or be able to ascertain using no more than routine experimentation, many equivalents to the specific embodiments of the invention described herein. Such equivalents are intended to be encompassed by the following claims.

## Claims

1. A pharmaceutical composition comprising:
a plurality of retinal pigment epithelial (RPE) cells; and
a pharmaceutically acceptable carrier;
wherein the average melanin content of said plurality of RPE cells is between 1-5 pg/cell; and wherein the pharmaceutical composition comprises between 1,000 and 1×10⁹ viable RPE cells.

2. The pharmaceutical composition of claim 1, wherein at least 50%, at least 60%, at least 70%, or at least 80% of the RPE cells in said pharmaceutical composition are bestrophin+.

3. The pharmaceutical composition of claim 1 or claim 2, wherein at least 80%, at least 85%, at least 90%, at least 95%, or at least 99% of the RPE cells in said pharmaceutical composition are PAX6+ and/or MITF+.

4. The pharmaceutical composition of any one of the preceding claims, wherein no more than about one cell per million cells and optionally no more than two cells per nine million cells in said pharmaceutical composition are positive for both OCT-4 and alkaline phosphatase (AP) expression.

5. The pharmaceutical composition according to any one of the preceding claims, wherein said RPE cells are human.

6. The pharmaceutical composition according to any one of the preceding claims, wherein said RPE cells are genetically engineered, for example to produce one or more factors that inhibit angiogenesis.

7. The pharmaceutical composition according to any one of the preceding claims, wherein said RPE cells are produced *in vitro* from a pluripotent stem cell.

8. A kit comprising a pharmaceutical composition of any one of the preceding claims and a separate container comprising a pharmaceutically acceptable diluent in a volume sufficient to dilute said plurality of RPE cells to a desired target concentration.

9. A cryopreserved composition comprising:
a plurality of cryopreserved retinal pigment epithelial (RPE) cells contained in a cryopreservation medium;
wherein the average melanin content of said plurality of cryopreserved RPE cells is between 1-5 pg/cell and the RPE cells having an average maturity level at the time of freezing such that the RPE cells recovered subsequent to thawing having a seeding efficiency of at least about 60%, and wherein said cryopreserved composition comprises between 5,000 and 1 × 10⁸ viable RPE cells at the time of freezing.

10. A method of producing retinal pigment epithelial (RPE) cells for use in a pharmaceutical preparation, comprising:
(a) culturing RPE cells under adherent conditions to form a substantially monolayer culture of pigmented RPE cells having a cobblestone morphology;
(b) passaging the RPE cells at least once at a time prior to the RPE cells reaching an average melanin content greater than 8 pg/cell; and
(c) after the one or more passages, harvesting RPE cells for cryopreservation or pharmaceutical formulation, wherein at the time of harvesting the RPE cells have an average melanin content between 1-5 pg/cell.

11. The method of claim 10, wherein said RPE cells are produced from pluripotent stem cells.

12. A pharmaceutical preparation comprising RPE cells suitable for treatment of retinal degradation, wherein said RPE cells (a) are differentiated from pluripotent stem cells, (b) contain an average melanin content of between 1-5 pg/cell, and (c) have at least one of the following properties:
attach to the Bruch's membrane after transplantation,
absorb stray light after transplantation,
have greater average telomere length than RPE cells derived from donated human tissue,
have greater replicative lifespan in culture than RPE cells derived from donated human tissue,
have lower A2E content than RPE cells derived from donated human tissue,
have lower lipofuscin content than RPE cells derived from donated human tissue,
exhibit less accumulated ultraviolet damage than RPE cells derived from donated human tissue, or
contain a greater number of phagosomes than RPE cells derived from donated human tissue.

13. The pharmaceutical composition according to any one of claims 1-7 or preparation according to claim 12, for use in treating retinal degeneration, including macular degeneration such as age-related macular degeneration, Stargardt's Disease, Angioid streaks and Myopic Macular Degeneration.

14. The pharmaceutical composition according to any one of claims 1-7 or preparation according to claim 12, wherein the composition or preparation is formulated for transplantation with a matrix, substrate, scaffold, or graft.

15. The pharmaceutical composition according to any one of claims 1-7 or preparation according to claim 12, wherein said RPE cells exhibit one or more of the following characteristics:
a replicative lifespan that is greater than the replicative lifespan of RPE cells obtained from adult eyes or fetal tissue;
an average telomere length that is at least 30 percent of the telomere length of a hESC and/or human iPS cell (or the average of a population of hESC and/or human iPS cells), or at least 40, 50, 60, 70 80 or 90 percent of the telomere length of an hESC and/or human iPS cell;
a mean terminal restriction fragment length (TRF) that is longer than 4 kb, or longer than 5, 6, 7, 8, 9, 10, 11, 12 or even 13kb, or 10kb or longer;
an average lipofuscin content that is less than 50 percent of the average lipofuscin content of the equivalent number of RPE cells isolated from adult eyes, or less than 40, 30, 20 or 10 percent of the average lipofuscin content of the equivalent number of RPE cells isolated from adult eyes;
an average N-retinylidene-N-retinylethanolamine (A2E) content that is less than 50 percent of the average A2E content of the equivalent number of RPE cells isolated adult eyes, or less than 40, 30, 20 or 10 percent of the average A2E content of the equivalent number of RPE cells isolated from adult eyes;
an average N-retinylidene-N-retinylethanolamine (A2E) content that is less than 50 ng per 10⁵ (100,000) cells;
a rate of phagocytosis of photoreceptor outer segments (POS) that is at least 50 percent greater than the rate of phagocytosis of POS for an equivalent number of RPE cells isolated adult eyes, or at least than 75, 100, 150 or 200 percent greater than the rate of phagocytosis of POS for an equivalent number of RPE cells isolated adult eyes;
rate of phagocytosis of photoreceptor outer segments (POS) that is at least 20 percent of the total concentration of POS after 24 hours, or at least than 25, 30, 25, 40 or 50 percent of the total concentration of POS after 24 hours;
a decreased level of accumulated oxidative stress and/or DNA damage compared to RPE cells isolated from an adult host;
an average proteasome activity that is at least 50 percent greater than the average proteosome activity of the equivalent number of RPE cells isolated from adult eyes, or at least 60, 70, 80, 90 or 100 percent greater than the average proteosome activity of the equivalent number of RPE cells isolated from adult eyes;
an average accumulation of ubiquitin conjugates that is less than 50 percent of the average accumulation of ubiquitin conjugates for an equivalent number of RPE cells isolated from adult eyes, or less than 40, 30, 20 or even 10 percent of the average accumulation of ubiquitin conjugates of the equivalent number of RPE cells isolated from adult eyes.

16. The composition of claim 1, wherein the composition further comprises a ROCK inhibitor, such as Y-27632.

## Patentansprüche

1. Pharmazeutische Zusammensetzung, umfassend:
eine Vielzahl von retinalen Pigmentepithelzellen (RPE); und einen pharmazeutisch annehmbaren Träger;
wobei der durchschnittliche Melaningehalt der Vielzahl von RPE-Zellen zwischen 1 - 5 pg/Zelle ist;
und wobei die pharmazeutische Zusammensetzung zwischen 1.000 und 1 × 10⁹ lebensfähige RPE-Zellen umfasst.

2. Pharmazeutische Zusammensetzung nach Anspruch 1, wobei wenigstens 50 %, wenigstens 60 %, wenigstens 70 % oder wenigstens 80 % der RPE-Zellen in der pharmazeutischen Zusammensetzung Bestrophin+ sind.

3. Pharmazeutische Zusammensetzung nach Anspruch 1 oder Anspruch 2, wobei wenigstens 80 %, wenigstens 85 %, wenigstens 90 %, wenigstens 95 % oder wenigstens 99 % der RPE-Zellen in der pharmazeutischen Zusammensetzung PAX6+ und/oder MITF+ sind.

4. Pharmazeutische Zusammensetzung nach einem der vorhergehenden Ansprüche, wobei nicht mehr als etwa eine Zelle pro Million Zellen und gegebenenfalls nicht mehr als zwei Zellen pro neun Millionen Zellen in der pharmazeutischen Zusammensetzung positiv für die Expression von sowohl OCT-4 und alkalischer Phosphatase (AP) sind.

5. Pharmazeutische Zusammensetzung nach einem der vorhergehenden Ansprüche, wobei die RPE-Zellen human sind.

6. Pharmazeutische Zusammensetzung nach einem der vorhergehenden Ansprüche, wobei die RPE-Zellen genetisch verändert sind, zum Beispiel um einen oder mehrere Faktoren herzustellen, die Angiogenese hemmen.

7. Pharmazeutische Zusammensetzung nach einem der vorhergehenden Ansprüche, wobei die RPE-Zellen in vitro aus einer pluripotenten Stammzelle hergestellt sind.

8. Kit, umfassend eine pharmazeutische Zusammensetzung nach einem der vorhergehenden Ansprüche und einen separaten Behälter, umfassend ein pharmazeutisch annehmbares Verdünnungsmittel in einem Volumen, das ausreicht, um die Vielzahl von RPE-Zellen auf eine gewünschte Zielkonzentration zu verdünnen.

9. Kryokonservierte Zusammensetzung, umfassend:
eine Vielzahl von kryokonservierten retinalen Pigmentepithelzellen (RPE), die in einem Kryokonservierungsmedium enthalten sind;
wobei der durchschnittliche Melaningehalt der Vielzahl von kryokonservierten RPE-Zellen zwischen 1 - 5 pg/Zelle beträgt und die RPE-Zellen zum Zeitpunkt des Gefrierens einen durchschnittlichen Reifegrad aufweisen, so dass die RPE-Zellen nach dem Auftauen mit einer Aussaateffizienz von wenigstens etwa 60 % wiedergewonnen werden, und wobei die kryokonservierte Zusammensetzung zwischen 5.000 und 1 × 10⁸ lebensfähige RPE-Zellen zum Zeitpunkt des Gefrierens umfasst.

10. Verfahren zur Herstellung von retinalen Pigmentepithelzellen (RPE) zur Verwendung in einer pharmazeutischen Zubereitung, umfassend:
(a) Kultivieren von RPE-Zellen unter adhärenten Bedingungen, um eine im Wesentlichen monoschichtige Forma-Kultur von pigmentierten RPE-Zellen mit einer Kopfsteinpflastermorphologie durchzuführen;
(b) Passagieren der RPE-Zellen wenigstens einmal pro Zeiteinheit, bevor die RPE-Zellen einen durchschnittlichen Melaningehalt von mehr als 8 pg/Zelle erreichen; und
(c) nach dem einmaligen oder mehrmaligen Passagieren Ernten von RPE-Zellen zur Kryokonservierung oder pharmazeutischen Formulierung, wobei die RPE-Zellen zum Zeitpunkt des Erntens einen durchschnittlichen Melaningehalt von zwischen 1 - 5 pg/Zelle aufweisen.

11. Verfahren nach Anspruch 10, wobei die RPE-Zellen aus pluripotenten Stammzellen hergestellt werden.

12. Pharmazeutische Zubereitung, umfassend RPE-Zellen, die zur Behandlung des retinalen Abbaus geeignet sind, wobei die RPE-Zellen (a) von pluripotenten Stammzellen differenziert werden, (b) einen durchschnittlichen Melaningehalt von zwischen 1 - 5 pg/Zelle enthalten und (c) wenigstens eine der folgenden Eigenschaften aufweisen:
Anhängen an die Bruch'sche Membran nach Transplantation,
Absorbieren von Streulicht nach Transplantation,
Aufweisen von größerer durchschnittlicher Telomerlänge als RPE-Zellen, die aus gespendetem humanen Gewebe gewonnen werden,
Aufweisen einer größeren Replikationslebensdauer in Kultur als RPE-Zellen, die aus gespendetem humanen Gewebe gewonnen werden,
Aufweisen eines niedrigeren A2E-Gehalts als RPE-Zellen, die aus gespendetem humanen Gewebe gewonnen werden,
Aufweisen eines geringeren Lipofuszin-Gehalts als RPE-Zellen, die aus gespendetem humanen Gewebe gewonnen werden,
Aufweisen von weniger akkumulierten UV-Schäden als RPE-Zellen, die aus gespendetem humanen Gewebe gewonnen werden, oder
Enthalten einer größeren Anzahl von Phagosomen als RPE-Zellen, die aus gespendetem humanem Gewebe gewonnen werden.

13. Pharmazeutische Zusammensetzung nach einem der Ansprüche 1-7 oder einer Zubereitung nach Anspruch 12 zur Verwendung bei der Behandlung von Netzhautdegenerationen, einschließlich Makuladegenerationen wie altersbedingte Makuladegeneration, Stargardt'sche Krankheit, Angioidstreifen und Myopie-Makuladegeneration.

14. Pharmazeutische Zusammensetzung nach einem der Ansprüche 1-7 oder Zubereitung nach Anspruch 12, wobei die Zusammensetzung oder Zubereitung zur Transplantation mit einer Matrix, einem Substrat, einem Gerüst oder einem Transplantat formuliert ist.

15. Pharmazeutische Zusammensetzung nach einem der Ansprüche 1-7 oder Zubereitung nach Anspruch 12, wobei die RPE-Zellen eine oder mehrere der folgenden Eigenschaften aufweisen:
eine replikative Lebensdauer, die größer ist als die replikative Lebensdauer von RPE-Zellen, die aus adulten Augen oder fetalem Gewebe gewonnen werden;
eine durchschnittliche Telomerlänge, die wenigstens 30 Prozent der Telomerlänge einer hESC- und/oder humanen iPS-Zelle (oder der Durchschnitt einer Population von hESC- und/oder humanen iPS-Zellen) oder wenigstens 40, 50, 60, 70, 80 oder 90 Prozent der Telomerlänge einer hESC- und/oder humanen iPS-Zelle beträgt;
eine mittlere terminale Restriktionsfragmentlänge (TRF), die länger als 4 kb oder länger als 5, 6, 7, 8, 9, 10, 11, 12 oder sogar 13 kb ist, oder 10 kb oder länger;
einen durchschnittlichen Lipofuszin-Gehalt, der weniger als 50 Prozent des durchschnittlichen Lipofuszin-Gehalts der äquivalenten Anzahl von aus adulten Augen isolierten RPE-Zellen oder weniger als 40, 30, 20 oder 10 Prozent des durchschnittlichen Lipofuszin-Gehalts der äquivalenten Anzahl von aus adulten Augen isolierten RPE-Zellen ist;
einen durchschnittlichen N-Retinyliden-N-Retinylethanolamin (A2E)-Gehalt, der weniger als 50 Prozent des durchschnittlichen A2E-Gehalts der äquivalenten Anzahl von aus adulten Augen isolierten RPE-Zellen oder weniger als 40, 30, 20 oder 10 Prozent des durchschnittlichen A2E-Gehalts der äquivalenten Anzahl von RPE-Zellen ist, die aus adulten Augen isoliert werden;
einen durchschnittlichen N-Retinyliden-N-Retinylethanolamin (A2E)-Gehalt, der weniger als 50 ng pro 10⁵ (100.000) Zellen beträgt;
eine Phagozytoserate der äußeren Segmente des Photorezeptors (POS), die wenigstens 50 Prozent höher ist als die Phagozytoserate des POS für eine gleichwertige Anzahl von RPE-Zellen, die aus adulten Augen isoliert wurden, oder wenigstens 75, 100, 150 oder 200 Prozent höher ist als die Phagozytoserate des POS für eine gleichwertige Anzahl von RPE-Zellen, die von adulten Augen isoliert wurden;
Phagozytoserate der äußeren Segmente des Photorezeptors (POS), die wenigstens 20 Prozent der Gesamtkonzentration des POS nach 24 Stunden oder wenigstens 25, 30, 25, 40 oder 50 Prozent der Gesamtkonzentration des POS nach 24 Stunden beträgt;
ein vermindertes Maß an akkumuliertem oxidativem Stress und/oder DNA-Schäden im Vergleich zu RPE-Zellen, die aus einem adulten Wirt isoliert wurden;
eine durchschnittliche Proteasomaktivität, die wenigstens 50 Prozent größer ist als die durchschnittliche Proteosomaktivität der äquivalenten Anzahl von RPE-Zellen, die aus adulten Augen isoliert wurden, oder wenigstens 60, 70, 80, 90 oder 100 Prozent größer ist als die durchschnittliche Proteosomaktivität der äquivalenten Anzahl von aus adulten Augen isolierten RPE-Zellen;
eine durchschnittliche Akkumulation von Ubiquitinkonjugaten, die weniger als 50 Prozent der durchschnittlichen Akkumulation von Ubiquitinkonjugaten für eine äquivalente Anzahl von RPE-Zellen, die aus adulten Augen isoliert wurden, oder weniger als 40, 30, 20 oder sogar 10 Prozent der durchschnittlichen Akkumulation von Ubiquitinkonjugaten der äquivalenten Anzahl von aus adulten Augen isolierten RPE-Zellen beträgt.

16. Zusammensetzung nach Anspruch 1, wobei die Zusammensetzung ferner einen ROCK-Inhibitor, wie beispielsweise Y-27632, umfasst.

## Revendications

1. Composition pharmaceutique comprenant :
une pluralité de cellules de l'épithélium pigmentaire rétinien (EPR) ; et
un véhicule pharmaceutiquement acceptable ;
dans laquelle la teneur moyenne en mélanine de ladite pluralité de cellules EPR est comprise entre 1 et 5 pg/cellule ;
et où la composition pharmaceutique comprend entre 1 000 et 1 × 10⁹ cellules EPR viables.

2. Composition pharmaceutique selon la revendication 1, dans laquelle au moins 50 %, au moins 60 %, au moins 70 %, ou au moins 80 % des cellules EPR dans ladite composition pharmaceutique sont bestrophine+.

3. Composition pharmaceutique selon la revendication 1 ou la revendication 2, dans laquelle au moins 80 %, au moins 85 %, au moins 90 %, au moins 95 %, ou au moins 99 % des cellules EPR dans ladite composition pharmaceutique sont PAX6+ et/ou MITF+.

4. Composition pharmaceutique selon l'une quelconque des revendications précédentes, dans laquelle au maximum environ une cellule par million de cellules et facultativement au maximum deux cellules pour neuf millions de cellules dans ladite composition pharmaceutique sont positives pour l'expression de OCT-4 et de la phosphatase alcaline (PA).

5. Composition pharmaceutique selon l'une quelconque des revendications précédentes, dans laquelle lesdites cellules EPR sont humaines.

6. Composition pharmaceutique selon l'une quelconque des revendications précédentes, dans laquelle lesdites cellules EPR sont génétiquement manipulées, par exemple pour produire un ou plusieurs facteurs qui inhibent l'angiogenèse.

7. Composition pharmaceutique selon l'une quelconque des revendications précédentes, dans laquelle lesdites cellules EPR sont produites *in vitro* à partir d'une cellule souche pluripotente.

8. Kit comprenant une composition pharmaceutique selon l'une quelconque des revendications précédentes et un récipient distinct comprenant un diluant pharmaceutiquement acceptable dans un volume suffisant pour diluer ladite pluralité de cellules EPR à une concentration cible souhaitée.

9. Composition cryoconservée comprenant :
une pluralité de cellules de l'épithélium pigmentaire rétinien (EPR) cryoconservées contenues dans un milieu de cryoconservation ;
dans laquelle la teneur moyenne en mélanine de ladite pluralité de cellules EPR cryoconservées est comprise entre 1 et 5 pg/cellule et les cellules EPR présentent un niveau de maturité moyen au moment de la congélation de sorte que les cellules EPR récupérées après la décongélation présentent une efficacité d'ensemencement d'au moins environ 60 %, et où ladite composition cryoconservée comprend entre 5 000 et 1 × 10⁸ cellules EPR viables au moment de la congélation.

10. Procédé de production de cellules de l'épithélium pigmentaire rétinien (EPR) destinées à être utilisées dans une préparation pharmaceutique, comprenant :
(a) la mise en culture de cellules EPR dans des conditions adhérentes afin de former une culture essentiellement monocouche de cellules EPR pigmentées présentant une morphologie en pavés ;
(b) le passage des cellules EPR au moins une fois avant que les cellules EPR n'atteignent une teneur moyenne en mélanine supérieure à 8 pg/cellule ; et
(c) après le un ou plusieurs passages, la récolte des cellules EPR pour une cryoconservation ou une formulation pharmaceutique, où, au moment de la récolte, les cellules EPR possèdent une teneur moyenne en mélanine comprise entre 1 et 5 pg/cellule.

11. Procédé selon la revendication 10, dans lequel lesdites cellules EPR sont produites à partir de cellules souches pluripotentes.

12. Préparation pharmaceutique comprenant des cellules EPR appropriées pour le traitement de la dégradation rétinienne, dans laquelle lesdites cellules EPR (a) sont différenciées à partir de cellules souches pluripotentes, (b) contiennent une teneur moyenne en mélanine comprise entre 1 et 5 pg/cellule, et (c) possèdent au moins une des propriétés suivantes :
un attachement à la membrane de Bruch après une transplantation,
une absorption de la lumière diffusée après une transplantation,
une longueur de télomère moyenne plus importante que des cellules EPR dérivées d'un don de tissu humain,
une durée de vie de réplication en culture plus longue que des cellules EPR dérivées d'un don de tissu humain,
une teneur en A2E moins élevée que des cellules EPR dérivées d'un don de tissu humain,
une teneur en lipofuscine moins élevée que des cellules EPR dérivées d'un don de tissu humain,
des dommages induits par les ultraviolets accumulés moindres que des cellules EPR dérivées d'un don de tissu humain, ou
un plus grand nombre de phagosomes que des cellules EPR dérivées d'un don de tissu humain.

13. Composition pharmaceutique selon l'une quelconque des revendications 1 à 7 ou préparation selon la revendication 12, destinée à être utilisée dans le traitement de la dégénérescence rétinienne, comprenant la dégénérescence maculaire telle que la dégénérescence maculaire liée à l'âge, la maladie de Stargardt, les stries angioïdes et la dégénérescence maculaire myopique.

14. Composition pharmaceutique selon l'une quelconque des revendications 1 à 7 ou préparation selon la revendication 12, où la composition ou préparation est formulée pour une transplantation avec une matrice, un substrat, un échafaudage, ou une greffe.

15. Composition pharmaceutique selon l'une quelconque des revendications 1 à 7 ou préparation selon la revendication 12, dans laquelle lesdites cellules EPR exhibent une ou plusieurs des caractéristiques suivantes :
une durée de vie de réplication qui est plus longue que la durée de vie de réplication de cellules EPR obtenues à partir de yeux adultes ou d'un tissu fœtal ;
une longueur moyenne de télomère qui est au moins 30 pour cent de la longueur de télomère d'une CSEh et/ou d'une cellules iPS humaine (ou la moyenne d'une population de cellules CSEh et/ou iPS humaines), ou au moins 40, 50, 60, 70, 80 ou 90 pour cent de la longueur de télomère d'une cellule CSEh et/ou d'une cellule iPS ;
une longueur moyenne de fragment de restriction terminal (TRF) qui est plus longue que 4 kb, ou plus longue que 5, 6, 7, 8, 9, 10, 11, 12 ou même 13 kb, ou 10 kb ou plus ;
une teneur moyenne en lipofuscine qui est moins de 50 pour cent de la teneur moyenne en lipofuscine du nombre équivalent de cellules EPR isolées à partir de yeux adultes, ou moins de 40, 30, 20 ou 10 pour cent de la teneur moyenne en lipofuscine du nombre équivalent de cellules EPR isolées à partir de yeux adultes ;
une teneur moyenne en N-rétinylidène-N-rétinyléthanolamine (A2E) qui est moins de 50 pour cent de la teneur moyenne en A2E du nombre équivalent de cellules EPR isolées à partir de yeux adultes, ou moins de 40, 30, 20 ou 10 pour cent de la teneur moyenne en A2E du nombre équivalent de cellules EPR isolées à partir de yeux adultes ;
une teneur moyenne en N-rétinylidène-N-rétinyléthanolamine (A2E) qui est moins de 50 ng pour 10⁵ (100 000) cellules ;
un taux de phagocytose des segments externes des photorécepteurs (POS) qui est au moins 50 pour cent plus élevé que le taux de phagocytose des POS pour un nombre équivalent de cellules EPR isolées à partir de yeux adultes, ou au moins 75, 100, 150 ou 200 pour cent plus élevé que le taux de phagocytose des POS pour un nombre équivalent de cellules EPR isolées à partir de yeux adultes ;
un taux de phagocytose des segments externes des photorécepteurs (POS) qui est au moins 20 pour cent de la concentration totale des POS au bout de 24 heures, ou au moins 25, 30, 25, 40 ou 50 pour cent la concentration totale des POS au bout de 24 heures ;
un taux réduit de stress oxydatif et/ou dommages à l'ADN accumulé(s) par comparaison à des cellules EPR isolées à partir d'un hôte adulte ;
une activité moyenne du protéasome qui est au moins 50 pour cent plus élevée que l'activité moyenne du protéasome du nombre équivalent de cellules EPR isolées à partir de yeux adultes, ou au moins 60, 70, 80, 90 ou 100 pour cent plus élevé que l'activité moyenne du protéasome du nombre équivalent de cellules EPR isolées à partir de yeux adultes ;
une accumulation moyenne de conjugués d'ubiquitine qui est moins de 50 pour cent de l'accumulation moyenne des conjugués d'ubiquitine pour un nombre équivalent de cellules EPR isolées à partir de yeux adultes, ou moins de 40, 30, 20 ou même 10 pour cent de l'accumulation moyenne de conjugués d'ubiquitine du nombre équivalent de cellules EPR isolées à partir de yeux adultes.

16. Composition selon la revendication 1, où la composition comprend en outre un inhibiteur de ROCK, tel que Y-27632.
